# EUROPEAN PATENT SPECIFICATION

(11) **EP 2 307 572 B1**
(45) Date of publication and mention of the grant of the patent: **31.08.2016**
(21) Application number: 09773018.8
(22) Date of filing: 03.07.2009
(51) Int. Cl.: C12Q 1/68

(54) **DIAGNOSTIC POLYMORPHISMS FOR CARDIAC DISEASE**
DIAGNOSTISCHE POLYMORPHISMEN FÜR HERZKRANKHEIT
POLYMORPHISMS D'UN SEUL NUCLÉOTIDE (SNP) DIAGNOSTIQUES POUR LES MALADIES CARDIAQUES

(30) Priority: 03.07.2008 US 129550 P; 13.08.2008 US 136117 P
(43) Date of publication of application: 13.04.2011
(73) Proprietor: MOR RESEARCH APPLICATIONS LTD., 69710 Tel-Aviv (IL)
(72) Inventor: AMIR, Offer, 34323 Haifa (IL); LEWIS, Basil S, 34753 Haifa (IL)
(74) Representative: Maslanka, Dorota
(86) International application number: PCT/IB2009/052892
(87) International publication number: WO 2010/001358

(56) References cited:
- WO-A1-96/05324
- WO-A2-00/22166
- US-A1- 2004 005 566
- NORDLIE ET AL: "Genetic contributors toward increased risk for ischemic heart disease" JOURNAL OF MOLECULAR AND CELLULAR CARDIOLOGY, ACADEMIC PRESS, GB, vol. 39, no. 4, 1 October 2005 (2005-10-01), pages 667-679, XP005217190 ISSN: 0022-2828
- ASSELBERGS FOLKERT W ET AL: "A role for CETP TaqIB polymorphism in determining susceptibility to atrial fibrillation: a nested case control study" BMC MEDICAL GENETICS, BIOMED CENTRAL, LONDON, GB, vol. 7, no. 1, 19 April 2006 (2006-04-19), page 39, XP021015835 ISSN: 1471-2350
- SCALBERT ET AL: "Implication of microRNAs in the cardiovascular system" CURRENT OPINION IN PHARMACOLOGY, ELSEVIER SCIENCE PUBLISHERS, NL, vol. 8, no. 2, 19 February 2008 (2008-02-19), pages 181-188, XP022535300 ISSN: 1471-4892
- WIESFELD A C P ET AL: "Genetic aspects of atrial fibrillation" CARDIOVASCULAR RESEARCH, OXFORD UNIVERSITY PRESS, vol. 67, no. 3, 15 August 2005 (2005-08-15), pages 414-418, XP004985944 ISSN: 0008-6363
- IAKOUBOVA ET AL: "Association of the Trp719Arg Polymorphism in Kinesin-Like Protein 6 With Myocardial Infarction and Coronary Heart Disease in 2 Prospective Trials" JOURNAL OF THE AMERICAN COLLEGE OF CARDIOLOGY, ELSEVIER, NEW YORK, NY, US, vol. 51, no. 4, 22 January 2008 (2008-01-22), pages 435-443, XP022432404 ISSN: 0735-1097
- MIYASAKA YOKO ET AL: "Secular trends in incidence of atrial fibrillation in Olmsted County, Minnesota, 1980 to 2000, and implications on the projections for future prevalence", CIRCULATION, vol. 114, no. 2, July 2006 (2006-07), pages 119-125, ISSN: 0009-7322
- CORELL ET AL: "Prevalence and prognostic significance of atrial fibrillation in outpatients with heart failure due to left ventricular systolic dysfunction", EUROPEAN JOURNAL OF HEART FAILURE, ELSEVIER, AMSTERDAM, NL, vol. 9, no. 3, 2 March 2007 (2007-03-02), pages 258-265, XP005911965, ISSN: 1388-9842, DOI: 10.1016/J.EJHEART.2006.08.004

## Description

### FIELD OF THE INVENTION

The present invention relates to diagnostic markers, and more specifically to the use of a polymorphism, including a single nucleotide polymorphism (SNP), or a combination of such markers, for diagnosis of cardiac disease, such as heart failure and atrial fibrillation.

In particular, the present invention relates to the use of a polymorphism for diagnosis of susceptibility to cardiac disease in a subject, wherein said polymorphism occurs in a renin angiotensin system gene.

### BACKGROUND OF THE INVENTION

Heart failure (HF) is a condition in which the heart is unable to pump sufficient blood throughout the body. Recently, evidence has accumulated that genetic factors may have a potential role in the pathogenesis of AF in HF patients [1,2].

Atrial fibrillation (AF), which is an arrhythmia defined by the absence of coordinated atrial systole, is a common complication in heart failure patients, and is usually associated with advanced disease and aggravated symptoms [3].

The renin-angiotensin-aldosterone system (RAAS) is a hormone system which plays an important role in regulating blood volume and systemic vascular resistance, which together influence cardiac output and arterial pressure. Renin, which is primarily released by the kidneys, stimulates the formation of angiotensin in blood and tissues, which in turn stimulates the release of aldosterone from the adrenal cortex.

The RAAS appears to be a relevant contributing cause in the pathogenesis of heart failure [4], and AF [1,3,5] including myocardial remodeling [4], regulation of blood pressure, and vascular smooth muscle growth and proliferation [6].

Angiotensin II is the predominant neurohormone in the RAAS, and regulates a number of physiologic responses, including fluid homeostasis, aldosterone production, renal function, vascular smooth muscle contraction, sympathetic nervous activity and salt retention [6]. Angiotensin II plays a key role in the pathophysiology of HF, and treatment with angiotensin (AT)-II receptor antagonists has been suggested in the management of AF patients [9]. Most of the known effects of angiotensin II are mediated through the angiotensin II type 1 receptor (AT1R).

The most extensively studied polymorphism in the AT1R gene is the A1166C variant. The functional significance of this gene variation is uncertain because of its location in the 3'-untranslated region (UTR)[10]. However, this polymorphism has been linked to enhanced physiological responses to Ang II resulting in increased vasoconstrictor activity [11].

Previous clinical studies suggested that AT1R polymorphism was associated with left ventricular hypertrophy [12,13], autonomic modulation of heart rate [2], vascular manifestations of atherosclerosis [14], coronary artery disease[15-18], and for development/progression of renal failure [19-21]. Worsening renal functions and ischemic etiology have both been shown to be associated with a more advanced HF disease and an increased mortality [22,23].

However, most studies did not find a role for the AT1R polymorphism in the determination of LV size and performance, both in healthy individuals and in patients with coronary artery disease [24-29]. Hamon et al showed that subjects homozygous for the AT1R CC mutation did have a lower ejection fraction than those with at least a single A allele (AC+AA)[30]. Apart from the possible association of the AT1R polymorphism with a tendency towards systemic hypertension[19], there was no association between the AT1R CC genotype and either cardiac or vascular structural abnormalities [14,32].

In the human heart, angiotensin II is produced from angiotensin I by the angiotensin-converting enzyme (ACE) and the heart chymase (CMA) pathways. Human heart chymase is a chymotrypsin-like serine protease that is the most catalytically efficient enzyme described, thus far, for the cleavage of angiotensin I to angiotensin II [33]. Angiotensin II is primarily (80%) generated via the chymase pathway [34]. Heart chymase has been implicated in the process of acute inflammation [35], apoptosis of cardiac myocytes, proliferation of fibroblasts6 and tissue remodeling [37-39].

A functional polymorphism of the human *ACE* gene (GenBank accession no. AF118569) was described in which the presence (insertion: I allele), rather than the absence (deletion: D allele), of a 287-bp Alu repeat element in intron 16 (rs4646994) is associated with lower enzyme activity [40,41]. In a review of the literature, Bleumink et al recognized the debatable data in the literature regarding the significance *of ACE* I/D polymorphism in heart failure [42]. In several different ethnic groups; Caucasians, Chinese, black South Africans and Japanese, there was no association with either ischemic or non-ischemic cardiomyopathy [42]. On the contrary, a very few studies did suggest an association between the DD genotype and transplant- free survival rates. Interestingly this poor outcome associated with the genetic polymorphism was blunted with beta blocker treatment [43]. The adverse impact of the DD genotype was also demonstrated in a Swedish population study, but only in concert with several other polymorphisms and not by itself [44].

Aldosterone, an important peptide produced following RAAS activation, plays an important role in growth promotion and cardiac fibrosis, which contributes to ventricular remodeling and was suggested to have an impact on the pathogenesis of HF and AF [45,46]. The final step in the aldosterone synthetic pathway is via an enzymatic reaction catalyzed by aldosterone synthase. The aldosterone synthase (*CYP11B2*) gene (a 9-exon gene localized to chromosome 8q22; GenBank accession no. AC073385) [2] contains a common T-344C polymorphism (a thymidine to cytosine substitution) within its promoter region (rs1799998) [47]. The C allele has been associated with increased binding to the steroidogenic transcription factor 1 (SF-1) [48] as well as with increased aldosterone synthase activity [49,50].

There is disagreement regarding the prevalence and clinical consequences of aldosterone synthase gene polymorphism in patients with systemic hypertension or HF [51,52]. Aldosterone synthase promoter -344C allele linked to higher aldosterone levels has been associated with poorer event- free survival in blacks with HF [53].

WO0022166 provides methods for assessing cardiovascular status in an individual, which comprise determining the sequence at one or more polymorphic positions within the human genes encoding ACE, AT1, AGT, renin, aldosterone synthase, type-2 angiotensin II receptor, endothelin receptor, and beta -adrenoceptor. This document also discloses isolated nucleic acids encoding polymorphisms in genes encoding ACE, AT1, AGT, renin, aldosterone synthase, type-2 angiotensin II receptor, endothelin receptor and beta -adrenoceptor, nucleic acid probes that hybridized to polymorphic positions, kits for the prediction of cardiovascular status, and nucleic acid and peptide targets for use in identifying candidate cardiovascular drugs.

US2004005566 relates to kits and methods for assessing the cardiovascular health of a human and the human's susceptibility to cardiovascular disorders. The methods involve assessing occurrence in the human's genome of one or more polymorphisms (e.g., single nucleotide polymorphisms) that occur in one or more genes associated disclosed herein and that are associated with a disorder in humans.

WO9605324 discloses a method for in vitro detection of a predisposition to myocardial infarction characterized by the simultaneous detection of two different and correlated markers: an insertion/deletion polymorphism in the angiotensin I converting enzyme gene and a polymorphism of the gene coding for the AT1 type angiotensin II receptor gene in the 1166 position region of the gene coding portion.
NORDLIE ET AL: "Genetic contributors toward increased risk for ischemic heart disease" JOURNAL OF MOLECULAR AND CELLULAR CARDIOLOGY, ACADEMIC PRESS, GB, vol. 39, no. 4, 1 October 2005 (2005-10-01), pages 667-679, XP005217190 ISSN: 0022-2828. This document is a review describing representative studies investigating the genes considered most likely to potentially contribute toward an increased risk for CAD and MI. Genes resulting in inherited disorders with which an increased risk of CAD and MI is associated are discussed, as well as a number of candidate genes that may play a role in the multifactorial inheritance of CHD risk.
ASSELBERGS FOLKERT W ET AL: "A role for CETP TaqIB polymorphism in determining susceptibility to atrial fibrillation: a nested case control study" BMC MEDICAL GENETICS, BIOMED CENTRAL, LONDON, GB, vol. 7, no. 1, 19 April 2006 (2006-04-19), page 39, XP021015835 ISSN: 1471-2350. This document is a study investigating the genetic and environmental characteristics of atrial fibrillation (AF).
SCALBERT ET AL: "Implication of microRNAs in the cardiovascular system" CURRENT OPINION IN PHARMACOLOGY, ELSEVIER SCIENCE PUBLISHERS, NL, vol. 8, no. 2, 19 February 2008 (2008-02-19), pages 181-188, XP022535300 ISSN: 1471-4892. This document discusses diagnostic use and therapeutic modulation of individual miRNAs or miRNA clusters in cardiovascular diseases.

### SUMMARY OF THE INVENTION

There is a need for, and it would be useful to have sensitive and accurate markers for diagnosis of cardiac disease.

The present invention provides one or more polymorphisms, including single nucleotide polymorphisms (SNPs), or combinations thereof, for diagnosis of cardiac disease, further characterized in that said cardiac disease comprises heart failure and atrial fibrillation, wherein said atrial fibrillation is a complication of heart failure.

In particular, the present invention refers to the use of a polymorphism for diagnosis of susceptibility to cardiac disease in a subject, wherein said polymorphism occurs in a renin angiotensin system gene, characterized in that said polymorphism occurs in AT1R, further characterized in that said cardiac disease comprises heart failure and atrial fibrillation, wherein said atrial fibrillation is a complication of heart failure.

A nucleotide position in genome at which more than one sequence is possible in a population, is referred to herein as a "polymorphic site" or "polymorphism". Where a polymorphic site is a single nucleotide in length, the site is referred to as a SNP. For example, if at a particular chromosomal location, one member of a population has an adenine and another member of the population has a thymine at the same position, then this position is a polymorphic site, and, more specifically, the polymorphic site is a SNP. Polymorphic sites may be several nucleotides in length due to insertions, deletions, conversions or translocations. As described herein, although reference may be made to an "SNP", it is understood to include any type of polymorphism.

Each version of the sequence with respect to the polymorphic site is referred to herein as an "allele" of the polymorphic site. Thus, in the previous example, the SNP allows for both an adenine allele and a thymine allele. Typically, a reference nucleotide sequence is referred to for a particular gene e.g. in NCBI databases (www.ncbi.nlm.nih.gov). Alleles that differ from the reference are referred to as "variant" alleles. The polypeptide encoded by the reference nucleotide sequence is the "reference" polypeptide with a particular reference amino acid sequence, and polypeptides encoded by variant alleles are referred to as "variant" polypeptides with variant amino acid sequences. Nucleotide sequence variants can result in changes affecting properties of a polypeptide. These sequence differences, when compared to a reference nucleotide sequence, include insertions, deletions, conversions and substitutions: e.g. an insertion, a deletion or a conversion may result in a frame shift generating an altered polypeptide; a substitution of at least one nucleotide may result in a premature stop codon, amino acid change or abnormal mRNA splicing; the deletion of several nucleotides, resulting in a deletion of one or more amino acids encoded by the nucleotides; the insertion of several nucleotides, such as by unequal recombination or gene conversion, resulting in an interruption of the coding sequence of a reading frame; duplication of all or a part of a sequence; transposition; or a rearrangement of a nucleotide sequence, as described in detail above.

Such sequence changes may alter the polypeptide encoded by a gene which in turn may alter the functionality and/or other properties of the polypeptide. For example, a nucleotide change resulting in a change in polypeptide sequence can alter the physiological properties of a polypeptide dramatically by resulting in altered activity, distribution and stability or otherwise affect on properties of a polypeptide.

Alternatively, nucleotide sequence variants can result in changes affecting transcription of a gene or translation of its mRNA, without affecting the polypeptide itself (of course a combination of both types of effects is also possible). A polymorphic site located in a regulatory region of a gene may result in altered transcription of a gene e.g. due to altered tissue specificity, altered transcription rate or altered response to transcription factors. A polymorphic site located in a region corresponding to the mRNA of a gene may result in altered translation of the mRNA e.g. by inducing stable secondary structures to the mRNA and affecting the stability of the mRNA. Such sequence changes may alter the expression of a gene and hence may have physiological effects. However, the present invention is not limited to polymorphisms in which there is a direct effect on the expression of the gene and/or on the resultant polypeptide.

The term "gene," as used herein, refers to an entirety containing entire transcribed region and all regulatory regions of a gene. The transcribed region of a gene including all exon and intron sequences of a gene including alternatively spliced exons and introns so the transcribed region of a gene contains in addition to polypeptide encoding region of a gene also regulatory and 5' and 3' untranslated regions present in transcribed RNA. Each gene described herein has been assigned a specific and unique nucleotide sequence by the scientific community. By using the name of the gene as provided herein, those skilled in the art will readily find the nucleotide sequences of a gene and its encoded mRNAs as well as amino acid sequences of its encoded polypeptides, although some genes may have been known with other name(s) in the art.

As used herein the phrase "diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

As used herein the phrase "diagnosing" refers to classifying a disease or a symptom, determining a severity of the disease, monitoring disease progression, forecasting an outcome of a disease and/or prospects of recovery. The term "detecting" may also optionally encompass any of the above.

As used herein, "about" means plus or minus approximately ten percent of the indicated value.

Other features and advantages of the invention will be apparent from the following detailed description, and from the claims.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 shows AT1R A1166C genotyping in ischemic and non-ischemic HF patients;
FIG. 2 is a Kaplan-Meier plot of survival in HF patients according to AT1R A1166C genotype;
FIG. 3 shows prevalence of atrial fibrillation in chronic systolic heart failure patients by CYP11B2 T-344C genotype TT = homozygous for the -344T allele, TC = heterozygous; CC = homozygous for the -344C allele; and
FIG. 4A shows Kaplan-Meier survival curves according to circulating TNF-alpha levels (below and above median); and FIG 4B shows Cox proportional hazard ratio curves according to combined circulating TNF-alpha and IL-10 levels (both below and above median).

### DESCRIPTION OF THE PREFERRED EMBODIMENTS

The present invention provides the use of a single nucleotide polymorphism (SNP), or a combination of such SNPs. for the diagnosis of cardiac disease, further characterized in that said cardiac disease comprises heart failure and atrial fibrillation, wherein said atrial fibrillation is a complication of heart failure.

Although the association between gene polymorphisms and heart disease, especially coronary artery disease (CAD), has been investigated, the relationship to systolic HF is less understood. Surprisingly, the present inventors have found that a number of different SNPs may in fact be related to the pathogenesis, diagnosis and prognosis of systolic HF and/or other cardiac diseases.

According to some embodiments of the present invention, there is provided the use of a single nucleotide polymorphism (SNP) for diagnosis and/or prognosis of cardiac disease in a subject, further characterized in that said cardiac disease comprises heart failure arid atrial fibrillation, wherein said atrial fibrillation is a complication of heart failure.

According to some embodiments, the single nucleotide polymorphism occurs in a gene selected from the group consisting of a renin angiotensin aldosterone system gene, an adrenergic receptor gene, an inflammatory path gene, a metabolic pathway gene, a cell proliferation gene, a natriuretic peptide receptor gene, a plasminogen activator inhibitor gene and a platelet- activating factor gene.

Examples of such polymorphisms are presented in Appendix I. Examples of single nucleotide polymorphisms of the renin angiotensin gene include AT1R (such as A1166C polymorphism), CYP11B2 (such as a T-344C promoter polymorphism), CMA1 (such as G-1903A polymorphism) and BDKRB2 polymorphisms.

Examples of polymorphisms of the adrenergic receptor gene include polymorphisms of ADRB2 (such as Arg (A)16, A46, Gin (Q)27, C79] or Ile (1)164, T491), ADRB1 (such as Gly (G)49, G145 or Gly (G)389, G1165), ADRA1A (such as Cys (Q347, T1039), and ADRA2B (such as ADRA2B 894± AGAGGAGGA insertion/deletion).

Examples of polymorphisms of inflammatory pathway genes include polymorphisms of interleukin (IL)-10 (such as A-592 or G-1082), IL-6 (such as C (G-reverse)-174), tumor necrosis factor (TNF) (such as A-318), IL-1B (such as T315), IL-1RN (such as 86-bp tandem repeat), and C-reactive protein (CRP) (such as C552). Examples of polymorphisms of metabolic pathway genes include perixosome proliferator-activated receptor genes (such as PPARA, PPARG and PPARGC1A), nuclear respiratory genes (such as NRF1 and GABPB1), NOS3 and GNB3.

An example of a cell proliferation gene is FGF2; examples of natriuretic peptide genes include NPR1 and NPR3; an example of a plasminogen activator inhibitor gene is SERPINE 1; and an example of a platelet-activating factor gene is PLA2G7.

In particular, the present invention refers to the use of a polymorphism for diagnosis of susceptibility to cardiac disease in a subject, wherein said polymorphism occurs in a renin angiotensin system gene, characterized in that said polymorphism occurs in AT1R, further characterized in that said cardiac disease comprises heart failure and atrial fibrillation, wherein said atrial fibrillation is a complication of heart failure.

According to one preferred embodiment, the AT1R gene comprises SEQ ID NO: 2.

According to another preferred embodiment, the AT1R polymorphism is an A1166C polymorphism.

### Polymorphisms Related to RAAS (renin-angiotensin-aldosterone system) Activity

The combination of one or more treatments which target the RAAS products is the cornerstone of HF (heart failure) therapy. However, the efficacy of such combined targeted "anti-RAAS therapy" is debatable as it may be too aggressive. Such combined therapy may produce severe side effects including hypotension, hyperkalemia and renal deterioration.

Accordingly, without wishing to be limited by a single hypothesis, it is believed that specific genetic analysis that identifies patients with one or more polymorphisms will enable the detection of patients with a predisposition for potentially suffering significant side effects due to the anti-RAAS therapy. On the other hand, genetic analysis is also expected to assist in detecting patients who would benefit from the aggressive "Anti-RAAS Therapy", such as combination of three /four anti RAAS therapy medications, for blocking the high activity of this system without such significant side effects.

More specifically, according to some embodiments of the present invention, there is provided one or more polymorphisms, such as SNPs, which are believed to affect RAAS (renin-angiotensin-aldosterone system) activity or at least to predict patients who may suffer from altered RAAS activity. Such polymorphisms are expected to have prognostic and/or diagnostic importance, in terms of clinical manifestations and long-term survival of patients with heart disease, such as chronic systolic HF, and preferably also for determining which patients may potentially be predisposed to side effects from anti-RAAS therapy as opposed to patients who may be potentially predisposed to benefit from such therapy. Surprisingly, the present inventors found that SNPs in the following genes may have such prognostic and/or diagnostic importance: AT1R, CYP11B2, CMA1, ACE and BDKRB2.

The present inventors examined AT1R polymorphism in patients with systolic HF and its relation to clinical manifestations and patient outcome. As described in detail in Example 1 below, 134 patients with HF and reduced systolic function were genotyped for the AT1R A1166C genotype, using polymerase chain reaction and restriction fragment length polymorphism. The relationship between AT1R A1166C polymorphism and clinical, electrocardiographic, echocardiographic and laboratory parameters in patients with ischemic and non-ischemic etiology was studied, and the relation between AT1R genotype and long-term (30 months) patient survival was examined.

It was found that in HF patients, the frequency of the AT1R 1166C allele and especially the CC genotype was similar to that of the general population, but was associated with an ischemic and not a non-ischemic etiology (p=0.02). The CC genotype was associated with more advanced disease and more severe abnormalities of renal function (p=0.008). Survival analysis showed that AT1R CC homozygous patients had significantly higher mortality (p=0.008) (adjusted OR for mortality 6.35, 95% confidence interval 1.49 - 11.21, p=0.01). The results imply that there is a decreased ability to adapt to myocardial damage and cardiovascular dysfunction in patients without the A allele of the AT1 receptor. None of the 50 patients with non-ischemic cardiomyopathy were homozygous for the C allele, possibly due to play of chance or to a different pathophysiologic effect in these circumstances.

The present study demonstrates that CC homozygous patients tend to have reduced LV function (relative difference of 12%). The lack of statistical significant in systolic and diastolic echocardiographic parameters might be either because patients share homogenous phenotype of advanced systolic HF disease, which masks possible differences between the groups, or because other mechanisms are involved. In the present study, AT1R CC homozygous genotype was significantly associated with ischemic etiology and poorer renal function.

The mechanism by which AT1R A1166C may affect HF is in unknown. Given the potent characteristics of angiotensin II in the cardiac remodeling process and in cell growth regulation, AT1R polymorphism may be expected to alter RAAS activation, with consequent clinical effects. This may occur via several other mechanisms. Moreover, the position of this polymorphism in the 3'UTR region of the gene implies it may influence AT1R transcriptional activity. Indeed, it has been recently reported that this polymorphism is mapped to microRNAs (miRNA) target sites and therefore can affect gene expression via miRNA regulation [55]. In this regard, the 1166 C allele rather than the A allele has been associated with increased AT1R expression. It is therefore, reasonable to believe that any effects attributed to AT1R genotype, would become overt mainly in patients homozygous for the 1166C allele compared to patients carrying the +1166A (AA+AC) genotypes.

Alternatively, although it has been hypothesized that the A1166C polymorphism itself possesses bona fide effects on HF phenotype, there exists the possibility that other markers in linkage disequilibrium with this gene are causative, as was previously suggested by Tiret et al [56]. It should be noted that regardless of the reason for the diagnostic and/or prognostic efficacy of the polymorphism, it is encompassed within the present invention for its prognostic and/or diagnostic efficacy.

The population frequency of the AT1R A1166C in the present study by the present inventors was found to be 74 and 26% for A and C alleles, respectively. This allele distribution showed a similarity to the respective frequencies reported in dbSNP using different European Caucasian populations or CEPH samples (65-75 and 25-35% for the A and C alleles, respectively). Considerable interethnic variation in the frequencies of this polymorphism has been demonstrated with the -1166C allele being rarer in Afro-American, and Asian populations (94-97 and 3-6% for the A and C alleles, respectively, dbSNP) compared with European Caucasian groups [29, 57], which is consistent with the present findings.

The present inventors have therefore determined that AT1R A1166C polymorphism is a major determinant of late outcome in patients with ischemic cardiomyopathy. Patients homozygous for a gene variation associated with increased AT1R expression and enhanced receptor activity are more likely to have poor prognosis and higher mortality. These findings imply not necessarily a causal relation, but presumably (without wishing to be limited by a single hypothesis) a diminished adaptive capability for the AT1R 1166CC genotype group. In patients homozygous for the C allele, the observed significant clinical deterioration is possible attributed to exaggerated neurohormonal activation of RAAS, again without wishing to be limited by a single hypothesis. These patients may benefit from intensified medical treatment including aggressive anti-RAAS therapy such as a combined "triad" regimen of ACEI, ARB and direct aldosterone antagonists. Future treatment may alter or blunt RAAS activity. The findings support the principle of genome-based therapies in the future treatment of HF patients.

The present inventors also analyzed the possible association between aldosterone synthase (*CYP11B2*) T-344C polymorphism, which is associated with increased aldosterone activity, and the prevalence of AF in 191 consecutive patients who had symptomatic systolic HF (left ventricular ejection fraction <40%) for at least 3 months prior to recruitment.

It was found that *CYP11B2* T-344C promoter polymorphism is associated with predisposition to clinical AF in patients with HF. Hence, in systolic HF patients, polymorphism of the aldosterone synthase, *CYP11B2* CC genotype, may serve as a significant marker for the presence of AF and emphasizes genetic predilection for differences in the clinical course of HF patients.

As described in greater detail in Example 2 below, genomic DNA was extracted from peripheral blood leukocytes using a standard protocol. Subjects were genotyped for the *CYP11B2* polymorphism, using the polymerase chain reaction-restriction fragment length polymorphism approach.

Atrial fibrillation (AF) was found to be present in 57 (32%) of HF patients. The -344 CC genotype was found to be a strong independent marker for AF. Almost half (45%) patients with this genotype had AF, compared to a quarter (27%) with - 344 TT and TC genotypes (p=0.02). A multivariate stepwise logistic regression model which included age, sex, New York Heart Association (NYHA) class, *CYP11B2-*344CC genotype and echocardiographic measurements of left ventricular ejection fraction (LVEF), left atrial (LA) dimension, left ventricular end diastolic diameter and mitral regurgitation severity showed that the *CYP11B2* CC genotype (adjusted for age and left atrial size) was an independent predictor of AF (adjusted odds ratio 2.59, 95% confidence interval 1.68 - 3.98, p=0.02).

It was determined that the *CYP11B2* T-344 C promoter polymorphism associated with aldosterone synthase expression is related to a 2-3 fold increased prevalence of AF in HF patients. The -344 CC genotype was shown to be a strong independent marker for AF, and almost half the patients with this genotype were found to suffer from AF, compared to a quarter of those with the -344 TT and TC genotypes.

The prevalence of AF (32%) in the HF population was in the expected range [58,59]. Three parameters were associated with AF: LA size, age and *CYP11B2* CC genotype. Age is a well known determinant of AF, in both the general population and in HF patients. The present inventors and others did not find LVEF to be a significant predictor of AF in these patients with severe HF [60]. Also, in contrast to previous reports 1, NYHA class was not a significant correlate of AF in the present population. It is possible that this association was obscured in the relatively ill homogenous population studied as described below, as 55% of the patients in NYHA Class 3-4..

LA size is related to cardiac remodeling, and an increased LA dimension contributes to the development of AF in HF. The pathogenesis of AF is mediated through both mechanical and electrical remodeling via sympathetic activation and inflammation [61,62]. The RAS-aldosterone axis plays a crucial part in these processes [63,64]. In the failing heart, there is a significant increase in aldosterone expression [65]. This occurs as the activity of aldosterone synthase (CYP11B2), the key enzyme in the aldosterone production, is increased in HF patients [66]. Several reports, in different ethnic populations, suggested that patients who are homozygous for the C allele of the *CYP11B2* gene promoter polymorphism (T-344C) may have an adverse outcome. According to these reports, these individuals suffer from higher blood pressure and have elevated left ventricular mass and hypertrophy [67,68]. In patients with idiopathic dilated cardiomyopathy, the *CYP11B2* CC genotype was associated with larger left ventricular volumes and significantly elevated plasma levels of aldosterone [51]. Moreover, in Afro-American HF patients, the *CYP11B2* CC genotype was associated with higher mortality rates compared to the *CYP11B2* TT genotype [53].

In the present study, by contrast, *CYP11B2* CC genotype was a significant predictor of AF but had no direct correlation with LA size. Although the present inventors did not examine direct inflammatory mediators, it is believed (without wishing to be limited by a single hypothesis) that *CYP11B2* CC genotype may have contributed to AF pathogenesis through neurohormonal, inflammatory and autonomic system activation [61,62,64,69].

The genetic predisposition of HF patients to AF may have important practical implications. Beta blocker therapy, with known RAS antagonistic characteristics, has been suggested to reduce AF prevalence in systolic HF patients [59]. More specific therapy with direct aldosterone antagonists may offer stronger anti-remodeling properties. This concept, especially in the *CYP11B2* CC genotype subpopulation, was also implied recently by others [63,70] and may potentially decrease AF prevalence in these patients.

The present inventors further studied ACE and CMA polymorphisms and their relationship to HF.

Two candidate polymorphisms were studied in the genes encoding these two enzymes, a functional polymorphism of the human *ACE* gene (GenBank accession no. AF118569) involving the presence (insertion: I allele), rather than the absence (deletion: D allele), of a 287-bp Alu repeat element in intron 16 (rs4646994), and a novel single nucleotide polymorphism (SNP) (G/A transition at position -1903 of the 5' untranscribed region of the gene, rs1800875) close to the regulatory region of the CMA1 gene (GenBank accession no. M64269. There is an impressive shortage of data in the literature regarding the impact of this specific polymorphism on systolic HF. Based on the premise that ACE I/D and *CMA1* (-1903G/A) may affect RAAS activity, the present authors hypothesized that these polymorphisms may have clinical importance in patients with chronic systolic HF. The prevalence of these two polymorphisms of the *ACE* /*CMA1* genes was evaluated among chronic systolic HF patients compared to healthy controls and their relation to the etiology (ischemic/non-ischemic) of HF. The association of these gene polymorphisms with the clinical manifestations of HF patients was also examined.

As described in detail in Example 3 below, 195 patients with HF and systolic LV dysfunction (ejection fraction <40%) for *ACE* insertion (I)/deletion (D) and *CAM1* (-1903G/A) polymorphisms were genotyped. HF etiology and patients' clinical manifestations were analyzed in relation to genotype subtypes.

The *CMA*1 -1903 GG genotype was found to be associated with a non-ischemic HF etiology (χ2=6.67, *P*=0.009). In the group of HF patients, the odds ratio of *CMA1* GG genotype having a non-ischemic etiology was 2.48 (95% C.I. 1.23-5.00). The *CMA1* GG genotype was associated with lower ejection fraction (*P*=0.005). Conversely, the *ACE* D allele had no detectable impact on systolic HF phenotype. It was therefore concluded that in patients with chronic systolic HF, the *CMA1* polymorphism was related to non-ischemic etiology of HF. Patients homozygous for the G allele had a significantly greater reduction in systolic LV function.

The study showed that in patients with chronic systolic HF, the *CMA1 -* 1903G/A polymorphism, and in particular homozygosity for the G allele, was more frequent in patients with a non-ischemic etiology of HF and was associated with a greater reduction in LV ejection fraction. The overall frequency of the GG genotype in HF patients was similar to that in the general population, implying then, not necessarily a causal relation, but presumably a differing adaptation to myocardial damage (without wishing to be limited by a single hypothesis).

The association between *CMA1* gene polymorphisms and heart disease, studied mainly in patients with hypertrophic cardiomyopathy [71-73] has not been clear. The present inventors believe the present study to be the first which demonstrates an association between *CAM1* -1903G/A polymorphism and LV systolic dysfunction. Proposed mechanisms include the possibility that the polymorphism, which is located in promoter of the *CAM1* gene, alters protein expression, or if not functional, may be in linkage disequilibrium with other causative alleles [42].

Effects of *CMA1* polymorphism may be mediated through an acceleration of the remodeling process in patients with HF, and mainly in patients with non-ischemic cardiomyopathy (without wishing to be limited by a single hypothesis). Chymase is produced from mast cells and is not inhibited by angiotensin-converting enzyme inhibitors [74]. In HF patients, mast cells increase in number in the failing myocardium [75], and may be implicated in ventricular dilatation and cardiac decompensation [76].

Without wishing to be limited by a single hypothesis, these changes may be attributed to local angiotensin II activity, which induces hypertrophy of cardiac myocytes and myocardial fibrosis and therefore is the most important remodeling factor in the heart [77,78]. Chymase may be responsible for the vast majority of production of local angiotensin II in the myocardium [34]. In addition to the effects associated with direct angiotensin II production, chymase is associated with apoptosis; TGF-β mediated fibrosis [79], collagen formation [80] and fibroblast differentiation to myofibroblasts [36,76,81]. Moreover, mast cell chymase produced in the myocardium can directly induce acute inflammation and affect tissue remodeling through activation of matrix metalloproteinases [38] and IL-1β precursors [37], and stimulation of IL-8 release resulting in recruitment of granulocytes [39].

All of these are important features of the cardiac remodeling process, which may explain the present finding of lower LV ejection fraction in patients with the *CMA1* GG genotype (without wishing to be limited by a single hypothesis). Of note, as per inclusion criteria, all study patients had systolic heart failure (EF<40%), with a mean LVEF of 24±6:5%. Thus, the absolute EF difference of 4 points in the CAM1 GG sub-group (25% vs. 21%), actually reflects a 16% change when compared to the mean EF and is therefore statistically very significant (P = 0.005).

It is less clear why *CMA1* polymorphism is associated with cardiomyopathy of non-ischemic etiology. It may be related to the long term impact of the remodeling process in systolic HF. In patients with ischemic etiology, it is not uncommon that HF symptoms start after initial extensive myocardial damage while the remodeling process contributes little to the progression of HF. On the other hand, in non-ischemic cardiomyopathy, the remodeling process may have greater importance and be linked more closely to the inflammatory process. In an animal model of viral myocarditis, there was an increased density of myocardial mast cells with a simultaneous up regulation in gene expression of inflammatory cytokines and mouse mast cell protease-5 (which is the counterpart of the human chymase) [35], indicating that mast cell chymase both mediates and accelerates inflammatory pathways and is a crucial player in the remodeling process. Moreover, the myocardial remodeling phenomenon in HF progression may be the end point of several pathways, only some of which are mediated through cardiac angiotensin II formation. In non-ischemic cardiomyopathy, the chymase-dependent remodeling process may be predominant, causing the observed reduced systolic function in the present *CMA1* GG patients (without wishing to be limited by a single hypothesis).

The population frequency of the *CMA1* -1903G/A genotype was found to be 53% and 47% for A and G alleles, respectively. This allele distribution showed a similarity to the respective frequencies reported in dbSNP using a Caucasian group (58% and 42% for the A and G alleles, respectively). Considerable interethnic variation in the frequencies of this polymorphism has been demonstrated, with the - 1903G allele being rarer in Caucasian populations compared with Afro-American, Chinese and Japanese groups (18-20% and 80-82% for the A and G alleles, respectively, dbSNP), which is consistent with the present findings.

The present inventors did not find a clinical association with the *ACE* I/D genotype in HF patients. Although an association between *ACE* I/D polymorphism and cardiomyopathy has previously been reported [82,83], other studies did not confirm such relationship and in those which did, the study cohorts deviated from the Hardy-Weinberg equilibrium [42,84]. Some authors have suggested that although there was no causative relation between the *ACE* I/D polymorphism and cardiomyopathy, HF patients with the *ACE* DD genotype have poor outcome and increased mortality [43]. The present inventors, as others [85] did not find such a correlation. The vast majority of the patients of the present study were treated with pharmacotherapy involving modulation of the RAAS, including beta blocker, ACEI and/or ARB. More than a quarter were treated in addition with direct aldosterone antagonists.

The clinical impact of the *ACE* I/D polymorphism may have been attenuated by these treatments, as demonstrated previously [43,86]. Another explanation may be that *ACE* I/D genotype acts only in concert with other polymorphisms as a synergistic genetic polymorphism in order for its prognostic implications to become evident [44]. Polymorphism in the chymase gene, less blunted by medical therapy, did have clinical implications and a lower LV ejection fraction in the present patients.

It was concluded that *CMA1* promoter polymorphism was associated with patients (particularly with non-ischemic etiology for HF) who had greater reduction in measured systolic LV function. In contrast, *ACE* I/D polymorphism had no relation to the level of cardiac function. Although a single center with relatively small patient numbers was studied, the findings were fairly robust by statistical analysis. The findings may explain differences in response to therapies aimed at modulation of the RAAS in patients with apparently similar HF profiles and treatments.

The present invention, in at least some embodiments as described in greater detail below, comprises test kits and diagnostic methods for detecting one or more RAAS-related polymorphisms, optionally and preferably for prognostic and diagnostic uses in relation to heart disease, more preferably for HF (heart failure) and optionally and most preferably for determining which patients have a predisposition toward potential significant side effects with anti- RAAS therapy and which patients may be expected to potentially benefit from such therapy.

### Polymorphisms Related to Sympathetic Activity

Enhanced sympathetic activation has a central role in the development of heart failure. Increased sympathetic activity is known for its deleterious effects on the myocardium and the coronary system, either alone or in concert with other systems such as with the RAAS system for facilitating fibrosis, apoptosis, necrosis and fatal gene activation, leading to morbidity and mortality. Clearly genetic analysis of genes related to such enhanced sympathetic activity would be useful as a diagnostic and prognostic tool

According to some embodiments of the present invention, there is provided one or more polymorphisms of the sympathetic nerve system receptors on the myocardium itself, specifically the beta (1/2)-adrenoceptor and the alpha-1 and 2 and its subtypes such as alpha- 2C-adrenoceptor. Each of them may alter the sympathetic influence and consequently may cause enhance sympathetic tone manifest as a trigger for myocardial damage, coronary events, cardiac remodeling and higher arrhythmia and mortality rates.

It was previously demonstrated that different patients gave different responses to the same regimen therapy in beta blockers as seen in several trials, suggesting that the different sub-type populations may be an important factor in determining the patient response. These different populations may be a reflection of different beta/alpha adrenoceptor in these populations. Tracing the specific polymorphism in the individual patient may be crucial factor in matching the relevant anti- sympathetic therapy for him as well {Domanski MJ, Krause-Steinrauf H, Massie BM, Deedwania P, Follmann D, Kovar D, Murray D, Oren R, Rosenberg Y, Young J, Zile M, Eichhorn E; BEST Investigators: A comparative analysis of the results from 4 trials of beta-blocker therapy for heart failure: BEST, CIBIS-II, MERIT-HF, and COPERNICUS J Card Fail. 2003 Oct;9(5):354-63}.

Some non-limiting examples of polymorphisms related to the sympathetic system, and also relating to differential activity therein, including but are not limited to polymorphisms of the adrenergic receptor gene include polymorphisms of ADRB2 (such as Arg (A)16, A46, Gln (Q)27, C79] or Ile (I)164, T491), ADRB1 (such as Gly (G)49, G145 or Gly (G)389, G1165), ADRA1A (such as Cys (C)347, T1039), and ADRA2B (such as ADRA2B 894± AGAGGAGGA insertion/deletion).

The present invention, in at least some embodiments as described in greater detail below, comprises test kits and diagnostic methods for detecting one or more sympathetic system-related polymorphisms, optionally and preferably for prognostic and diagnostic uses in relation to heart disease, more preferably for HF (heart failure) and optionally and most preferably for determining which patients have a predisposition to benefit from sympathetic system related therapies such as beta blocker therapies for example.

### Polymorphisms Related to Inflammatory Activity

As described in greater detail with regard to the Example below, different cytokines have different effects on heart failure (HF) patients. In particular, it was noted that IL-10 plays a major role in patients with HF. As opposed to known inflammatory cytokines such as TNF-alpha, IL-10 was proposed in the past to have a protective effect as a non- inflammatory cytokine. However, surprisingly, the present inventors found that the mortality in patients with combined elevation of both IL-10 and TNF-alpha was the highest, suggesting that IL-10 may have a counter- productive effect. The interaction between the different cytokines, such as IL-10/ TNF-alpha, was further elaborated as described in greater detail below.

Since the production of such cytokines is regulated through various genetic factors, according to at least some embodiments of the present invention, there is provided one or more polymorphisms for the above mentioned cytokines as being important factors in the pathogenesis, predisposition and prognosis of HF which may have treatment implications, for example in terms of selecting one or more therapies for patients having such polymorphisms. Furthermore, according to at least some embodiments of the present invention, there is provided one or more inflammatory activity related polymorphisms, which may optionally not be polymorphisms for the above mentioned cytokines.

Some non-limiting examples of polymorphisms related to inflammatory activity, and also relating to differential levels of such activity, include but are not limited to polymorphisms of inflammatory pathway genes, including but not limited to polymorphisms of interleukin (IL)-10 (such as A-592 or G-1082), IL-6 (such as C (G-reverse)-174), tumor necrosis factor (TNF) (such as A-318), IL-1B (such as T315), IL-1RN (such as 86-bp tandem repeat), and C-reactive protein (CRP) (such as C552).

The present invention, in at least some embodiments as described in greater detail below, comprises test kits and diagnostic methods for detecting one or more inflammatory activity-related polymorphisms, optionally and preferably for prognostic and diagnostic uses in relation to heart disease, more preferably for HF (heart failure) and optionally and most preferably for determining which patients have a predisposition to benefit from inflammatory activity related therapies.

### Polymorphisms Related to Cell Proliferation

Natriuretic peptides (BNP, NT-proBNP) have been widely used for the diagnosis and prognostic evaluation of HF (heart failure), as a non-limiting example of a diagnostic and prognostic cell proliferation system. The importance of B-type natriuretic peptide (BNP) as a diagnostic and therapeutic modality in cardiovascular disease and specifically in HF is well known {Ang DS, Wei L, Kao MP, Lang CC, Struthers AD.A comparison between B-type natriuretic peptide, global registry of acute coronary events (GRACE) score and their combination in ACS risk stratification. Heart. 2009 Apr 6; Hobbs RE. Using BNP to diagnose, manage, and treat heart failure.,cleve Clin J Med. 2003 Apr;70(4):333-6}. BNP levels correlate clinical, physiologic and prognosis in HF and acute coronary syndromes as well. Accordingly, analysis of the genetic variation of the cell proliferation genes, including those related to natriuretic peptides, may provide a diagnostic and/or prognostic tool for heart failure.

NRP1 is a membrane-bound coreceptor to a tyrosine kinase receptor for both vascular endothelial growth factor (VEGF; MIM 192240) and semaphorin (see SEMA3A; MIM 603961) family members. NRP1 plays versatile roles in angiogenesis. The neuropilins-1 and -2 (NRP1 and NRP2) function as receptors vascular endothelial growth factor and have been implicated in angiogenesis. Hypoxia and nutrient deprivation stimulate the rapid loss of NRP1 expression in endothelial. NRP2 expression, in contrast, is maintained under these conditions.

B-type natriuretic peptide (BNP) is a peptide hormone of myocardial origin with significant cardioprotective properties. It was shown by the present inventors that in heart failure patients referred to an outpatient specialized heart failure center, an upper tertile NT-proBNP level identified patients at high risk for mortality. A single high > 550 pg/ml NT-proBNP measurement appears to be useful for selecting patients for care in a heart failure center, and a level > 2000 pg/ml for assigning patients to high priority management {Amir O, Paz H, Ammar R, Yaniv N, Schliamser JE, Lewis BS.Isr Med Assoc J. 2008:152-3. Usefulness and predictive value of circulating NT-proBNP levels to stratify patients for referral and priority treatment in a specialized outpatient heart failure center. Isr Med Assoc J. 2008; 10(2):109-12}.

Patients with either heart failure or myocardial ischemia present with high levels of BNP in plasma and elevated expression in the myocardium. It was shown that hypoxia via the induction of hypoxia inducible factor 1 (HIF-1) stimulated protein release of BNP and VEGF as manifested by an increased of mRNA levels of BNP.

According to at least some embodiments of the present invention, there is provided one or more polymorphisms for the above mentioned natriuretic peptides as being important factors in the pathogenesis, predisposition and prognosis of HF which may have treatment implications, for example in terms of selecting one or more therapies for patients having such polymorphisms. Furthermore, according to at least some embodiments of the present invention, there is provided one or more cell proliferation related polymorphisms, which may optionally not be polymorphisms for the above mentioned natriuretic peptides.

Some non-limiting examples of polymorphisms related to cell proliferation, and also relating to differential levels of such activity, include but are not limited to polymorphisms of cell proliferation genes, including but not limited to FGF2; and/or polymorphisms of natriuretic peptide genes, including but not limited to NPR1 and NPR3.

The present invention, in at least some embodiments as described in greater detail below, comprises test kits and diagnostic methods for detecting one or more cell proliferation-related polymorphisms, including but not limited to polymorphisms associated with natriuretic peptides, optionally and preferably for prognostic and diagnostic uses in relation to heart disease, more preferably for HF (heart failure).

### Polymorphisms related to metabolic pathway genes

Cellular energy production is tightly linked to metabolic demand. The capacity for cellular ATP production is controlled, in part, by the expression levels of nuclear genes involved in mitochondrial oxidative metabolism. Accordingly, cellular energy metabolism necessitates transduction of diverse signals related to cellular energy demands to the nucleus.

The PPAR gene pathway consists of interrelated genes that encode transcription factors, enzymes, and downstream targets which coordinately act to regulate cellular processes central to glucose and lipid metabolism. The pathway includes the PPAR genes themselves, other class II nuclear hormone receptor transcription factors within the PPAR family, PPAR co-activators, PPAR co-repressors, and downstream metabolic gene targets.

The PPARγ coactivator-1α(PGC-1α), had been characterized as a broad regulator of cellular energy metabolism. PGC-1β, and the PGC-1-related protein, a family of inducible transcriptional coactivators responsive to selective physiological stimuli, which are mediated between the extracellular events and the regulation of genes involved in energy metabolism.

These transcription factors have been implicated in the development of myocardial hypertrophy and dilated cardiomyopathy as well as response to myocardial ischemia/infarction and, by association, ischemic cardiomyopathy.

Diabetes mellitus is a known risk factor for coronary atherosclerosis, myocardial infarction, and ischemic cardiomyopathy. Insulin resistance is associated with left hypertrophy and hypertensive cardiomyopathy. The relationship between insulin resistance and cardiomyopathy is less well established. Systemic and myocardial glucose uptake is compromised in heart failure independent of etiology. These abnormalities are associated with cellular deficits of insulin signaling. Insulin resistance and fatty acid excess are potential therapeutic targets in heart failure. Indeed, that shifting the energy substrate preference away from fatty acid metabolism and toward glucose metabolism could be an effective adjunctive treatment in patients with heart failure, in terms of left ventricular function and glucose metabolism improvement including Peroxisome proliferator activator receptor gamma agonists which are used in diabetes mellitus as they have combined antilipemic and insulinsensitizing activity. Similarly, genetic testing and drug therapy may apply to patients with heart failure and/or coronary artery disease with or without diabetes mellitus.

The enzyme Nitric oxide synthase (NOS) catalyzes the generation of NO (nitric oxide). All isoforms of NOS (C/I /E/N) exist in the heart, when in normal heart the e NOS is the dominant. NO in the heart decreases both oxygen consumption and glucose metabolism of the myocardium cells as well as possible lipid metabolism inhibition.

According to at least some embodiments of the present invention, there is provided one or more polymorphisms for the above mentioned metabolic pathway genes as being important factors in the pathogenesis, predisposition and prognosis of HF which may have treatment implications, for example in terms of selecting one or more therapies for patients having such polymorphisms. Furthermore, according to at least some embodiments of the present invention, there is provided one or more metabolic pathway related polymorphisms.

Some non-limiting examples of polymorphisms of metabolic pathway genes include but are not limited to perixosome proliferator-activated receptor genes (including but not limited to PPARA, PPARG and PPARGC1A), nuclear respiratory genes (including but not limited to NRF1 and GABPB 1), NOS3 and GNB3.

The present invention, in at least some embodiments as described in greater detail below, comprises test kits and diagnostic methods for detecting one or more metabolic pathway-related polymorphisms, including but not limited to polymorphisms associated with perixosome proliferator-activated receptor genes and/or nuclear respiratory genes, optionally and preferably for prognostic and diagnostic uses in relation to heart disease, more preferably for HF (heart failure).

### Polymorphisms related to blood related genes

Several studies suggested that inflammation has an important role in HF progression. Serum oxidative stress level is a crucial element of the inflammatory process, owing to the accumulation of reactive oxygen/nitrogen species that might provoke and exacerbate the myocardial damage of the already failing heart. Several medications claim to have at least some beneficial effects through anti-oxidant potential. The present inventors recently reported serum oxidative stress level correlates with clinical parameters in chronic systolic heart failure patients {Amir O et al; Clin Cardiol. 2009}. Plasma platelet-activating factor acetylhydrolase acts as a key defense against oxidative stress by hydrolyzing PAF and oxidized -phospholipids. Deficiency of the activity of this enzyme may thus potentially result in predisposition to myocardial damage leading to ischemic and non-ischemic cardiomyopathy and be a potential target for HF/CAD treatnment.

Fibrinolysis in blood is mainly reflected by the activities of tissue plasminogen activator (tPA) and of plasminogen activator inhibitor-1 (PAI-1). Plasminogen activator inhibitor-1 is a serine protease inhibitor (seipin) protein (SERPINE1). As the principal inhibitor of tissue plasminogen activator and urokinase, the activators of plasminogen and fibrinolysis. Accordingly, high PAI-1 levels have been associated with atherosclerotic plaque formation and in a prothrombotic state, carrying an increased risk of arterial occlusion and consequently with myocardial infarction. The human PAI-1 gene has been mapped on chromosome 7 (q21.3-q22) and contains 9 exons and 8 introns and a possible association with ischemic and non-ischemic cardiomyopathy will be tested. Changes in plasma fibrinolytic parameters were shown with acute AT1 antagonism via suppression of angiotensin II in HF patients and were associated with a significant improvement in plasma fibrinolytic parameters.

According to at least some embodiments of the present invention, there is provided one or more polymorphisms for the above mentioned blood related genes as being important factors in the pathogenesis, predisposition and prognosis of HF which may have treatment implications, for example in terms of selecting one or more therapies for patients having such polymorphisms. Furthermore, according to at least some embodiments of the present invention, there is provided one or more blood related polymorphisms.

Some non-limiting examples of polymorphisms of plasminogen activator inhibitor gene include but are not limited to SERPINE 1; and an example of a platelet-activating factor gene is PLA2G7.

The present invention, in at least some embodiments as described in greater detail below, comprises test kits and diagnostic methods for detecting one or more blood-related polymorphisms, including but not limited to polymorphisms associated with plasminogen activator inhibitor genes and/or platelet-activating factor genes, optionally and preferably for prognostic and diagnostic uses in relation to heart disease, more preferably for HF (heart failure).

### Diagnostic Methods and Test Kits

One major application of the current invention is diagnosing a susceptibility to a cardiac condition. The risk assessment methods and test kits of this invention can be applied to any healthy person as a screening or predisposition test, although the methods and test kits are preferably applied to high-risk individuals (who have e.g. family history of cardiac disease, one or more cardiac specific risk factors, one for more general risk factors such as obesity or any combination of these). Diagnostic tests that define genetic factors contributing to cardiac disease might be used together with or independent of the known clinical risk factors to define an individual's risk relative to the general population. Better means for identifying those individuals susceptible for cardiac disease should lead to better preventive and treatment regimens, including more aggressive management of the risk factors for cardiac disease such as obesity, lack of physical activity, hypercholesterolemia, elevated LDL cholesterol, low HDL cholesterol, elevated BP, cigarette smoking and inflammatory components as reflected by increased C-reactive protein levels or other inflammatory markers. Physicians may use the information on genetic risk factors to convince particular patients to adjust their life style e.g. to stop smoking, to reduce caloric intake or to increase exercise.

In one embodiment of the invention, diagnosis of a susceptibility to cardiac disease in a subject is made by detecting one or more polymorphisms, such as SNPs, as described herein in the subject's nucleic acid. The presence of cardiac disease associated alleles of the assessed polymorphisms in individual's genome indicates subject's increased risk for cardiac disease.

With regard the sequences listed herein by SEQ ID NO, it should be noted that all odd-numbered SEQ ID NOs relate to the WT (wild type) while all even-numbered SEQ ID NOs relate to the mutant SNP sequence. However, both types of sequences may optionally have diagnostic and/or prognostic uses as described herein.

Preferably according to at least some embodiments of the present invention, there is provided a polynucleotide comprising at least 10 contiguous nucleotides of a nucleotide sequence selected from the group consisting of the nucleotide sequences of even numbered SEQ ID NOs, or a complementary polynucleotide thereof. The polynucleotide comprises at least 10 contiguous nucleotides of a nucleotide sequence selected from the group consisting of nucleotide sequences of even numbered SEQ ID NOs and comprising a polymorphic site. The length of the polynucleotide is 10 to 400 nucleotides, and preferably 10 to 100 nucleotides, and more preferably 10 to 50 nucleotides. The polynucleotide may be DNA or RNA.

Preferably according to at least some embodiments of the present invention, there is provided an allele-specific polynucleotide for diagnosis of cardiovascular disease as described herein, hybridized with the polynucleotide including at least 10 contiguous nucleotides of a nucleotide sequence selected from the group consisting of nucleotide sequences of even numbered SEQ ID NOs and comprising the nucleotide of a polymorphic site or a complementary polynucleotide thereof.

The allele-specific polynucleotide refers to polynucleotide hybridized specifically with each allele. That is, the allele-specific polynucleotide is hybridized such that a base of a polymorphic site in polymorphic sequences of even numbered SEQ ID NOs can be specifically distinguished. The hybridization can usually be carried out under a strict condition, for example, in a salt concentration of 1 M or less and at a temperature of 25 C or higher. For example, 5xSSPE (750 mM NaCl, 50 mM Na phosphate, 5 mM EDTA, pH 7.4) and 25 to 30 C may optionally be suitable for the allele-specific probe hybridization, without wishing to be limited in any way.

According to at least some embodiments of the present invention, the allele-specific polynucleotide may optionally be a primer. The primer refers to a single-strand oligonucleotide capable of initiating a template-directed DNA synthesis in an appropriate buffer under an appropriate condition (for example, in the presence of four different nucleoside triphosphates and a polymerizing agent such as DNA, RNA polymerase or reverse transcriptase) at a proper temperature. The length of the primer may vary according to the purpose of use, but is usually 15 to 30 nucleotides. A short primer molecule generally requires lower temperatures to be stably hybridized with a template. The primer sequence does not necessarily need to be completely complementary with the template, but should be sufficiently complementary to be hybridized with the template. Preferably, the primer has 3' end arranged so as to correspond to the polymorphic sites of the sequences of the even numbered SEQ ID NOs. The primer is hybridised with a target DNA including the polymorphic site and initiates amplification of allele having complete homology to the primer. The primer is used as a primer pair with the other primer hybridized at the opposite side. Amplification is performed from the two primers, indicating that there is a specific allele. The primer of the present embodiment optionally includes a polynucleotide fragment used in a ligase chain reaction (LCR).

According to at least some embodiments of the present invention, the allele-specific polynucleotide may be a probe. The probe refers to a hybridization probe, which is an oligonucleotide capable of binding sequence-specifically to a complementary strand of a nucleic acid. Such a probe includes a peptide nucleic acid introduced by Nielsen, et al., Science 254, 1497-1500 (1991). The probe of the present invention is an allele-specific probe. When a polymorphic site is located in DNA fragments derived from two members of the same species, the allele-specific probe is hybridized with the DNA fragment derived from one member but is not hybridized with the DNA fragment derived from the other member. In this case, the hybridization condition should be sufficiently strict to be hybridized with only one allele by showing a significant difference in terms of the intensity of hybridization between alleles. The probe of the present invention is preferably arranged such that its central site (i.e., 7th position in a probe consisting of 15 nucleotides, or 8th or 9th position in a probe consisting of 16 nucleotides) has the polymorphic site of the above sequence. In this way, a hybridization difference between alleles can be caused. The probe of these embodiments of present invention can be used in a diagnosis method for detecting an allele, etc. The diagnosis method includes but is not limited to detection methods based on hybridization of nucleic acid such as southern blot. In a method using a DNA chip, the probe can previously be bound to a substrate of the DNA chip.

According to some embodiments of the present invention there is also provided a microarray including the polynucleotide of even numbered SEQ ID NOs or a complementary polynucleotide thereof. The microarray may include a DNA or RNA polynucleotide. The microarray has the same structure as a conventional microarray, except that it includes the polynucleotide of even numbered SEQ ID NOs.

According to some embodiments of the present invention there is also provided a kit including the polynucleotide of even numbered SEQ ID NOs. The kit can include a reagent for polymerization, for example, dNTP, various polymerization enzymes, a colorizing agent, etc., in addition to the polynucleotide of even numbered SEQ ID NOs. The kit can be used in diagnosis of cardiovascular disease, such as heart failure.

According to some embodiments of the present invention there is also provided a method of diagnosing cardiovascular disease, the method including: obtaining nucleic acid from a individual; and determining a nucleotide sequence of a polymorphic site of at least one polynucleotide selected from the group consisting of polynucleotides of even numbered SEQ ID NOs and their complementary polynucleotides. The method of diagnosing cardiovascular disease may further include deciding that the risk of cardiovascular disease is high when the nucleotide sequence of the polymorphic site is the same as at least one of risk alleles according to the sequences of the even numbered SEQ ID NOs.

The obtaining of nucleic acid from an individual can be carried out by a conventional DNA isolation method. For example, nucleic acid can be obtained by amplifying a target nucleic acid through polymerase chain reaction (PCR) and purifying the amplified product. In addition, LCR (Wu and Wallace, Genomics 4, 560 (1989), Landegren et al., Science 241, 1077 (1988)), transcription amplification (Kwoh et al., Proc. Natl. Acad. Sci. USA 86, 1173 (1989)), self-sustained sequence replication (Guatelli et al., Proc. Natl. Acad. Sci. USA 87, 1874 (1990)), and nucleic acid sequence based amplification (NASBA) can be used. Last two methods are associated with an isothermal reaction based on isothermal transcription and produce 30 or 100 times amplified single-strand RNA and double-strand DNA.

In an embodiment of the method, the determining nucleotide sequence of the polymorphic site includes hybridizing the nucleic acid sample onto a microarray on which a polynucleotide for diagnosis or treatment of cardiovascular disease comprising at least 10 contiguous nucleotides selected from the group consisting of nucleotide sequences of even numbered SEQ ID NOs and comprising the nucleotide of the polymorphic site, or a complementary polynucleotide thereof, is immobilized; and detecting the hybridization result.

The method of preparing a microarray by immobilizing a probe polynucleotide on a substrate is well known in the art. The immobilization of the probe polynucleotide associated with cardiovascular disease on a substrate can also be easily performed using a conventional technology. Also, the hybridization of nucleic acid on the microarray and the detection of the hybridisation result are well known in the art. For example, the nucleic acid sample is labelled with a fluorescent material, for example, a labelling material capable of generating detectable signals including Cy3 and Cy5, and then is hybridised on the microarray, followed by detecting signals generated from the labelling material.

In another embodiment, the method may further include determining that the individual belongs to a risk group having high probability of cardiovascular disease when the determined nucleotide sequence of the polymorphic site corresponds to the at least one polymorphic site selected from the group consisting of even numbered SEQ ID NOs in which nucleotides of the polymorphic sites are A, C, A, G and A, respectively. It can be determined that when many nucleic acid sequences having the risk allele are detected in an individual, the probability of belonging to a risk group is high.

According to some embodiments of the present invention there are also provided methods of diagnosing a susceptibility to cardiac disease in an individual comprising detection of a haplotype in a cardiac disease risk gene that is more frequently present in an individual having cardiac disease (affected), compared to the frequency of its presence in a healthy individual (control), wherein the presence of the haplotype is indicative of a susceptibility to cardiac disease.

Another non-limiting, illustrative application of the current invention is diagnosis of a molecular subtype of cardiac disease in a subject. Molecular diagnosis methods and kits of this embodiment of the present invention can be applied to a person having cardiac disease and/or to family members. In one preferred embodiment, molecular subtype of cardiac disease in an individual is determined to provide information of the molecular etiology of cardiac disease. When the molecular etiology is known, better diagnosis and prognosis of cardiac disease can be made and efficient and safe therapy for treating cardiac disease in an individual can be selected on the basis of this cardiac disease subtype. For example, the drug that is likely to be effective can be selected without (or with minimal) trial and error. Physicians may use the information on genetic risk factors with or without known clinical risk factors to convince particular patients to adjust their life style and manage cardiac disease risk factors and select intensified preventive and curative interventions for them.

In other embodiments, biomarker information obtained from methods and kits for determining molecular subtype of cardiac disease in an individual is for monitoring the effectiveness of their treatment. In one embodiment, methods and kits for determining molecular subtype of cardiac disease are used to select human subjects for clinical trials testing cardiac drugs. The kits provided for diagnosing a molecular subtype of cardiac disease in an individual comprise wholly or in part protocol and reagents for detecting one or more biomarkers and interpretation software for data analysis and cardiac disease molecular subtype assessment.

The diagnostic assays and kits of the invention may further comprise a step of combining non-genetic information with the biomarker data to make risk assessment, diagnosis or prognosis of cardiac disease. Useful non-genetic information comprises age, gender, smoking status, physical activity, waist-to-hip circumference ratio (cm/cm), the subject family history of cardiac disease, obesity, hypertriglyceridemia, low HDL cholesterol, HT and elevated BP. The detection method of the invention may also further comprise a step determining total cholesterol, HDL cholesterol, LDL cholesterol, triglyceride, or C-reactive protein concentration.

In diagnostic assays determination of the nucleotides present in one or more polymorphisms of this invention, including SNPs, in an individual's nucleic acid can be performed by any method or technique which can accurately determine nucleotides present in a polymorphic site. Numerous suitable methods have been described in the art [see e.g. 87,88], these methods include, but are not limited to, hybridization assays, ligation assays, primer extension assays, enzymatic cleavage assays, chemical cleavage assays and any combinations of these assays. The assays may or may not include PCR, solid phase step, a microarray, modified oligonucleotides, labeled probes or labeled nucleotides and the assay may be multiplex or singleplex. As it is obvious in the art the nucleotides present in a polymorphic site can be determined from either nucleic acid strand or from both strands.

In another embodiment of the invention, a susceptibility to cardiac disease is assessed from transcription products of one or more cardiac disease associated genes. Qualitative or quantitative alterations in transcription products can be assessed by a variety of methods described in the art, including e.g. hybridization methods, enzymatic cleavage assays, RT-PCR assays and microarrays. A test sample from an individual is collected and the alterations in the transcription of cardiac disease associated genes are assessed from the RNA molecules present in the sample. Altered transcription is diagnostic for a susceptibility to cardiac disease.

"Probes" or "primers" are oligonucleotides that hybridize in a base-specific manner to a complementary strand of nucleic acid molecules. By "base specific manner" is meant that the two sequences must have a degree of nucleotide complementarity sufficient for the primer or probe to hybridize to its specific target. Accordingly, the primer or probe sequence is not required to be perfectly complementary to the sequence of the template. Non-complementary bases or modified bases can be interspersed into the primer or probe, provided that base substitutions do not inhibit hybridization. The nucleic acid template may also include "non-specific priming sequences" or "nonspecific sequences" to which the primer or probe has varying degrees of complementarity. Probes and primers may include modified bases as in polypeptide nucleic acids. Probes or primers typically comprise about 15, to 30 consecutive nucleotides present e.g. in human genome and they may further comprise a detectable label, e.g., radioisotope, fluorescent compound, enzyme, or enzyme co-factor.

Probes and primers to a SNP described herein are described herein and/or can easily be designed using the flanking nucleotide sequences assigned to a SNP rs ID and standard probe and primer design tools. Primers and probes for other types of polymorphisms are also described herein and/or could easily be designed by one of ordinary skill in the art. Primers and probes for SNPs and/or other polymorphisms described herein can be used in risk assessment as well as molecular diagnostic methods and kits according to at least some embodiments of the present invention.

Diagnostic test kits (e.g. reagent kits) according to at least some embodiments of the present invention comprise reagents, materials and protocols for assessing one or more biomarkers, and instructions and software for comparing the biomarker data from a subject to biomarker data from healthy and diseased people to make risk assessment, diagnosis or prognosis of cardiac disease. Useful reagents and materials for kits include, but are not limited to PCR primers, hybridization probes and primers as described herein (e.g., labeled probes or primers), allele-specific oligonucleotides, reagents for genotyping SNP markers, reagents for detection of labeled molecules, restriction enzymes (e.g., for RFLP analysis), DNA polymerases, RNA polymerases, DNA ligases, marker enzymes, antibodies which bind to altered or to non-altered (native) cardiac disease risk gene encoded polypeptide, means for amplification of nucleic acids fragments from one or more cardiac disease risk genes described herein, means for analyzing the nucleic acid sequence of one or more cardiac disease risk genes or fragments thereof, or means for analyzing the sequence of one or more amino acid residues of cardiac disease risk gene encoded polypeptides, etc. In one embodiment, kit for diagnosing susceptibility cardiac disease comprises primers and reagents for detecting the nucleotides present in one or more polymorphisms described herein in an individual's nucleic acid.

Various types of biological samples may optionally be used with the polymorphisms of the present invention, for the diagnosis and/or prognosis of heart disease in a subject. Non-limiting examples of such sample types are described in greater detail below for the purpose of illustration only.

According to preferred embodiments of the present invention, examples of suitable biological samples which may optionally be used with preferred embodiments of the present invention include but are not limited to blood, serum, plasma, blood cells, urine, sputum, saliva, stool, spinal fluid or CSF, lymph fluid, the external secretions of the skin, respiratory, intestinal, and genitourinary tracts, tears, milk, neuronal tissue, lung tissue, any human organ or tissue, including any tumor or normal tissue, any sample obtained by lavage (for example of the bronchial system or of the breast ductal system).

Diagnosis of a disease according to at least some embodiments of the present invention can be effected by determining a polymorphism in a biological sample obtained from the subject, wherein such determination can be correlated with predisposition to, or presence or absence of the disease. It should be noted that a "biological sample obtained from the subject" may also optionally comprise a sample that has not been physically removed from the subject.

Numerous well known tissue or fluid collection methods can be utilized to collect the biological sample from the subject in order to detect the polymorphism in the subject.

Examples include, but are not limited to, fine needle biopsy, needle biopsy, core needle biopsy and surgical biopsy (e.g., brain biopsy), and lavage. Regardless of the procedure employed, once a biopsy/sample is obtained the level of the variant can be determined and a diagnosis can thus be made.

### EXAMPLES

Reference is now made to the following examples, which together with the above description, illustrate the invention in a non limiting fashion.

### Example 1: Association between AT1R polymorphism and heart failure

### Methods

### Study population

134 consecutive HF patients in a specialized HF center and 200 ethnically matched healthy control subjects who had no history or evidence of heart disease were studied. The HF patients had symptomatic systolic HF (echocardiographic LV ejection fraction <45%) for at least 3 months prior to recruitment. Etiology of HF was classified as ischemic or non-ischemic, based on a history of myocardial infarction and/or coronary angiography which were in keeping with the findings of reduced LV systolic function.

Clinical and laboratory data were recorded and blood samples were obtained for genotypic analysis. Patients were followed over a period of 30 months, or up to an end point of death. Patients and controls were ethnically matched Israeli Caucasians, with an equivalent ratio of Ashkenazi and non-Ashkenazi descent. The study was approved by the Institution Review Board (Helsinki committee) of the Lady Davis Carmel Medical Center, and all patients gave written informed consent before inclusion in the study and the start of any study related procedures.

### Genotyping for AT1R Polymorphism

Genomic DNA was extracted from peripheral blood leukocytes using a standard protocol [89]. Subjects were genotyped for the AT1R, using the polymerase chain reaction-restriction fragment length polymorphism (PCR-RFLP) approach. AT1R PCR fragments (404-bp length) encompassing the A1166C polymorphism were amplified from ≈20 ng of each DNA sample used as template in 20µl polymerase chain reactions (PCR) containing 0.2U Taq polymerase, 1×concentration of the supplied buffer, 0.2mmol/L concentration of each deoxynucleotide triphosphate, and 10 pmol of each of the following primers: AGAAGCCTGCACCATGTTTTGAG (sense) and CCTGTTGCTCCTCTAACGATTTA (antisense). The initial denaturation at 950C for 5 minutes was followed by 35 cycles of 940C for 30 seconds, 590C annealing for 30 seconds, and 650C elongation for 45 seconds. Then, 5µl of AT1R reaction was digested with 5 U of restriction endonuclease Dde I in the supplied (New England Biolabs, MA, USA) for 2 hours at 370C. In the presence of the 1166C allele, the 404-bp PCR product was cut into 2 fragments of 118 and 286 bp in length.

### Data analysis

The SPSS statistical package version 13.0 was used to perform all statistical evaluation (SSPS Inc., Chicago, IL, USA). A Chi-squared test was used to examine observed genotype frequencies in terms of the Hardy-Weinberg equilibrium and to compare the genotype frequencies between patients and controls. Genotype subtype comparisons were made by ANOVA and the Kruskal-Wallis test (asymmetrical data distribution). Continuous variables were compared by genotypes group by linear analysis of variance (ANOVA). Stepwise multiple linear regression analysis was used to evaluate whether the different AT1R alleles carried by each patient had statistical influence on clinical and laboratory parameters. Event-free survival was compared by genotype class by Kaplan-Meier log rank analysis. Multivariate stepwise logistic regression model was used for assessment of the dominant variable effecting mortality. Asymmetrically distributed variables were log transformed before regression analysis. Continuous data are presented as mean ± SD. Square multiple correlation coefficients (r²) were calculated.

### Results

### Clinical features

The clinical characteristics of the patients are summarized in Table 1.

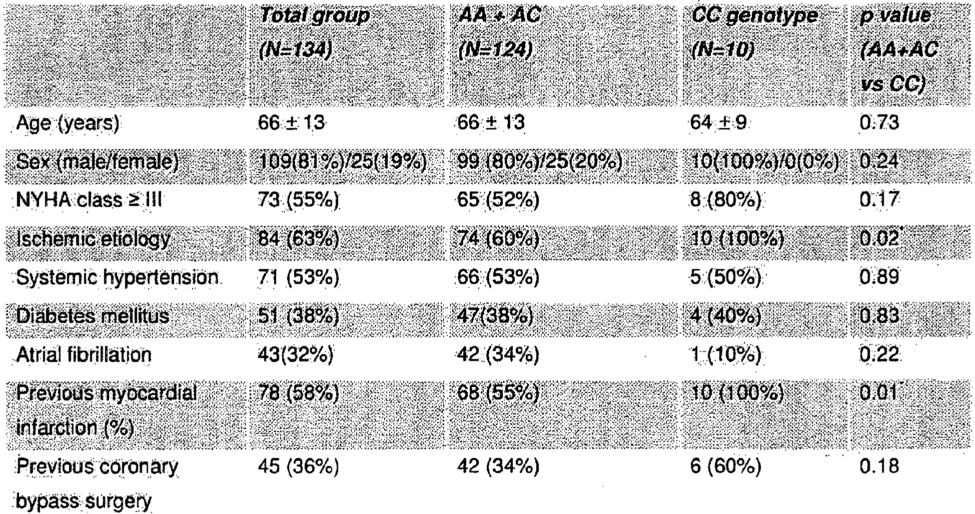

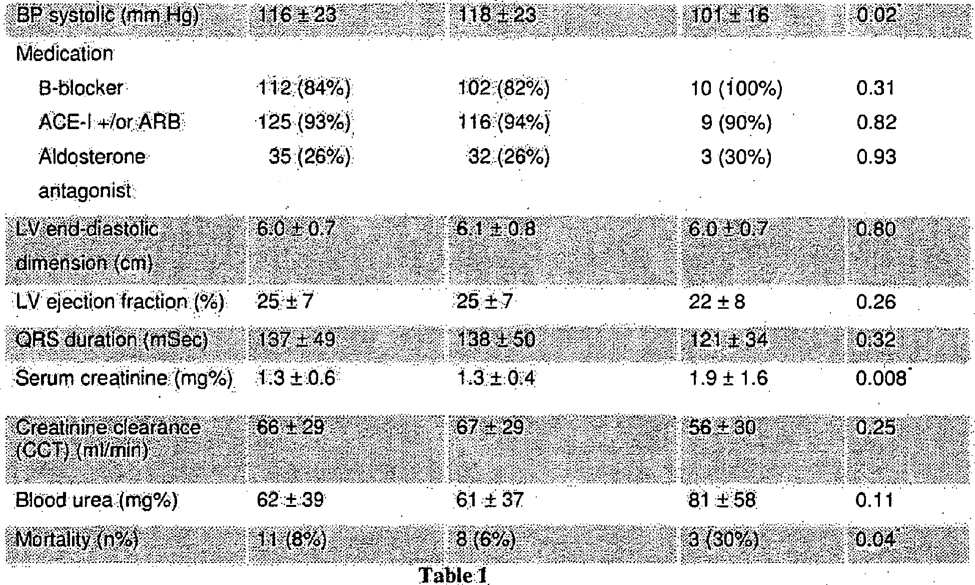

Patients were aged 66±13 years. 109 (81%) were males. The etiology of HF was ischemic heart disease in 84 (63%) patients, 71 (53%) gave a history or were treated for systemic hypertension, 51 (38%) for diabetes mellitus. Atrial fibrillation was present in 43 (32%) patients and mean QRS duration on the surface electrocardiogram was 137±49 milliseconds. Echocardiographic left ventricular (LV) end-diastolic dimension (6.0±0.7 cm) was increased and ejection fraction (EF) reduced (25±7%). Treatment included angiotensin converting enzyme inhibitor (ACEI) and/or angiotensin II receptors blockers (ARB) in 125 (93%) patients, aldosterone antagonists in 35 (26%) patients, and beta blockers in 112 (84%) patients.

Patients were all considerably disabled and 55% were in Functional Class 3 or 4 (New York Heart Association, NYHA). Over the course of follow-up, there were 11 (8%) deaths, 9 due to HF and 2 due to fatal arrhythmia.

### Genotype distribution

The data on allele and genotype frequencies in patients and controls is shown in Table 2.

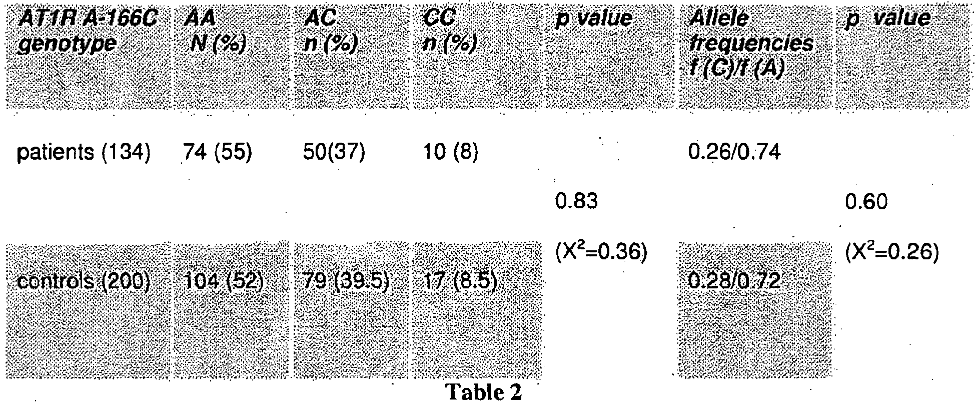

There was no deviation from Hardy-Weinberg equilibrium, in either the HF patients group (allele frequency A/C = 0.74/0.26, expected genotype frequencies % AA/AC/CC = 54%/39%/7%, X²=0.09, p = 0.95), or the control group (allele frequency A/C = 0.72/0.28, expected genotype frequencies % AA/AC/CC = 52%/40%/8%, X²=0.037, p = 0.98). Allele and genotype frequencies did not differ markedly between the groups (Table 2).

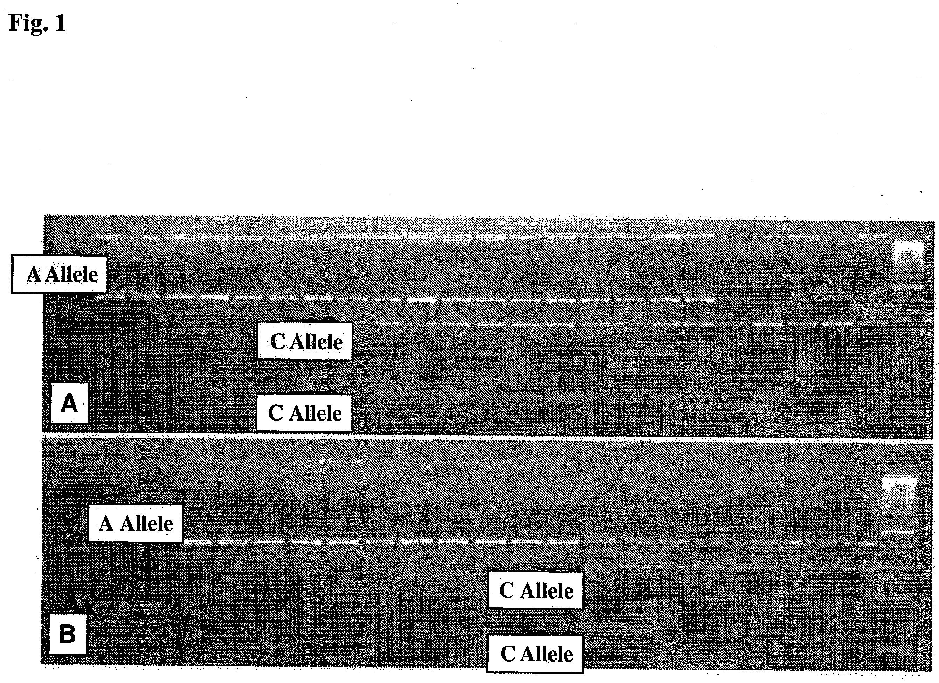

Figure 1 shows results of genotyping of ischemic (upper lanes, A) and non-ischemic (lower lanes, B) HF patients for the A1166C polymorphism of the *AT1R* gene, using polymerase chain reaction (PCR). In the presence of the 1166C allele, the 404-bp PCR product was cut into 2 fragments of 118 and 286 bp in length. Homozygosity for the 1166C allele was observed exclusively in ischemic patients (upper lanes 1-4). Each lane represents genotyping results of each individual patient.

Comparison of HF etiology by AT1R genotype (Figure 1 and Table 3) revealed that all 10 patients who were homozygous for the C allele had ischemic cardiomyopathy (X²=4.82, p=0.02).

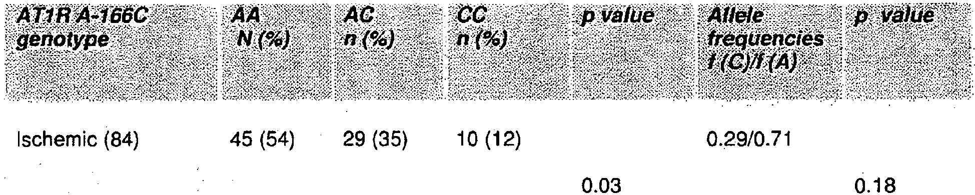

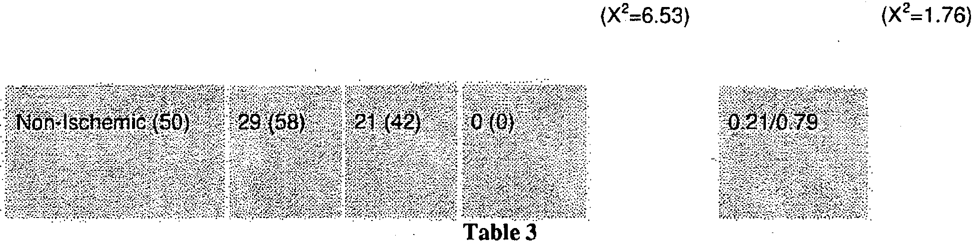

### Haplotype analysis and clinical findings

To determine genotype-phenotype correlations, we compared clinical findings in relation to AT1R genotype subtypes (Table 1). The AT1R CC genotype was associated with a higher serum creatinine level (p=0.008) and lower creatinine clearance. In a multivariate linear regression model which included the following clinical parameters: age, sex, BMI, etiology of ischemic cardiomyopathy, NYHA class, blood pressure, serum sodium level and mean QRS duration, AT1R CC genotype was the second (after age) most powerful determinant of serum creatinine (p=0.005). Most of the homozygous AT1R CC patients (80%) had a lower functional capacity, as manifested by an advanced NYHA class (NYHA≥3). Echocardiographic LV ejection fraction tended to be lower, but with overlap between the 2 groups (NS).

### Mortality and survival analysis

Mortality was greater in patients with C allele (% deaths AA/AC/CC = 5%, 8%, 30%; X²=7.08, p = 0.02). The AT1R CC genotype was associated with poorer survival, while the best survival was among AA and AC patients (% survival at 15/20/30 months = 98%/91%/89%), and the poorest for CC homozygous (% survival 86%/69%/34%) (X²=11.71, p =0.002). Mortality in patients homozygous for the C allele was significantly higher compared to patients with AA and AC subtypes (% deaths CC/AC+AA = 30%, 6%; X²=4.04, p=0.04).

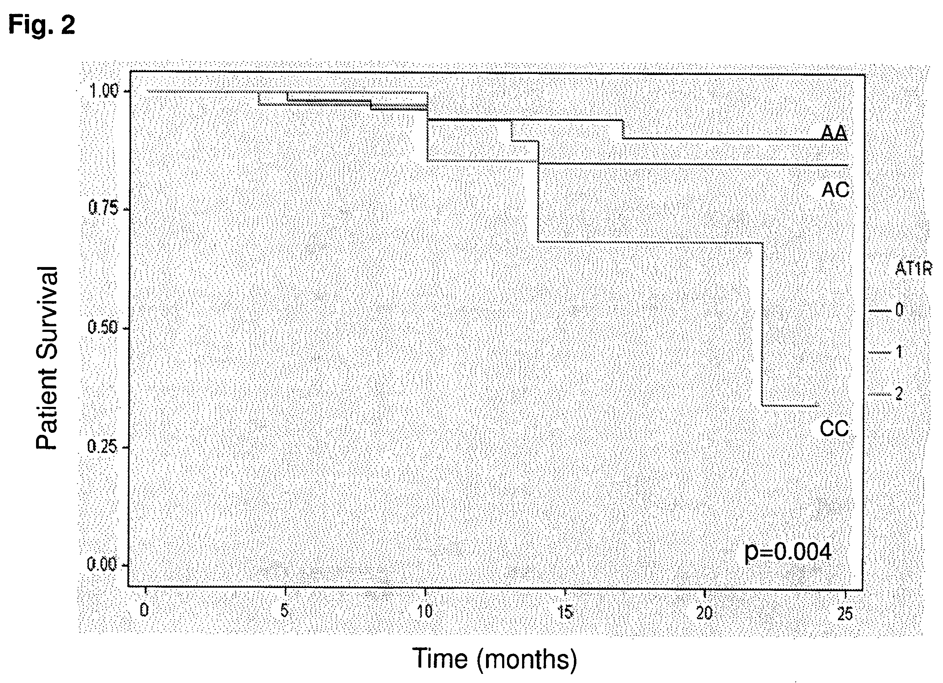

Survival analysis (Kaplan-Meier method) showed that patients with CC genotype had increased mortality and by 30 months a greater than two thirds probability of death, compared to >80% survival in patients with AA or AC alleles (Figure 2).

Since it is already known that the AST1R 1166CC genotype is associated with ischemic heart disease and poor renal function, these parameters have been controlled for in the multivariate analysis. Accordingly, a stepwise logistic regression ,model, adjusting for age, sex, BM1, ischemic/non-ischemic etiology, history of previous myocardial infarction, NYHA class, LVEF, blood pressure, baseline level of serum creatinine, serum sodium level and mean QRS duration, showed that ATIR CC genotype was the most powerful predictor of death (adjusted OR for mortality 6.35, 95% confidence interval 1.49-11.21, p =0.01).

### Example 2: Associations between aldosterone synthase (CYP11B2) T-344C polymorphism and atrial fibrillation

### Methods

### Study Population

The study population consisted of 191 HF patients, followed in a specialized tertiary referral HF center, and 200 ethnically matched healthy control subjects who had no history or evidence of heart disease. All the HF patients had symptomatic systolic HF (left ventricular ejection fraction, LVEF<40%) for at least 3 months prior to recruitment. Etiology of HF was classified as ischemic or non-ischemic, based on a history or lack thereof of myocardial infarction and/or coronary angiography, which were in keeping with the findings of reduced LV systolic function.

Clinical and laboratory data were recorded and blood samples were obtained for genotypic analysis. Echocardiographic measurements of LVEF, left ventricular end diastolic diameter and left atrial (LA) dimension were made. Atrial fibrillation was diagnosed in patients who had atrial fibrillation on at least 2 occasions on a standard 12 lead electrocardiographic recording. The study was approved by the Institution Review Board (Helsinki Committee) of the Lady Davis Carmel Medical Center, and all patients gave written informed consent.

### Genotyping for CYP11B2 polymorphism

Genomic DNA was extracted from peripheral blood leukocytes using a standard protocol [89]. Subjects were genotyped for the *CYP11B2* polymorphism, using the polymerase chain reaction-restriction fragment length polymorphism (PCR-RFLP) approach. *CYP11B2* PCR fragments (537-bp length) encompassing the T-344C polymorphism were amplified from ≈20 ng of each DNA sample used as template in 20µl polymerase chain reactions (PCR) containing 0.2U Taq polymerase, 1×concentration of the supplied buffer (New England Biolabs, MA, USA), 0.2mmol/L concentration of each deoxynucleotide triphosphate, and 10 pmol of each of the following primers: CAGGAGGAGACCCCATGTGA (sense) and CCTCCACCCTGTTCAGCCC (antisense). The initial denaturation at 950C for 5 minutes was followed by 35 cycles of 940C for 30 seconds, 650C annealing for 30 seconds, and 650C elongation for 45 seconds. Then, 5µl of *CYP11B2* reaction was digested with 5 U of restriction endonuclease *Hae* III in the supplied buffer for 2 hours at 370C. The -344T allele lacks a *Hae* III site present in the -344C allele, so - 344T alleles are detected as 273-bp fragments, while the -344C alleles are detected as *Hae* III fragments of 204 and 69-bp. Genotyping was performed by experienced staff. PCR scores by two independent investigators who were blind to subject data con-elated well (r²=0.991).

### Data analysis

The SPSS statistical package version 13.0 was used for statistical analysis (SSPS Inc., Chicago, IL, USA). A Chi-squared test was used to examine observed genotype frequencies in terms of the Hardy-Weinberg equilibrium, to compare the genotype frequencies between patients and controls, and for the analysis of gender ratios, presence of ischemic cardiomyopathy, hypertension, diabetes, and or atrial fibrillation. Genotype subtype comparisons were made by ANOVA and the Kruskal-Wallis test (asymmetrical data distribution). Continuous variables were compared by genotypes group by linear analysis of variance (ANOVA). Stepwise multiple linear regression analysis was used to evaluate whether the different *CYP11B2* alleles carried by each patient had statistical influence on clinical and laboratory parameters. Multivariate stepwise logistic regression model was used for assessment of the dominant variable affecting AF. Asymmetrically distributed variables were log transformed before regression analysis. Continuous data are presented as mean ± SD. Square multiple correlation coefficients (r²) were calculated.

### Results

The clinical characteristics of the patients are summarized in Table 4.
Patients were aged 65±13 years. 145 (81%) patients were males. The etiology of HF was ischemic in 112 (63%) patients, 97 (55%) patients had a history of, or treatment for, systemic hypertension, and 69 (39%) patients had diabetes mellitus. Atrial fibrillation was present in 57 (32%) patients. Mean QRS on the surface electrocardiogram was 138±50 milliseconds. Mean echocardiographic left ventricular ejection fraction (LVEF) was 24±7%.

Treatment included angiotensin converting enzyme inhibitors (ACEI) and/or angiotensin II receptor blockers (ARB) in 164 (92%) patients, aldosterone antagonists in 48 (27%) patients, and beta blockers in 151 (86%) patients. All patients were symptomatic and 97 (55%) patients were in functional class 3 or 4 (New York Heart Association, NYHA). Over a course of 22±7 months follow-up, there were 16 (9%) deaths.

**Table 4**

| ***Clinical characteristics*** | ***HF population*** | ***-344 CT + TT genotype*** | ***-344 CC Genotype*** | ***p value*** |
|---|---|---|---|---|
| | **(*n=191*)** | ***(n=136)*** | ***(n=55)*** | |
| Age (years) | 65 ± 13 | 64 ± 13 | 67 ± 13 | 0.11 |
| Sex (male/female) | 158 (83%)/ | 110 (80%)/ | 48 (87%)/ | 0.29 |
| | 33 (17%) | 26 (20%) | 7(13%) | |
| NYHA class ≥ III | 104 (54%) | 73 (54%) | 31 (56%) | 0.73 |
| Ischemic etiology | 122 (64%) | 84 (62%) | 38 (69%) | 0.33 |
| Systemic hypertension | 103 (54%) | 77 (59%) | 26 (47%) | 0.24 |
| Diabetes mellitus | 75 (39%) | 54 (40%) | 21 (38%) | 0.84 |
| Atrial fibrillation | 63 (33%) | 38 (28%) | 25 (45%) | 0.019 |
| Previous myocardial infarction (%) | 113 (59%) | 79 (58%) | 34 (62%) | 0.63 |
| Previous coronary bypass surgery | 64 (35%) | 45 (33%) | 19 (34.5%) | 0.84 |
| BP systolic (mmHg) Medication (n/%) | 114 ± 23 | 113± 24 | 113 ±21 | 0.93 |
| B-blocker | 165 (86%) | 117 (85%) | 48 (89%) | 0.52 |
| ACE-I +/or ARB | 176 (92%) | 127 (93%) | 49 (91%) | 0.65 |
| Aldosterone antagonist | 54 (28%) | 35 (26%) | 19 (34.5%) | 0.22 |
| LA size (cm) | 4.51 ± 0.57 | 4.49± 0.57 | 4.55 ± 0.59 | 0.55 |
| Mitral regurgitation severity (≥ III) | 48 (25) | 36 (26%) | 12 (22%) | 0.56 |
| LV end-diastolic dimension (cm) | 6.20 ± 0.83 | 6.21 ± 0.84 | 6.14 ± 0.79 | 0.49 |
| LV ejection fraction (%) | 24.1 ± 6.5 | 23.7 ± 6.6 | 24.8 ± 6.2 | 0.29 |
| QRS duration (msec) | 138 ± 46 | 135 ± 41 | 145 ± 56 | 0.27 |
| Creatinine clearance (ml/min) | 67.4 ± 30.2 | 67.0 ± 31.1 | 68.3 ± 27.9 | 0.79 |
| Mortality (%) | 17(9%) | 15(11%) | 2 (4%) | 0.10 |

The data on allele and genotype frequencies in patients and controls are shown in Table 5. There was no deviation from Hardy-Weinberg equilibrium, in either the HF patient group (allele frequency T/C = 0.48/0.52, expected genotype frequencies %TT/TC/CC = 23%/50%/27%, X²=0.42, p = 0.81) or the control group (allele frequency T/C = 0.48/0.52, expected genotype frequencies %TT/TC/CC = 23%/50%/27%, X²=1.78, p = 0.40). Allele and genotype frequencies did not differ markedly between the groups (Table 5), and were similar to previously reported numbers in normal Caucasian populations [48,52,90-92]. Frequencies from dbSNP, using data for HAPMAP (CEPH samples), or a Caucasian group show similarities to the present data.

**Table 5**

| | ***n (%)*** | ***n (%)*** | ***n (%)*** | ***p value*** | ***Allele frequencies*** | ***p value*** |
|---|---|---|---|---|---|---|
| ***CYP11B2-*344C/T genotype** | CC | CT | TT | | f (C)/f (T) 0.52/0.48 | |
| **patients (191)** | 55 (29) | 90 (47) | 46 (24) | 0.15 (X²=3.77) | | 0.87 (X²=0.023) |
| **controls (270)** | 64 (24) | 152 (56) | 54 (20) | | 0.52/0.48 | |

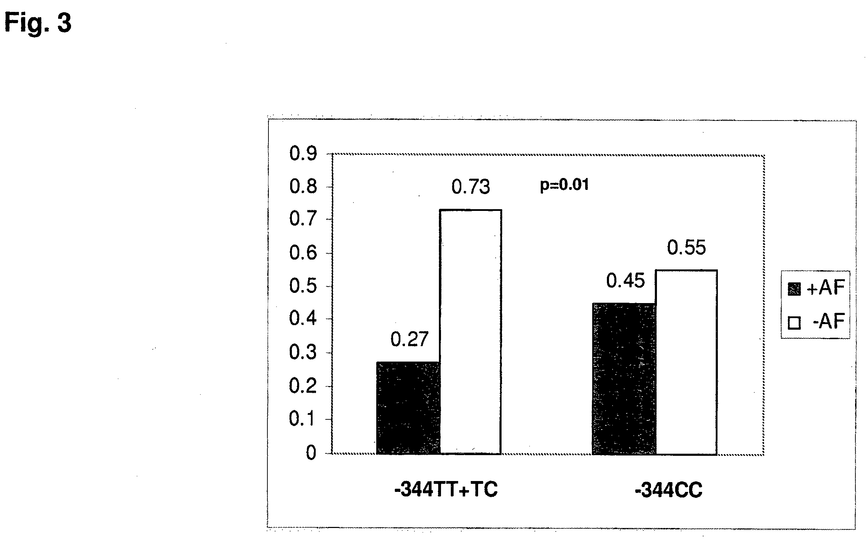

To determine genotype-phenotype correlations, patients' clinical characteristics were compared between *CYP11B2* genotype subtypes. *CYP11B2* polymorphism was not associated with the etiology of HF in these patients. There were no significant differences among the genotype subtypes in terms of gender distribution, history of hypertension or diabetes mellitus, medical therapy regimens, baseline systolic blood pressure levels, LVEF, LA size and the severity of mitral regurgitation (Table 4). However, the presence of AF was associated with *CYP11B2* genotype (Table 4, Figure 3). Compared with the TT and TC genotype subgroup, a significant proportion of patients who were homozygous for the C allele had AF (X2=4.80, p = 0.02). The odds ratio for AF based on the *CYP11B2* -344CC genotype was 2.24 (95% confidence interval 1.14-4.42).

To predict determinants of AF, relevant clinical measurements were included in a multivariate stepwise logistic regression model: age, sex, NYHA class, *CYP11B2* -344CC genotype and echocardiographic measurements of LA size, LVEF and mitral regurgitation severity. The most powerful predictors of AF were LA size and age: odds ratio for AF 5.10 (95% confidence interval 3.23 - 8.05) per 1cm increase in LA size (p=0.0004), and AF increasing 5.38% (95% confidence interval 3.48% - 7.31 %) with each year of increasing age, for a 10 year age difference a 69% (95% confidence interval 41% - 102%) increase (p=0.0039). The *CYP11B2* CC genotype remained an independent powerful predictor of AF (adjusted odds ratio 2.59, 95% confidence interval 1.68 - 3.98, p=0.02). There was no difference in clinical disability (NYHA class) or mortality in regard to *CYP11B2* genotype.

### Example 3: Association between Chymase and Angiotensin - Converting Enzyme Gene Polymorphisms in Chronic Systolic Heart Failure Patients

### Methods

### Study population

A case-control design was used to study 195 consecutive HF patients in a specialized HF center, and 200 population control subjects. Controls [165 (82.5%) males and 35 (17.5%) females, age 26 ± 4 years] were all healthy individuals who had no history of or treatment for coronary artery disease, diabetes mellitus, hypertension or hypercholesterolemia.

The study and control groups were all Israeli residents with an equivalent ratio of Non-Ashkenazi and Ashkenazi descent (2:1). The HF patients had symptomatic systolic HF (echocardiographic LV ejection fraction <40%) for at least 3 months prior to recruitment. Etiology of HF was classified as ischemic or non-ischemic, based on a history or not of myocardial infarction and/or coronary angiography which were in keeping with the findings of reduced LV systolic function. Clinical and laboratory data were recorded and blood samples were obtained for genotypic analysis. Patients were followed over a period of 30 months, or up to an end point of death. The study was approved by the Institution Review Board (Helsinki committee) of the Lady Davis Carmel Medical Center, and all patients gave written informed consent before inclusion in the study.

### Genotyping for ACE and CMA1 polymorphisms

Genomic DNA was extracted from peripheral blood leukocytes using a standard protocol [89]. Genotyping of the *ACE* I/D polymorphism was performed using polymerase chain reaction (PCR) according to the method of Lindpaintner et al.[93]. Genotyping for the *CMA1* 1903G/A polymorphism was conducted using the polymerase chain reaction-restriction fragment length polymorphism (PCR-RFLP) approach, as described by Pfeufer et al.[72]. PCR fragments were amplified from ≈20 ng of each DNA sample used as a template in 20µl polymerase chain reactions (PCR) containing 0.2U Taq polymerase, 1× concentration PCR buffer, 0.2mmol/L of each dNTP, and 10 pmol of each of the following primers: GGAAATGTGAGCAGATAGTGCAGT (*CMA1*-sense) and AATCCGGAGCTGGAGAACTCTTGTC (*CMA1*-antisense), and GCCCTGCAGGTGTCTGCAGCATGT (*ACE*-sense) and GGATGGCTCTCCCCGCCTTGTCTC (*ACE*-antisense).

The initial denaturation at 950C for 5 minutes was followed by 35 cycles of 940C for 30 seconds, 56-580C annealing for 30 seconds, and 650C elongation for 45 seconds. *ACE* I/D genotypes were designated as follows: I/I, a single band of 597-bp; D/I, two bands of 319- and 597-bp; and D/D, a single band of 319-bp. Because the D allele in heterozygous samples is preferentially amplified, there is a tendency to misclassify the *ACE* I/D genotype as the D/D genotype. In order to avoid this misclassification, a second PCR was performed using I-specific primers: TGGGACCACAGCGCCCGCCACTAC (I-specific -sense) and TCGCCAGCCCTCCCATGCCCATAA (I-specific -antisense). This PCR yields a 335-bp fragment only in the presence of the I allele, and no product in sample homozygous for the *D* allele. The *CMA1* PCR fragments (285-bp length) were digested with 10 U of restriction endonuclease *Bst* XI in the supplied buffer (New England Biolabs, MA, USA) for 2 hours at 550C. The -1903A allele lacks a *Bst* XI site present in the -1903G allele, so -1903A alleles are detected as uncut fragments of 285-bp while the -1903G alleles are detected as *Bst* XI fragments of 195 and 90-bp. Genotyping was performed by experienced staff. PCR scores by two independent investigators who were blind to subject data, correlated well (r²=0.991).

### Data analysis

The SPSS statistical package version 13.0 was used for statistical evaluation (SPSS Inc, Chicago IL, USA). A chi square test was used to confirm that observed genotype frequencies were in Hardy-Weinberg equilibrium and to compare the genotype frequencies between patients and controls. Genotype subtypes comparisons were made by ANOVA and the Kruskal-Wallis test (asymmetrical data distribution). Continuous variables were compared by genotypes group by linear analysis of variance (ANOVA). Stepwise multiple linear regression analysis was used to evaluate whether the number of ACE and *CMA1* alleles carried by each patient had statistical influence on clinical and laboratory parameters. Asymmetrically distributed variables were log transformed before regression analysis. Continuous data are presented as mean ± SD. Square multiple correlation coefficients (r²) were calculated. In order to adjust for multiple comparisons, P values were considered significant if <0.01.

### Results

The clinical characteristics of the patients are summarized in Table 6.

**Table 6**

| ***Clinical characteristics*** | ***All patients (n=195)*** | ***Ischemic etiology (n=124)*** | ***Non-ischemic etiology (n=71)*** |
|---|---|---|---|
| Age (years) | 64±13 | 68 ± 11 | 59 ± 14 |
| Sex (male/female) | 162 (83%)/ 33 (17%) | 162 (83%)/ 33 (17%) | 51 (72%)/ 20 (28%) |
| NYHA ≥ III (%) | 105 (54%) | 69 (56%) | 36 (51%) |
| Systemic hypertension (%) | 105 (54%) | 75 (60%) | 30 (42%) |
| Diabetes mellitus (%) | 77 (39%) | 58 (47%) | 19 (27%) |
| Atrial fibrillation (%) | 63 (32%) | 39 (31%) | 24 (34%) |
| Medication (n/%) | | | |
| B-blockers | 167 (87%) | 108 (87%) | 59 (83%) |
| ACE-I +/or ARB | 181 (93%) | 114 (92%) | 67 (94%) |
| Aldosterone antagonists | 56 (29%) | 37 (29%) | 19 (27%) |
| BP systolic (mm Hg) | 114 ± 24 | 112±23 | 117±25 |
| LV end-diastolic dimension (cm) | 6.2 ± 0.8 | 6.2 ± 0.8 | 6.2 ± 0.8 |
| LV ejection fraction (%) | 24 ± 6.5 | 25 ± 6.5 | 22 ± 6.2 |
| QRS duration (msec) | 137 ± 45.5 | 134 ± 40.1 | 143 ± 53.5 |
| Serum creatinine (mg/dL) | 1.35 ± 0.6 | 1.44 ± 0.7 | 1.18 ± 0.4 |
| Creatinine clearance (ml/min) | 67.2 ± 30.2 | 61.0 ± 26.0 | 78.8 ± 34.2 |
| Serum urea (mg/dL) | 63.1 ± 38.6 | 67.8 ± 38.2 | 54.6 ± 38.1 |
| Mortality (%) | 17 (9%) | 12 (10%) | 5 (7%) |

Patients were aged 64±13 years, 162 (83%) were males. The etiology of HF was ischemic heart disease in 124 (64%) patients, 105 (54%) gave a history of or were treated for hypertension, 77 (39%) for diabetes mellitus. Atrial fibrillation was present in 63 (32%) patients and the mean QRS on the surface electrocardiogram was 137±45.5 milliseconds. Mean echocardiographic left ventricular (LV) end-diastolic dimension was 6.2±0.8 cm and ejection fraction (EF) 24±6.5%. Treatment included angiotensin converting enzyme inhibitor (ACEI) and/or angiotensin II receptor blockers (ARB) in 181 (93%) patients, direct aldosterone antagonists in 56 (29%) patients, and beta blockers in 167 (87%) patients. Patients were all considerably disabled and 105 (54%) were in Functional Class 3 or 4 (New York Heart Association, NYHA). Over the course of follow-up, there were 17 (9%) deaths, 9 due to HF and 2 due to fatal arrhythmia.

The data on allele and genotype frequencies in patients and controls are shown in Table 7.

**Table 7**

| ***genotype*** | ***n (%)*** | ***n (%)*** | ***n (%)*** | ***Significance*** |
|---|---|---|---|---|
| ***CMA1-1903G*/*A*** | AA | AG | | |
| | | | | |
| **HF-all (195)** | 52 (27) | 102 (52) | 41 (21) | **P*=0.28*;* X²=2.52 |
| **Ischemic HF (124)** | 36 (29) | 69 (56) | 19 (15) | |
| **Non-Ischemic HF (71)** | 16 (23) | 33 (46) | 22 (31) | ***P*=0.03; X²=6.71 |
| **controls (200)** | 40 (20) | 112 (56) | 48 (24) | |
| | | | | |
| ***ACE* I/D** | II | ID | DD | |
| | | | | |
| **patients (195)** | 32 (16) | 85 (44) | 78 (40) | **P*=0.12; X²=4.21 |
| **Ischemic HF (124)** | 18 (14) | 54 (44) | 52 (42) | |
| **Non-Ischemic HF (71)** | 14 (20) | 31 (43) | 26 (37) | ***P*=0.58; X²=1.06 |
| **controls (200)** | 19 (10) | 93 (46) | 88 (44) | |

For both *ACE* I/D and *CMA1* -1903G/A polymorphisms, there was no deviation from Hardy-Weinberg equilibrium in either the HF patients group (all) (allele frequency *ACE* I/D = 0.38/0.62, expected genotype frequencies % II/ID/DD = 14%/47%/39%, χ²=0.60, *P* = 0.74; Allele frequency *CMA1* A/G = 0.53/0.47, expected genotype frequencies % AA/AG/GG = 28%/50%/22%, χ²=0.25, p=0.87), the ischemic HF patients group (allele frequency *ACE* I/D = 0.36/0.64, expected genotype frequencies % II/ID/DD = 13%/46%/41%, χ²=0.20, *P* = 0.90; Allele frequency *CMA1* A/G = 0.57/0.43, expected genotype frequencies % AA/AG/GG = 32.5%/49%/18.5%, χ²=1.08, *P*=0.58), the non-ischemic HF patients group (allele frequency *ACE* I/D = 0.42/0.58, expected genotype frequencies % II/ID/DD = 17%/49%/34%, χ²=0.47, *P =* 0.78; Allele frequency *CMA1* A/G = 0.46/0.54, expected genotype frequencies % AA/AG/GG = 21%/50%/29%, χ²=0.11, *P*=0.94), or the control group (allele frequency *ACE* I/D = 0.33/0.67, expected genotype frequencies % II/ID/DD = 11%/44%/45%, χ²=0.38, *P* = 0.82; Allele frequency *CMA1* A/G = 0.48/0.52, expected genotype frequencies % AA/AG/GG = 23%/50%/27%, χ²=1.45, *P* = 0.48).

The subjects' age, gender distribution, and Ashkenazi/ non-Ashkenazi ancestry did not differ by either *ACE* I/D or *CMA1* -1903G/A genotypes. For both *ACE* I/D and *CMA1* -1903G/A polymorphisms, frequencies from dbSNP, using data for mixed European or Caucasian populations, show similarities to the present data. Allele (and genotype) frequencies of the whole cohort of HF patients were similar to that amongst healthy controls (Table 7). However, *CMA1* -1903G/A allele and genotype frequencies of the non-ischemic patients differed significantly from those of ischemic patients (Table 7). Moreover, comparison of HF etiology by *CMA1* genotype revealed that the *CMA1* -1903GG genotype was associated with non-ischemic HF etiology (Table 7 and Table 8, χ²=6.67, *P* = 0.009). The odds ratio for the *CMA1* GG genotype in non-ischemic patients was 2.48 (95% confidence interval 1.23-5.00). Importantly, *ACE* I/D polymorphism was not associated with HF etiology in the patients of the present study.

**Table 8**

| ***Clinical characteristics*** | ***AA + AG genotype (n=154)*** | ***GG genotype (n-41)*** | ***p value*** |
|---|---|---|---|
| Age (years) | 65 ± 13 | 60 ± 13 | 0.029 |
| Sex (male/female) | 130 (84%)/24 (16%) | 32 (78%)/9 (22%) | 0.33 |
| NYHA class ≥ III | 86 (56%) | 19 (46%) | 0.27 |
| Ischemic etiology | 105 (68%) | 19 (46%) | 0.009 |
| Systemic hypertension | 89 (58%) | 19 (46%) | 0.18 |
| Diabetes mellitus | 61 (39%) | 17 (42.5%) | 0.81 |
| Atrial fibrillation | 53 (34%) | 10 (24%) | 0.22 |
| Previous myocardial infarction (%) | 91 (59%) | 23 (56%) | 0.69 |
| Previous coronary bypass surgery | 52 (34%) | 15 (36%) | 0.78 |
| Medication (n/%) | | | |
| B-blocker | 134 (87%) | 36 (87.5%) | 0.91 |
| ACE-I +/or ARB | 142 (92%) | 39 (96 %) | 0.76 |
| aldosterone antagonist | 45 (29%) | 11 (27.5%) | 0.83 |
| BP systolic (mm Hg) | 115 ± 24 | 110 ± 20 | 0.24 |
| LV end-diastolic dimension (cm) | 6.2 ± 0.8 | 6.2 ± 0.7 | 0.90 |
| LV ejection fraction (%) | 25 ± 6.5 | 21 ± 6.1 | 0.005 |
| QRS duration (msec) | 141 ± 48 | 125 ± 33 | 0.02 |
| Serum creatinine (mg/dL) | 1.3 ± 0.5 | 1.4 ± 0.9 | 0.53 |
| Creatinine clearance (ml/min) | 66.4 ± 0.0 | 70.3 ± 31.2 | 0.47 |
| Serum urea (mg/dL) | 62.5 ± 6.8 | 65.6 ± 45.2 | 0.69 |
| Mortality (%) | 15 (10%) | 2 (5%) | 0.32 |

To determine genotype-phenotype correlations, patients' clinical characteristics between genotype subtypes of each polymorphism were compared. Compared with the AA and AG genotype subgroup, homozygous *CMA1* GG patients had lower values of left ventricular ejection fraction (*P* = 0.005) (Table 8). Multivariate stepwise linear regression, adjusted for age, previous myocardial infarction, NYHA class, echocardiographic LV dimension and QRS duration on the surface electrocardiogram, showed that *CMA1* GG genotype (after echocardiographic LV dimension) was the most powerful independent predictor of reduced systolic function (adjusted odds ratio 32.6, 95% confidence interval *11.9 - 89.3, P* = 0.0007). The *ACE* D allele was not associated with the phenotypic expression of HF in our patients. It should be noted that no difference was found in clinical disability (NYHA class) and mortality in regard to either *CMA1* or *ACE* gene polymorphism.

### Example 4: Impact of Different Cytokines in Heart Failure (HF) Patients

As described above, different cytokines were shown to have different effects in HF patients. Without wishing to be limited by a single hypothesis, it is believed that these results support the potential diagnostic and prognostic use of polymorphism of inflammatory activity related genes, particularly (but not exclusively) with regard to polymorphisms of the cytokines themselves.

Interleukin-10 (IL-10) is an anti-inflammatory cytokine and consequently is considered by many to have a protective role in heart failure, as opposed to the "notorious" tumor necrosis factor-alpha (TNF-alpha). In the current study the hypothesis of the possible beneficial impact of IL-10 on mortality in systolic heart failure (HF) patients in relation to their circulating TNF-alpha levels was tested.
Methods: Circulating levels of IL-10 and TNF-alpha in 67 ambulatory systolic HF patients (aged 65±13) years were measured in the plasma via a blood test.
Results: Mortality was or tended higher in patients with higher levels (above median level) of circulating TNF-alpha (9/23, 39% vs 6/44, 14%, p=0.02) or IL-10 (10/34, 30% vs 5/33, 15%, p=0.10). However, mortality was highest in the sub-set of patients with elevation of both markers above median (7/16, 44% vs 8/51, 16%, p=0.019). Elevation of both markers was associated with more than a threefold hazard ratio for mortality (HR 3.67, 95% CI 1.14-11.78).

Tables 9-11 show information about the patients and also the relationship between the levels of various cytokines and various other clinical parameters of the patients.

**Patient characteristics (n=67) - Table 9**

| | |
|---|---|
| Age (years) m±sd | 65 ± 13 |
| Sex (male/female) | 58/9 |
| Left ventricular ejection fraction (%) m±sd | 25 ± 7 |
| Left ventricular end-diastolic diameter (mm) m±sd | 61 ± 7 |
| New York Heart association class ≥ III n (%) | 38 (57) |
| Ischemic etiology n (%) | 41 (61) |
| History of systemic hypertension n (%) | 32 (48) |
| Diabetes mellitus n (%) | 26 (39) |
| Atrial fibrillation n (%) | 22 (33) |
| Body mass index (kg/m²) m±sd | 29 ± 6 |
| Systolic blood pressure (mmHg) m±sd | 116 ± 21 |
| Medication n (%): | |
| Beta-blocker | 64 (95) |
| Angiotensin converting enzyme inhibitor(ACEI) | |
| +/or Angiotensin II receptor blocker (ARB) | 60 (89) |
| Aldosterone antagonist | 17 (25) |
| QRS duration (msec) m±sd | 135±48 |
| Chronic renal failure, (Cr>2 mg/dL) n (%) | 7(10) |
| Mortality n (%) | 15(22) |

**Relation between TNF-alpha, IL-10 and their combination and clinical parameters - Table 10**

| | **TNF-alpha** | | | **IL-10** | | | **TNF alpha and IL-10 combined** | | |
|---|---|---|---|---|---|---|---|---|---|
| ***Clinical parameter*** | ***Group 1 (n-44)*** | ***Group* 2 *(n=23)*** | **p value** | ***Group 1 (n=34)*** | ***Group 2 (n=33)*** | **p value** | ***Group 1 (n=51)*** | ***Group* 2 *(n=16)*** | **p value** |
| Age (years) | 63(4)* | 68(12) | 0.1 | 64(12) | 65(15) | 0.8 | 64(13) | 68(13) | 0.3 |
| New York Heart Association Class ≥III n (%) | 25(57) | 13(56) | 0.9 | 19(56) | 19(58) | 0.9 | 29(57) | 9(56) | 1.0 |
| Body mass index kg/m² | 29(6) | 29(5) | 0.6 | 28(5) | 30(6) | 0.4 | 29(6) | 30(6) | 0.6 |
| Systolic blood pressure (mmHg) | 117(23) | 114(16) | 0.6 | 119(22) | 113(20) | 0.2 | 118(22) | 110(15) | 0.2 |
| Left ventricular ejection fraction (%) | 25(7) | 25(6) | 0.7 | 26(7) | 25(7) | 0.5 | 26(7) | 23(5) | 0.08 |
| Left ventricular end-diastolic diameter (mm) | 62(6) | 61(8) | 0.5 | 61(7) | 63(7) | 0.3 | 61(7) | 62(8) | 0.7 |
| QRS duration (msec) | 134(52) | 137(42) | 0.8 | 130(38) | 140(57) | 0.4 | 132(50) | 143(42) | 0.5 |
| Six minute walk (meters) | 244(168) | 224(94) | 0.5 | 230(160) | 244(134) | 0.7 | 235(163) | 243(79) | 0.8 |
| Mortality n (%) | 6(14) | 9(39) | 0.02 | 5(15) | 10(30) | 0.1 | 8(16) | 7(44) | 0.019 |

| | | | | | | | | | |
|---|---|---|---|---|---|---|---|---|---|
| ****mean±1SD*/**inter quartile range*** | | | | | | | | | |

**Relation between TNF-alpha, IL-10 and their combination and laboratory parameters - Table 11**

| | **TNF-alpha** | | | **IL-10** | | | **TNF alpha - IL-10 combined** | | |
|---|---|---|---|---|---|---|---|---|---|
| ***Parameter*** | ***Group 1 n=44*** | ***Group 2 n=23*** | **p value** | ***Group 1 n=34*** | ***Group 2 n=33*** | **p value** | ***Group* 1 *n=51*** | ***Group 2 n=16*** | **p value** |
| NT- pro BNP (pg/ml) | 1139* | 3666 | 0.001* | 1403 | 2075 | 0.3 | 1292 | 4094 | 0.004 |
| Matrix metalloprotease-9 (ng/ml) | 467(2) | 79(2) | 0.006 | 53(2) | 592(2) | 0.6 | 508(2) | 762(2) | 0.06 |
| Troponin T(ng/ml) | 0.0(0.00*) | 0.02(0.05*) | 0.001 | 0.0(0.00*) | 0.0(0.04*) | 0.1 | 0.0(0.00 *) | 0.02(0.05*) | 0.002' |
| Hs-CRP (mg/dl) | 0.8(5) | 0.6(3) | 0.6 | 0.8(4) | 0.7(4) | 1.0 | 0.8(5) | 0.6(2) | 0.3 |
| Hemoglobin (gm/dl) | 13(1.7) | 11.8(1.6) | 0.007 | 12.8 (1.8) | 12.4(1.7) | 0.3 | 12.7(1.8 ) | 12.2(1.6) | 0.3 |
| Total cholesterol (mg/dl) | 155(36) | 135(32) | 0.033 | 147(37) | 150(36) | 0.7 | 15(37) | 138(33) | 0.2 |
| Serum creatinine (mg %) | 1.1(0.3) | 1.9(1.0) | 0.002 | 1.2(0.5) | 1.5(0.9) | 0.1 | 1.2(0.4) | 1.9(1.1) | 0.026 |
| CCT (ml/min) | 81(34) | 54(32) | 0.003 | 74(32) | 69(38) | 0.6 | 77(34) | 56 (34) | 0.036 |

| | | | | | | | | | |
|---|---|---|---|---|---|---|---|---|---|
| ****mean±1SD*/**inter quartile range*** | | | | | | | | | |

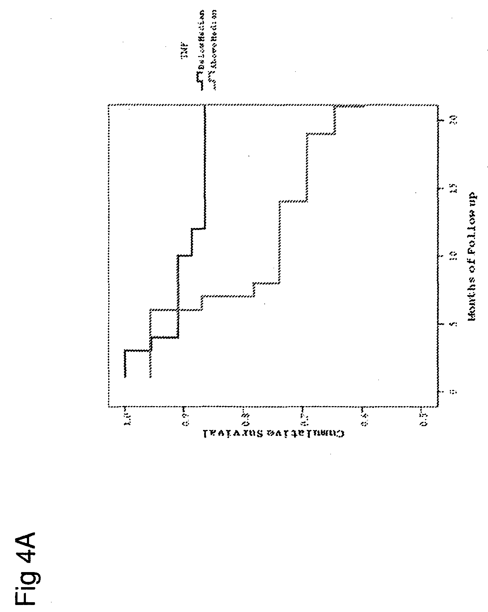

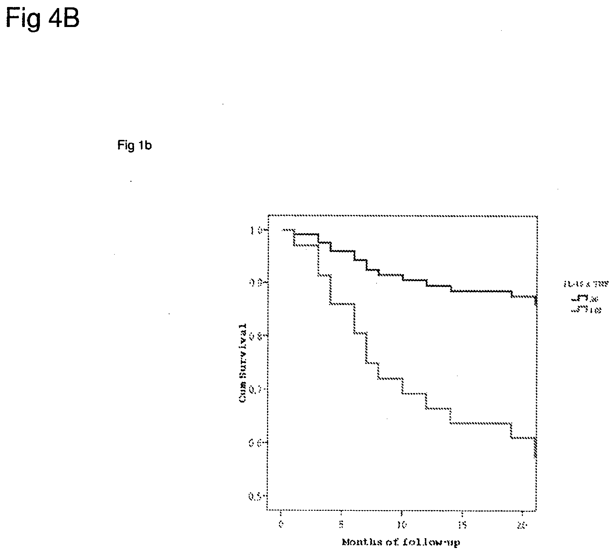

Figure 4A shows Kaplan-Meier survival curves according to circulating TNF- alpha levels (below and above median). Survival was reduced in patients with higher TNF-alpha levels (p=0.02). Figure 4B shows Cox proportional hazard ratio curves according to combined circulating TNF-alpha and IL-10 levels (both below and above median). Survival was reduced in patients with higher TNF/IL-10 levels (p=0.03) These results show that elevated circulating IL-10 levels in systolic HF patients do not have a protective counterbalance effect on mortality. Moreover, patients with elevated IL-10 and TNF-alpha had significantly higher mortality, suggesting that in fact such cytokines, and particularly polymorphisms of such cytokines, may have a significant biological effect which can be used for diagnostic and prognostic purposes.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. In addition, citation or identification of any reference in this application shall not be construed as an admission that such reference is available as prior art to the present invention.

### References

1. Fatkin D, Otway R, Vandenberg JI. Genes and atrial fibrillation: a new look at an old problem. Circulation 2007;116:782-792.
2. Bassett MH, White PC, Rainey WE. The regulation of aldosterone synthase expression. Mol Cell Endocrinol 2004;217:67-74.
3. De Ferrari GM, Klersy C, Ferrero P, Fantoni C, Salerno-Uriarte D, Manca L, Devecchi P, Molon G, Revera M, Cumis A, Sarzi Braga S, Accardi F, Salerno-Uriarte JA. Atrial fibrillation in heart failure patients: prevalence in daily practice and effect on the severity of symptoms. Data from the ALPHA study registry. Eur J Heart Fail 2007;9:502-509.
4. Fedak PW, Verma S, Weisel RD, Li RK: Cardiac remodeling and failure: from molecules to man (Part I). Cardiovasc Pathol 2005;14:1-11.
5. Goette A, Staack T, Rocken C, Amdt M, Geller JC, Huth C, Ansorge S, Klein HU, Lendeckel U. Increased expression of extracellular signal-regulated kinase and angiotensin-converting enzyme in human atria during atrial fibrillation. J Am Coll Cardiol 2000;35:1669-1677.
6. Stolarz K, Staessen JA, Kawecka-Jaszcz K, Brand E, Bianchi G, Kuznetsova T, Tikhonoff V, Thijs L, Reineke T, Babeanu S, Casiglia E, Fagard R, Filipovsky J, Peleska J, Nikitin Y, Struijker-Boudier H, Grodzicki T: Genetic variation in CYP11B2 and AT1R influences heart rate variability conditional on sodium excretion. Hypertension 2004;44:156-162.
7. Van Den Berg MP, Crijns HJGM, Van Veldhuisen DJ, al. e. Effects of lisinopril in patients with heart failure and chronic atrial fibrillation. J Cardiac Failure 1995;1:355-364.
8. Pedersen OD, Bagger H, Kober L, Torp-Pedersen C. Trandolapril reduces the incidence of atrial fibrillation after acute myocardial infarction in patients with left ventricular dysfunction. Circulation 1999;100:376-380.
9. Madrid AH, Bueno MG, Rebollo JM, Marin I, Pena G, Bernal E, Rodriguez A, Cano L, Cano JM, Cabeza P, Moro C. Use of irbesartan to maintain sinus rhythm in patients with long-lasting persistent atrial fibrillation: a prospective and randomized study. Circulation 2002;106:331-336.
10. Duncan JA, Scholey JW, Miller JA: Angiotensin II type 1 receptor gene polymorphisms in humans: physiology and pathophysiology of the genotypes. Curr Opin Nephrol Hypertens 2001;10:111-116.
11. van Geel PP, Pinto YM, Voors AA, Buikema H, Oosterga M, Crijns HJ, van Gilst WH. Angiotensin II type 1 receptor A1166C gene polymorphism is associated with an increased response to angiotensin II in human arteries. Hypertension. 2000 Mar;35(3):717-21).
12. Mettimano M, Romano-Spica V, Ianni A, Specchia M, Migneco A, Savi L: AGT and AT1R gene polymorphism in hypertensive heart disease. Int J Clin Pract 2002;56:574-577.
13. Makeeva OA, Puzyrev KV, Pavliukova EN, Koshel'skaia OA, Golubenko MV, Efimova EV, Kucher AN, Tsimbaliuk IV, Karpov RS, Puzyrev VP: [ACE and AGTR1 genes polymorphisms in left ventricular hypertrophy pathogenesis in humans]. Mol Biol (Mosk) 2004;38:990-996.
14. Tabara Y, Kohara K, Nakura J, Miki T: Risk factor-gene interaction in carotid atherosclerosis: effect of gene polymorphisms of renin-angiotensin system. J Hum Genet 2001;46:278-284.
15. Alvarez R, Reguero JR, Batalla A, Iglesias-Cubero G, Cortina A, Alvarez V, Coto E: Angiotensin-converting enzyme and angiotensin II receptor 1 polymorphisms: association with early coronary disease. Cardiovasc Res 1998;40:375-379.
16. Canavy I, Henry M, Morange PE, Tiret L, Poirier O, Ebagosti A, Bory M, Juhan-Vague I: Genetic polymorphisms and coronary artery disease in the south of France. Thromb Haemost 2000;83:212-216.
17. Fatini C, Abbate R, Pepe G, Battaglini B, Gensini F, Ruggiano G, Gensini GF, Guazzelli R: Searching for a better assessment of the individual coronary risk profile. The role of angiotensin-converting enzyme, angiotensin II type 1 receptor and angiotensinogen gene polymorphisms. Eur Heart J 2000;21:633-638.
18. Araujo MA, Goulart LR, Cordeiro ER, Gatti RR, Menezes BS, Lourenco C, Silva HD: Genotypic interactions of renin-angiotensin system genes in myocardial infarction. Int J Cardiol 2005;103:27-32.
19. Buraczynska M, Ksiazek P, Zaluska W, Spasiewicz D, Nowicka T, Ksiazek A: Angiotensin II type 1 receptor gene polymorphism in end-stage renal disease. Nephron 2002;92:51-55.
20. Fabris B, Bortoletto M, Candido R, Barbone F, Cattin MR, Calci M, Scanferla F, Tizzoni L, Giacca M, Carretta R: Genetic polymorphisms of the renin-angiotensin-aldosterone system and renal insufficiency in essential hypertension. J Hypertens 2005;23:309-316.
21. Hsu CC, Bray MS, Kao WH, Pankow JS, Boerwinkle E, Coresh J: Genetic variation of the renin-angiotensin system and chronic kidney disease progression in black individuals in the atherosclerosis risk in communities study. J Am Soc Nephrol 2006;17:504-512.
22. Bettencourt P, Ferreira A, Dias P, Pimenta J, Friões F, Martins L, Cerqueira-Gomes M.Predictors of prognosis in patients with stable mild to moderate heart failure.J Card Fail. 2000 Dec;6(4):306-13/
23. Damman K, Navis G, Voors AA, Asselbergs FW, Smilde TD, Cleland JG, van Veldhuisen DJ, Hillege HLWorsening renal function and prognosis in heart failure: systematic review and meta-analysis.J Card Fail. 2007 Oct;13(8):599-608.
24. Gruchala M, Ciecwierz D, Ochman K, Wasag B, Koprowski A, Wojtowicz A, Dubaniewicz W, Targonski R, Sobiczewski W, Grzybowski A, Romanowski P, Limon J, Rynkiewicz A: Left ventricular size, mass and function in relation to angiotensin-converting enzyme gene and angiotensin-II type 1 receptor gene polymorphisms in patients with coronary artery disease. Clin Chem Lab Med 2003;41:522-528.
25. Andrikopoulos GK, Richter DJ, Needham EW, Tzeis SE, Zairis MN, Gialafos EJ, Vogiatzi PG, Papasteriadis EG, Kardaras FG, Foussas SG, Gialafos JE, Stefanadis CI, Toutouzas PK, Mattu RK: The paradoxical association of common polymorphisms of the renin-angiotensin system genes with risk of myocardial infarction. Eur J Cardiovasc Prev Rehabil 2004,11:477-483.
26. Kuznetsova T, Staessen JA, Thijs L, Kunath C, Olszanecka A, Ryabikov A, Tikhonoff V, Stolarz K, Bianchi G, Casiglia E, Fagard R, Brand-Herrmann SM, Kawecka-Jaszcz K, Malyutina S, Nikitin Y, Brand E: Left ventricular mass in relation to genetic variation in angiotensin II receptors, renin system genes, and sodium excretion. Circulation 2004;110:2644-2650.
27. Andrikopoulos GK, Tzeis SM, Needham EW, Richter DJ, Zairis MN, Gialafos EJ, Kardaras FG, Foussas SG, Stefanadis CI, Toutouzas PK, Mattu R: Lack of association between common polymorphisms in genes of the renin-angiotensin system and mortality after myocardial infarction. Cardiology 2005;103:185-188.
28. Methot J, Hamelin BA, Bogaty P, Arsenault M, Plante S, Poirier P: ACE-DD genotype is associated with the occurrence of acute coronary syndrome in postmenopausal women. Int J Cardiol 2005;105:308-314.
29. Ulgen MS, Ozturk O, Yazici M, Kayrak M, Alan S, Koc F, Tekes S: Association between A/CI 166 gene polymorphism of the angiotensin II type 1 receptor and biventricular functions in patients with acute myocardial infarction. Circ J 2006;70:1275-1279.
30. Hamon M, Amant C, Bauters C, Richard F, Helbecque N, McFadden E, Lablanche JM, Bertrand M, Amouyel P: Association of angiotensin converting enzyme and angiotensin II type 1 receptor genotypes with left ventricular function and mass in patients with angiographically normal coronary arteries. Heart 1997;77:502-505.
31. Henskens LH, Spiering W, Stoffers HE, Soomers FL, Vlietinck RF, de Leeuw PW, Kroon AA: Effects of ACE I/D and AT1R-A1166C polymorphisms on blood pressure in a healthy normotensive primary care population: first results of the Hippocates study. J Hypertens 2003;21:81-86.
32. Castellano M, Muiesan ML, Beschi M, Rizzoni D, Cinelli A, Salvetti M, Pasini G, Porteri E, Bettoni G, Zulli R, Agabiti-Rosei E: Angiotensin II type 1 receptor A/C1166 polymorphism. Relationships with blood pressure and cardiovascular structure. Hypertension 1996;28:1076-1080.
33. Urata H, Kinoshita A, Misono KS, Bumpus FM, et al. Identification of a highly specific chymase as the major angiotensin II-forming enzyme in the human heart. J Biol Chem 1990;265:22348-22357.
34. Urata H, Boehm KD, Philip A, Kinoshita A, et al. Cellular localization and regional distribution of an angiotensin II-forming chymase in the heart. J Clin Invest 1993;91:1269-1281.
35. Kitaura-Inenaga K, Hara M, Higuchi K, Yamamoto K, et al. Gene expression of cardiac mast cell chymase and tryptase in a murine model of heart failure caused by viral myocarditis. Circ J 2003;67:881-884.
36. Hara M, Matsumori A, Ono K, Kodo H, et al. Mast cells cause apoptosis of cardiomyocytes and proliferation of other intramyocardial cells in vitro. Circulation 1999;100:1443-1449.
37. Mizutani H, Schechter N, Lazarus G, Black RA, et al. Rapid and specific conversion of precursor interleukin 1 beta (IL-1 beta) to an active IL-1 species by human mast cell chymase. J Exp Med 1991;174:821-825.
38. Lees M, Taylor DJ, Woolley DE. Mast cell proteinases activate precursor forms of collagenase and stromelysin, but not of gelatinases A and B. Eur J Biochem 1994; 223:171-177.
39. Huang C, Friend DS, Qiu WT, Wong GW, et al. Induction of a selective and persistent extravasation of neutrophils into the peritoneal cavity by tryptase mouse mast cell protease 6. J Immunol 1998;160:1910-1919.
40. Rigat B, Hubert C, Alhenc-Gelas F, Cambien F, et al. An insertion/deletion polymorphism in the angiotensin I-converting enzyme gene accounting for half the variance of serum enzyme levels. J Clin Invest 1990;86:1343-1346.
41. Danser AH, Schalekamp MA, Bax WA, van den Brink AM, et al. Angiotensin-converting enzyme in the human heart. Effect of the deletion/insertion polymorphism. Circulation 1995;92:1387-1388.
42. Bleumink GS, Schut AF, Sturkenboom MC, Deckers JW, et al. Genetic polymorphisms and heart failure. Genet Med 2004;6:465-474.
43. McNamara DM, Holubkov R, Janosko K, Palmer A, et al. Pharmacogenetic interactions between beta-blocker therapy and the angiotensin-converting enzyme deletion polymorphism in patients with congestive heart failure. Circulation 2001; 103:1644-1648.
44. Andersson B, Blange I, Sylvén C. Angiotensin-II type 1 receptor gene polymorphism and long-term survival in patients with idiopathic congestive heart failure. Eur J Heart Fail 1999;1:363-369.
45. Hayashi M, Tsutamoto T, Wada A, Maeda K, Mabuchi N, Tsutsui T, Matsui T, Fujii M, Matsumoto T, Yamamoto T, Horie H, Ohnishi M, Kinoshita M. Relationship between transcardiac extraction of aldosterone and left ventricular remodeling in patients with first acute myocardial infarction: extracting aldosterone through the heart promotes ventricular remodeling after acute myocardial infarction. J Am Coll Cardiol 2001;38:1375-1382.
46. Dixen U, Ravn L, Soeby-Rasmussen C, Paulsen AW, Pamer J, Frandsen E, Jensen GB. Raised plasma aldosterone and natriuretic peptides in atrial fibrillation. Cardiology 2007;108:35-39.
47. White PC, Hautanen A, Kupari M. Aldosterone synthase (CYP11B2) polymorphisms and cardiovascular function. J Steroid Biochem Mol Biol 1999;69:409-412.
48. White PC, Slutsker L. Haplotype analysis of CYP11B2. 1995. Endocr Res 1995;21:437-442.
49. Brand E, Chatelain N, Mulatero P, Fery I, Curnow K, Jeunemaitre X, Corvol P, Pascoe L, Soubrier F. Structural analysis and evaluation of the aldosterone synthase gene in hypertension. Hypertension 1998;32:198-204.
50. White PC, Rainey WE. Editorial: polymorphisms in CYP11B genes and 11-hydroxylase activity. J Clin Endocrinol Metab 2005;90:1252-1255.
51. Takai E, Akita H, Kanazawa K, Shiga N, Terashima M, Matsuda Y, Iwai C, Miyamoto Y, Kawai H, Takarada A, Yokoyama M. Association between aldosterone synthase (CYP11B2) gene polymorphism and left ventricular volume in patients with dilated cardiomyopathy. Heart 2002;88:649-650.
52. Barbato A, Russo P, Siani A, Folkerd EJ, Miller MA, Venezia A, Grimaldi C, Strazzullo P, Cappuccio FP. Aldosterone synthase gene (CYP11B2) C-344T polymorphisms, plasma aldosterone, renin activity and blood pressure in a multi-ethnic population. J Hypertens 2004;22:1895-1901.
53. McNamara DM, Tam SW, Sabolinski ML, Tobelmann P, Janosko K, Taylor AL, Cohn JN, Feldman AM, Worcel M. Aldosterone synthase promoter polymorphism predicts outcome in African Americans with heart failure: results from the A-HeFT Trial. J Am Coll Candiol 2006;48:1277-1282.
54. Tsai CT, Lai LP, Lin JL, Chiang FT, Hwang JJ, Ritchie MD, Moore JH, Hsu KL, Tseng CD, Liau CS, Tseng YZ. Renin-angiotensin system gene polymorphisms and atrial fibrillation. Circulation 2004;109:1640-1646.
55. Sethupathy P, Borel C, Gagnebin M, Grant GR, Deutsch S, Elton TS, Hatzigeorgiou AG, Antonarakis SE. Human microRNA-155 on chromosome 21 differentially interacts with its polymorphic target in the AGTR1 3' untranslated region: a mechanism for functional single-nucleotide polymorphisms related to phenotypes. Am J Hum Genet. 2007 Aug;81(2):405-13.
56. Tiret L, Bonnardeaux A, Poirier O, Ricard S, Marques-Vidal P, Evans A, Arveiler D, Luc G, Kee F, Ducimetière P, et al. Synergistic effects of angiotensin-converting enzyme and angiotensin-II type 1 receptor gene polymorphisms on risk of myocardial infarction. Lancet. 1994 Oct 1;344(8927):910-3.
57. Pallaud C, Stranieri C, Sass C, Siest G, Pignatti F, Visvikis S: Candidate gene polymorphisms in cardiovascular disease: a comparative study of frequencies between a French and an Italian population. Clin Chem Lab Med 2001;39:146-154.
58. Beyerbach DM, Zipes DP. Mortality as an endpoint in atrial fibrillation. Heart Rhythm 2004;1:B8-18, discussion B18-19.
59. Flather MD, Shibata MC, Coats AJ, Van Veldhuisen DJ, Parkhomenko A, Borbola J, Cohen-Solal A, Dumitrascu D, Ferrari R, Lechat P, Soler-Soler J, Tavazzi L, Spinarova L, Toman J, Bohm M, Anker SD, Thompson SG, Poole-Wilson PA. Randomized trial to determine the effect of nebivolol on mortality and cardiovascular hospital admission in elderly patients with heart failure (SENIORS). Eur Heart J 2005;26:215-225.
60. Pai RG, Varadarajan P. Prognostic significance of atrial fibrillation is a function of left ventricular ejection fraction. Clin Cardiol 2007;30:349-354.
61. Van Wagoner DR. Recent insights into the pathophysiology of atrial fibrillation. Semin Thorac Cardiovasc Surg 2007;19:9-15.
62. Issac TT, Dokainish H, Lakkis NM. Role of inflammation in initiation and perpetuation of atrial fibrillation: a systematic review of the published data. J Am Coll Cardiol 2007;50:2021-2028.
63. Choudhury A, Varughese GI, Lip GY. Targeting the renin-angiotensin-aldosterone-system in atrial fibrillation: a shift from electrical to structural therapy? Expert Opin Pharmacother 2005;6:2193-2207.
64. Boos CJ, Anderson RA, Lip GY. Is atrial fibrillation an inflammatory disorder? Eur Heart J 2006;27:136-149.
65. Heymes C, Garnier A, Fuchs S, Bendall JK, Nehme J, Ambroisine ML, Robidel E, Swynghedauw B, Milliez P, Delcayre C. Aldosterone-synthase overexpression in heart: a tool to explore aidosterone's effects. Mol Cell Endocrinol 2004;217:213-219.
66. Yoshimura M, Nakamura S, Ito T, Nakayama M, Harada E, Mizuno Y, Sakamoto T, Yamamuro M, Saito Y, Nakao K, Yasue H, Ogawa H. Expression of aldosterone synthase gene in failing human heart: quantitative analysis using modified real-time polymerase chain reaction. J Clin Endocrinol Metab 2002;87:3936-3940.
67. Satoh M, Nakamura M, Saitoh H, Satoh H, Akatsu T, Iwasaka J, Masuda T, Hiramori K. Aldosterone synthase (CYP11B2) expression and myocardial fibrosis in the failing human heart. Clin Sci (Lond) 2002;102:381-386.
68. Yu HM, Lin SG, Liu GZ, Zhang YQ, Ma WJ, Deng CY. Associations between CYP11B2 gene polymorphisms and the response to angiotensin-converting enzyme inhibitors. Clin Pharmacol Ther 2006;79:581-589.
69. Chen PS, Tan AY. Autonomic nerve activity and atrial fibrillation. Heart Rhythm 2007;4:S61-64.
70. Biolo A, Chao T, Duhaney TA, Kotlyar E, Allensworth-Davies D, Loscalzo J, Sam F. Usefulness of the aldosterone synthase gene polymorphism C-344-T to predict cardiac remodeling in African-Americans versus non-African-Americans with chronic systolic heart failure. Am J Cardiol 2007;100:285-290.
71. Pfeufer A, Osterziel KJ, Urata H, Borck G, et al. Angiotensin-converting enzyme and heart chymase gene polymorphisms in hypertrophic cardiomyopathy. Am J Cardiol 1996;78:362-364.
72. Pfeufer A, Busjahn A, Vergopoulos A, Knoblauch H, et al. Chymase gene locus is not associated with myocardial infarction and is not linked to heart size or blood pressure. Am J Cardiol 1998;82:979-981.
73. Fischer M, Lieb W, Marold D, Berthold M, et al. Lack of association of a 9 bp insertion/deletion polymorphism within the bradykinin 2 receptor gene with myocardial infarction. Clin Sci (Lond) 2004; 107:505-511.
74. Guo C, Ju H, Leung D, Massaeli H, et al. A novel vascular smooth muscle chymase is upregulated in hypertensive rats. J Clin Invest 2001;107:703-715.
75. Patella V, Marino I, Arbustini E, Lamparter-Schummert B, et al. Stem cell factor in mast cells and increased mast cell density in idiopathic and ischemic cardiomyopathy. Circulation 1998;97:971-978.
76. Hara M, Ono K, Hwang MW, Iwasaki A, et al. Evidence for a role of mast cells in the evolution to congestive heart failure. J Exp Med 2002;195:375-381.
77. Semeri GG, Boddi M, Cecioni I, Vanni S, et al. Cardiac angiotensin II formation in the clinical course of heart failure and its relationship with left ventricular function. Circ Res 2001;88:961-968.
78. Chen LY, Li P, He Q, Jiang LQ, et al. Transgenic study of the function of chymase in heart remodeling. J Hypertens 2002;20:2047-2055.
79. Lindstedt KA, Wang Y, Shiota N, Saarinen J, et al. Activation of paracrine TGF-beta1 signaling upon stimulation and degranulation of rat serosal mast cells: a novel function for chymase. Faseb J 2001;15:1377-1388.
80. Petrov VV, Fagard RH, Lijnen PJ. Stimulation of collagen production by transforming growth factor-beta1 during differentiation of cardiac fibroblasts to myofibroblasts. Hypertension 2002;39:258-263.
81. Matsumoto T, Wada A, Tsutamoto T, Ohnishi M, et al. Chymase inhibition prevents cardiac fibrosis and improves diastolic dysfunction in the progression of heart failure. Circulation 2003;107:2555-2558.
82. Raynolds MV, Bristow MR, Bush EW, Abraham WT, et al. Angiotensin-converting enzyme DD genotype in patients with ischaemic or idiopathic dilated cardiomyopathy. Lancet 1993;342:1073-1075.
83. Harn HJ, Chang CY, Ho LI, Liu CA, et al. Evidence that polymorphism of the angiotensin I converting enzyme gene may be related to idiopathic dilated cardiomyopathy in the Chinese population. Biochem Mol Biol Int 1995;35:1175-1181.
84. Tiret L, Mallet C, Poirier O, Nicoud V, et al. Lack of association between polymorphisms of eight candidate genes and idiopathic dilated cardiomyopathy: the CARDIGENE study. J Am Coll Cardiol 2000;35:29-35.
85. Vancura V, Hubacek J, Malek I, Gebauerová M, et al. Does angiotensin-converting enzyme polymorphism influence the clinical manifestation and progression of heart failure in patients with dilated cardiomyopathy? Am J Cardiol 1999;83:461-462, A10.
86. McNamara DM, Holubkov R, Postava L, Janosko K, et al. Pharmacogenetic interactions between angiotensin-converting enzyme inhibitor therapy and the angiotensin-converting enzyme deletion polymorphism in patients with congestive heart failure. J Am Coll Cardiol 2004;44:2019-2026.
87. Kwok PY. Methods for genotyping single nucleotide polymorphisms. Annu Rev Genomics Hum Genet. 2001;2:235-58
88. Syvänen AC. Accessing genetic variation: genotyping single nucleotide polymorphisms. Nat Rev Genet. 2001 Dec;2(12):930-42.
89. Sambrook J, Fritsch EF, Maniatis T. Molecular Cloning. New York: Cold Spring Harbor, 1989.
90. Kupari M, Hautanen A, Lankinen L, Koskinen P, Virolainen J, Nikkila H, White PC. Associations between human aldosterone synthase (CYP11B2) gene polymorphisms and left ventricular size, mass, and function. Circulation 1998;97:569-575.
91. Schunkert H, Hengstenberg C, Holmer SR, Broeckel U, Luchner A, Muscholl MW, Kurzinger S, Doring A, Hense HW, Riegger GA. Lack of association between a polymorphism of the aldosterone synthase gene and left ventricular structure. Circulation 1999;99:2255-2260.
92. Stella P, Bigatti G, Tizzoni L, Barlassina C, Lanzani C, Bianchi G, Cusi D. Association between aldosterone synthase (CYP11B2) polymorphism and left ventricular mass in human essential hypertension. J Am Coll Cardiol 2004;43:265-270.
93. Lindpaintner K, Pfeffer MA, Kreutz R, Stampfer MJ, et al. A prospective evaluation of an angiotensin-converting-enzyme gene polymorphism and the risk of ischemic heart disease. N Engl J Med 1995;332:706-711.

### APPENDIX I

### AT1R

SNP name and number (in NCBI SNP database):
   AT1R A1166C rs5186
Gene name and number (in NCBI nucleotide database):
   AT1R angiotensin II receptor, type 1, NM_000685

### 1. Sequence of WT allele (A1166):

CAGCTTCTAAAATATATTCCCCCAAAAGCCAAATCCCACTCAAACCTTTCA
ACAAAAATGAGCACGCTTTCCTACCGCCCCTCAGATAATGTAAGCTCATC
CACCAAGAAGCCTGCACCATGTTTTGAGGTTGAGTGACATGTTCGAAACC
TGTCCATAAAGTAATTTTGTGAAAGAAGGAGCAAGAGAACATTCCTCTGC
AGCACTTCACTACCAAATGAGCATTAGCTACTTTTCAGAATTGAAGGAGA
AAATGCATTATGTGGACTGAACCGACTTTTCTAAAGCTCTGAACAAAAGC
TTTTCTTTCCTTTTGCAACAAGACAAAGCAAAGCCACATTTTGCATTAGAC
AGATGACGGCTGCTCGAAGAACAATGTCAGAAACTCGATGAATGTGTTGA
TTTGAGAAATTTTACTGACAGAAATGCAATCTCCCTAGCCTGCTTTTGTCC
TGTTATTTTTTATTTCCACATAAAGGTATTTAGAATA

### 2. Sequence of mutant (SNP bearing) allele (C1166):

CAGCTTCTAAAATATATTCCCCCAAAAGCCAAATCCCACTCAAACCTTTCA
ACAAAAATGAGCACGCTTTCCTACCGCCCCTCAGATAATGTAAGCTCATC
CACCAAGAAGCCTGCACCATGTTTGAGGTTGAGTGACATGTTCGAAACC
TGTCCATAAAGTAATTTTGTGAAAGAAGGAGCAAGAGAACATTCCTCTGC
AGCACTTCACTACCAAATGAGCCTTAGCTACTTTTCAGAATTGAAGGAGA
AAATGCATTATGTGGACTGAACCGACTTTTCTAAAGCTCTGAACAAAAGC
TTTTCTTTCCTTTTGCAACAAGACAAAGCAAAGCCACATTTTGCATTAGAC
AGATGACGGCTGCTCGAAGAACAATGTCAGAAACTCGATGAATGTGTTGA
TTTGAGAAATTTTACTGACAGAAATGCAATCTCCCTAGCCTGCTTTTGTCC
TGTTATTTTTTATTTCCACATAAAGGTATTTAGAATA

### CYP11B2:

SNP name and SNP number (in NCBI SNP database):
   CYP11B2 T-344C rs1799998
Gene name and Genebank number (in NCBI nucleotide database):
   CYP11B2 aldosterone synthase, AC073385

### 3. Sequence of WT allele (-344T) (please note sequence is in reverse):

TCAAGGCTGGAGGCCCCCAGCCAAAGGTAGATGAAGGAGAAGTCAGGTG
CCTAATTCCCCATTGCTGCAAGTCCTGCTGGTCTGAGGATGCTGAGAAAA
GGCGTGGGGTCTGGACTGGGGGTCCATGCTGGTGGAAGGTGGTGGGACCT
GGCCTCTCCTTTCTCCAGGGCTGAGAGGAGTAAAATGGATGGGGACTTTA
TCTTATCGTGAGATGAGAGGGAGCCTTGGATTCTTTTAATAGACTTTATTT
TTATACCACAGATTTAGTTCATTGCAAAATTGATCAAAAACTGCAGAAAA
TGTCCACGTACCCCCTGCCCTGACACAGGTACACCCTCCACCACAGGAGC
GCACTGGTTCCATGTGAGGAATCTCGATACGTTGTTATCAACCAGGGTCCT
GGAGTCACATGGGGTCTCCTCCTGGTGGTCAACATGCTGTGGGTTTTGACA
AATGTATGTAATTTGTATCCCTCCTTGCAGGATCCTAG

### 4. Sequence of mutant (SNP bearing) allele (-344C) (please note sequence is in reverse):

TCAAGGCTGGAGGCCCCCAGCCAAAGGTAGATGAAGGAGAAGTCAGGTG
CCTAATTCCCCATTGCTGCAAGTCCTGCTGGTCTGAGGATGCTGAAAA
GGCGTGGGGTCTGGACTGGGGGTCCATGCTGGTGGAAGGTGGTGGGACCT
GGCCTCTCCTTTCTCCAGGGCTGAGAGGAGTAAAATGGATGGGGACTTTA
TCTTATCGTGAGATGAGAGGGGGCCTTGGATTCTTTTAATAGACTTTATTT
TTATACCACAGATTTAGTTCATTGCAAAATTGATCAAAAACTGCAGAAAA
TGTCCACGTACCCCCTGCCCTGACACAGGTACACCCTCCACCACAGGAGC
GCACTGGTTCCATGTGAGGAATCTCGATACGTTGTTATCAACCAGGGTCCT
GGAGTCACATGGGGTCTCCTCCTGGTGGTCAACATGCTGTGGGTTTTGACA
AATGTATGTAATTTGTATCCCTCCTTGCAGGATCCTAG

### CMA1

SNP name and number (in NCBI SNP database):
   CMA1 G-1903A rs1800875
Gene name and number (in NCBI nucleotide database):
   chymase 1, mast cell, M64269

### 5. Sequence of WT allele (G-1903):

GTTCACTACCAACATGCTATATATAAAATAACCAAAGGGGGAAGAAGAA
AGAGAAAAAGGAAATCTCTTAAAATACACAGGTATACATATGACAAAGC
AAAGAAGGAAATGTGAGCAGATAGCGCAGTCCTCGTTTCTGAAATTGGTC
CCCTGACTGGGGCTATACCTATTCCATTTCCTCACCCTCAGCCAGGCAGGT
GGAGCAAAACTTAAGTCTTGGTGGATCTGAATCTTGATGCTGTGGAGCTG
TCTTACTAGCCCCAGACTACCTGCCTCTCAATTTCTAATTATATCAGTGAA
AGCAAACAGCTTTGATTTGTTTAAGCCTCTGATTTTTGGTCTAACTGATG
TAAGACCACAAGACAAGAGTTCTCCAGCTCCGGATTCTCTTCTGTTCTGTT
AATGGTGAAATGCCCAGAG

### 6. Sequence of mutant (SNP bearing) allele (G-1903A):

GTTCACTACCAACATGCTATATATAAAATAACCAAAGGGGGAAGAAGAA
AGAGAAAAAGGAAATCTCTTAAAATACACAGGTATACATATGACAAAGC
AAAGAAGGAAATGTGAGCAGATAGCGCAGTCCTCGTTTCTGAAATTGGTC
CCCTGACTGGGGCTATACCTATTCCATTTCCTCACCCTCAGCCAGGCAGGT
GAAGCAAAACTTAAGTCTTGGTGGATCTGAATCTTGATGCTGTGGAGCTG
TCTTACTAGCCCCAGACTACCTGCCTCTCAATTTCTAATTATATCAGTGAA
AGCAAACAGCTTTGATTTGTTTAAGCCTCTGATTTTTGGTCTAACTGATG
TAAGACCACAAGACAAGAGTTCTCCAGCTCCGGATTCTCTTCTGTTCTGTT
AATGGTGAAATGCCCAGAG

### BDKRB2

SNP name and number (in NCBI SNP database): Not found
   The SNP is an insertion/deletion of 9 bp (nucleotides).
Gene name and number (in NCBI nucleotide database):
   bradykinin receptor B2, NM_000623

### 7. Sequence of WT allele (insertion):

CTCCGAGGAGGGGTGGGGACGGTCCTGACGGTGGGGACATCAGGCTGCC
CCGCAGTACCAGGGAGCGACTTGAAGTGCCCATGCCGCTTGCTCCGGGAG
AAGCCCAGGTGTGGCCTCACTCACATCCCACTCTGAGTCCA

### 8. Sequence of mutant allele (deletion):

CTCCGAGGAGGGGTGGGACGGTXXXXXXXXGGGGACATCAGGCTGCC
CCGCAGTACCAGGGAGCGACTTGAAGTGCCCATGCCGCTTGCTCCGGGAG
AAGCCCAGGTGTGGCCTCACTCACATCCCACTCTGAGTCCA

### ADRB2

SNP name and number (in NCBI SNP database):
   ADRB2 Gly (G)16 Arg (R) [G46A (according to mRNA NM_000024) nucleotide numbering starts at the start codon], rs 1042713
Gene name and number (in NCBI nucleotide database):
   adrenergic, beta-2-, receptor, surface, NM_000024
   Also known as: BAR; B2AR; ADRBR; ADRB2R; BETA2AR

### 9. Sequence of WT allele [Gly (G)16, G46]:

CGGCTTCTTCAGAGCACGGGCTGGAACTGGCAGGCACCGCGAGCCCCTAG
CACCCGACAAGCTGAGTGTGCAGGACGAGTCCCCACCACACCCACACCAC
AGCCGCTGAATGAGGCTTCCAGGCGTCCGCTCGCGGCCCGCAGAGCCCCG
CCGTGGGTCCGCCCGCTGAGGCGCCCCCAGCCAGTGCGCTCACCTGCCAG

ACTGCGCGCCATGGGGCAACCCGGGAACGGCAGCGCCTTCTTGCTGGCAC
CCAATGGAAGCCATGCGCCGGACCACGACGTCACGCAGGAAAGGGACGA
GGTGTGGGTGGTGGGCATGGGCATCGTCATGTCTCTCATCGTCCTGGCCAT
CGTGTTTGGCAATGTGCTGGTCATCACAGCCATTGCCAAGTTCGAGCGTCT
GCAGACGGTCACCAACTACTTCATCACTTCACTGGCCTGTGCTGATCTGGT
CATGGGCCTGGCAGTGGTGCCCTTTGGGGCCGCCCATATTCTTATGAAAAT
GTGGACTT

### 10. Sequence of mutant (SNP bearing) allele [Arg (A)16, A46]:

CGGCTTCTTCAGAGCACGGGCTGGAACTGGCAGGCACCGCGAGCCCCTAG
CACCCGACAAGCTGAGTGTGCAGGACGAGTCCCCACCACACCCACACCAC
AGCCGCTGAATGAGGCTTCCAGGCGTCCGCTCGCGGCCCGCAGAGCCCCG
CCGTGGGTCCGCCCGCTGAGGCGCCCCCAGCCAGTGCGCTCACCTGCCAG
ACTGCGCGCCATGGGGCAACCCGGGAACGGCAGCGCCTTCTTGCTGGCAC
CCAATAGAAGCCATGCGCCGGACCACGACGTCACGCAGGAAAGGGACGA
GGTGTGGGTGGTGGGCATGGGCATCGTCATGTCTCTCATCGTCCTGGCCAT
CGTGTTTGGCAATGTGCTGGTCATCACAGCCATTGCCAAGTTCGAGCGTCT
GCAGACGGTCACCAACTACTTCATCACTTCACTGGCCTGTGCTGATCTGGT
CATGGGCCTGGCAGTGGTGCCCTTTGGGGCCGCCCATATTCTTATGAAAAT
GTGGACTT

### ADRB2

SNP name and number (in NCBI SNP database):
   ADRB2 Glu (E) 27 Gln (Q), [G79C (according to mRNA NM_000024), nucleotide numbering starts at the start codon], rs1042714
Gene name and number (in NCBI nucleotide database):
   adrenergic, beta-2-, receptor, surface, NM_000024
   Also known as: BAR; B2AR; ADRBR; ADRB2R; BETA2AR

### 11. Sequence of WT allele [Glu (E)27, G79]:

GCACCGCGAGCCCCTAGCACCCGACAAGCTGAGTGTGCAGGACGAGTCCC
CACCACACCCACACCACAGCCGCTGAATGAGGCTTCCAGGCGTCCGCTCG
CGGCCCGCAGAGCCCCGCCGTGGGTCCGCCCGCTGAGGCGCCCCCAGCCA
GTGCGCTCACCTGCCAGACTGCGCGCCATGGGGCAACCCGGGAACGGCAG
CGCCTTCTTGCTGGCACCCAATGGAAGCCATGCGCCGGACCACGACGTCA
CGCAGGAAAGGACGAGGTGTGGGTGGTGGGCATGGGCATCGTCATGTC
TCTCATCGTCCTGGCCATCGTGTTTGGCAATGTGCTGGTCATCACAGCCAT
TGCCAAGTTCGAGCGTCTGCAGACGGTCACCAACTACTTCATCACTTCACT
GGCCTGTGCTGATCTGGTCATGGGCCTGGCAGTGGTGCCCTTTGGGGCCG
CCCATATTCTTATGAAAATGTGGACTTTTGGCAACTTCTGGTGCGAGTTTT
GGACTTCCA

### 12. Sequence of mutant (SNP bearing) allele [Gln (Q)27, C79]:

GCACCGCGAGCCCCTAGCACCCGACAAGCTGAGTGTGCAGGACGAGTCCC
CACCACACCCACACCACAGCCGCTGAATGAGGCTTCCAGGCGTCCGCTCG
CGGCCCGCAGAGCCCCGCCGTGGGTCCGCCCGCTGAGGCGCCCCCAGCCA
GTGCGCTCACCTGCCAGACTGCGCGCCATGGGGCAACCCGGGAACGGCAG
CGCCTTCTTGCTGGCACCCAATGGAAGCCATGCGCCGGACCACGACGTCA
CGCAGCAAAGGACGAGGTGTGGGTGGTGGGCATGGGCATCGTCATGTCT
CTCATCGTCCTGGCCATCGTGTTTGGCAATGTGCTGGTCATCACAGCCATT
GCCAAGTTCGAGCGTCTGCAGACGGTCACCAACTACTTCATCACTTCACTG
GCCTGTGCTGATCTGGTCATGGGCCTGGCAGTGGTGCCCTTTGGGGCCGCC
CATATTCTTATGAAAATGTGGACTTTTGGCAACTTCTGGTGCGAGTTTTGG
ACTTCCA

### ADRB2

SNP name and number (in NCBI SNP database):
   ADRB2 Thr(T) 164 Ile (I), [C491T (according to mRNA NM_000024), nucleotide numbering starts at the start codon, position on NW_001838953 is 6928765 and
   position on NT_029289 is 9369821], rs1800888.
Gene name and number (in NCBI nucleotide database):
   adrenergic, beta-2-, receptor, surface, NM_000024 (mRNA transcript) from homo sapiens chromosome 5 genomic contig (NW_001838953 or NT_029289).
   Also known as: BAR; B2AR; ADRBR; ADRB2R; BETA2AR

### 13. Sequence of WT allele [Thr(T)164, C491]:

ATCTGGTCATGGGCCTGGCAGTGGTGCCCTTTGGGGCCGCCCATATTCTTA
TGAAAATGTGGACTTTTGGCAACTTCTGGTGCGAGTTTTGGACTTCCATTG
ATGTGCTGTGCGTCACGGCCAGCATTGAGACCCTGTGCGTGATCGCAGTG
GATCGCTACTTTGCCATTACTTCACCTTTCAAGTACCAGAGCCTGCTGACC
AAGAATAAGGCCCGGGTGATCATTCTGATGGTGTGGATTGTGTCAGGCCT
TACCTCCTTCTTGCCCATTCAGATGCACTGGTACCGGGCCACCCACCAGGA
AGCCATCAACTGCTATGCCAATGAGACCTGCTGTGACTTCTTCACGAACC
AAGCCTATGCCATTGCCTCTTCCATCGTGTCCTTCTACGTTCCCCTGGTGAT
CATGGTCTTCGTCTACTCCAGGGTCTTTCAGGAGGCCAAAAGGCAGCTCC
AGAAGATTGACAAATCTGAGGGCCGCTTCCATGTCCAGAACCTTAGCCAG
GTGGA

### 14. Sequence of mutant (SNP bearing) allele [Ile (I)164, T491]:

ATCTGGTCATGGGCCTGGCAGTGGTGCCCTTTGGGGCCGCCCATATTCTTA
TGAAAATGTGGACTTTTGGCAACTTCTGGTGCGAGTTTTGGACTTCCATTG
ATGTGCTGTGCGTCACGGCCAGCATTGAGACCCTGTGCGTGATCGCAGTG
GATCGCTACTTTGCCATTACTTCACCTTTCAAGTACCAGAGCCTGCTGACC
AAGAATAAGGCCCGGGTGATCATTCTGATGGTGTGGATTGTGTCAGGCCT

TATCTCCTTCTTGCCCATTCAGATGCACTGGTACCGGGCCACCCACCAGGA
AGCCATCAACTGCTATGCCAATGAGACCTGCTGTGACTTCTTCACGAACC
AAGCCTATGCCATTGCCTCTTCCATCGTGTCCTTCTACGTTCCCCTGGTGAT
CATGGTCTTCGTCTACTCCAGGGTCTTTCAGGAGGCCAAAAGGCAGCTCC
AGAAGATTGACAAATCTGAGGGCCGCTTCCATGTCCAGAACCTTAGCCAG
GTGGA

### ADRB1

SNP name and number (in NCBI SNP database):
   ADRB1 Ser (S) 49 Gly (G), [A145G (according to mRNA NM_000684), nucleotide numbering starts at the start codon], rs 1801252
Gene name and number (in NCBI nucleotide database):
   adrenergic, beta-1-, receptor, NM_000684
   Also known as: RHR; B1AR; ADRB1R; BETA1AR

### 15. Sequence of WT allele [Ser (S)49; A145]:

TGACCCGGCCGCGACCTCCCTCTGCGCACCACGCCGCCCGGGCTTCTGGG
GTGTTCCCCAACCACGGCCCAGCCCTGCCACACCCCCGCCCCCGGCCTCC
GCAGCTCGGCATGGGCGCGGGGGTGCTCGTCCTGGGCGCCTCCGAGCCCG
GTAACCTGTCGTCGGCCGCACCGCTCCCCGACGGCGCGGCCACCGCGGCG
CGGCTGCTGGTGCCCGCGTCGCCGCCCGCCTCGTTGCTGCCTCCCGCCAGC
GAAAGCCCCGAGCCGCTGTCTCAGCAGTGGACAGCGGGCATGGGTCTGCT
GATGGCGCTCATCGTGCTGCTCATCGTGGCGGGCAATGTGCTGGTGATCG
TGGCCATCGCCAAGACGCCGCGGCTGCAGACGCTCACCAACCTCTTCATC
ATGTCCCTGGCCAGCGCCGACCTGGTCATGGGGCTGCTGGTGGTGCCGTT
CGGGGCCACCATCGTGGTGTGGGGCCGCTGGGAGTACGGCTCCTTCTTCT
GCGAGCTGT

### 16. Sequence of mutant (SNP bearing) allele [Gly(G)49, G145]:

TGACCCGGCCGCGACCTCCCTCTGCGCACCACGCCGCCCGGGCTTCTGGG
GTGTTCCCCAACCACGGCCCAGCCCTGCCACACCCCCGCCCCCGGCCTCC
GCAGCTCGGCATGGGCGCGGGGGTGCTCGTCCTGGGCGCCTCCGAGCCCG
GTAACCTGTCGTCGGCCGCACCGCTCCCCGACGGCGCGGCCACCGCGGCG
CGGCTGCTGGTGCCCGCGTCGCCGCCCGCCTCGTTGCTGCCTCCCGCCAGC
GAAGGCCCCGAGCCGCTGTCTCAGCAGTGGACAGCGGGCATGGGTCTGCT
GATGGCGCTCATCGTGCTGCTCATCGTGGCGGGCAATGTGCTGGTGATCG
TGGCCATCGCCAAGACGCCGCGGCTGCAGACGCTCACCAACCTCTTCATC
ATGTCCCTGGCCAGCGCCGACCTGGTCATGGGGCTGCTGGTGGTGCCGTT
CGGGGCCACCATCGTGGTGTGGGGCCGCTGGGAGTACGGCTCCTTCTTCT
GCGAGCTGT

### ADRB1

SNP name and number (in NCBI SNP database):
   ADRB1 Arg (R) 389 Gly (G), [C1165G (according to mRNA NM_000684), nucleotide numbering starts at the start codon,], rs1801253
Gene name and number (in NCBI nucleotide database):
   adrenergic, beta-1-, receptor, NM_000684
   Also known as: RHR; B1AR; ADRB1R; BETA1AR

### 17. Sequence of WT allele [Arg (R)389; C1165]:

CCCCGCGCCCCGCCGCCGCCGCCGCCACCGCCCCGCTGGCCAACGGGCGT
GCGGGTAAGCGGCGGGCCCTCGCGCCTCGTGGCCCTGCGCGAGCAGAAG
GCGCTCAGGACGCTGGGCATCATCATGGGCGTCTTCACGCTCTGCTGGCT
GCCCTTCTTCCTGGCCAACGTGGTGAAGGCCTTCCACCGCGAGCTGGTGCC
CGACCGCCTCTTCGTCTTCTTCAACTGGCTGGGCTACGCCAACTCGGCCTT
CAACCCCATCATCTACTGCCGCAGCCCCGACTTCCGCAAGGCCTTCCAGC
GACTGCTCTGCTGCGCGCGCAGGGCTGCCCGCCGGCGCCACGCGACCCAC
GGAGACCGGCCGCGCGCCTCGGGCTGTCTGGCCCGGCCCGGACCCCCGCC
ATCGCCCGGGGCCGCCTCGGACGACGACGACGACGATGTCGTCGGGGCCA
CGCCGCCCGCGCGCCTGCTGGAGCCCTGGGCCGGCTGCAACGGCGGGGCG
GCGGCGGACAGCGACTCGAGCCTGGACGAGCCGTGCCGCCCCGGCTTCGC
CTCGGAATCCAAGGTGTAGGGCCCGGCGCGGGGCGCGGACTCCGGGCAC
G

### 18. Sequence of mutant (SNP bearing) allele [Gly (G)389, G1165]:

CCCCGCGCCCCGCCGCCGCCGCCACCGCCCCGCTGGCCAACGGGCGT
GCGGGTAAGCGGCGGGCCCTCGCGCCTCGTGGCCCTGCGCGAGCAGAAG
GCGCTCAGGACGCTGGGCATCATCATGGGCGTCTTCACGCTCTGCTGGCT
GCCCTTCTTCCTGGCCAACGTGGTGAAGGCCTTCCACCGCGAGCTGGTGCC
CGACCGCCTCTTCGTCTTCTTCAACTGGCTGGGCTACGCCAACTCGGCCTT
CAACCCCATCATCTACTGCCGCAGCCCCGACTTCCGCAAGGCCTTCCAGG
GACTGCTCTGCTGCGCGCGCAGGGCTGCCCGCCGGCGCCACGCGACCCAC
GGAGACCGGCCGCGCGCCTCGGGCTGTCTGGCCCGGCCCGGACCCCCGCC
ATCGCCCGGGGCCGCCTCGGACGACGACGACGACGATGTCGTCGGGGCCA
CGCCGCCCGCGCGCCTGCTGGAGCCCTGGGCCGGCTGCAACGGCGGGGCG
GCGGCGGACAGCGACTCGAGCCTGGACGAGCCGTGCCGCCCCGGCTTCGC
CTCGGAATCCAAGGTGTAGGGCCCGGCGCGGGGCGCGGACTCCGGGCAC
G

### ADRA1A

SNP name and number (in NCBI SNP database):
   ADRA1A Arg (R)347 Cys (C), [C1039T according to mRNA variants (NM_000680, NM_033302, NM_033303, NM_033304), nucleotide numbering starts at the starts codon,], rs 1048101
Gene name and number (in NCBI nucleotide database):
   adrenergic, alpha-1A-, receptor, NM_000680, NM_033302, NM_033303, NM_033304(4 different splice variants)
   Also known as: ADRA1C; ADRA1L1; ALPHA1AAR

### 19. Sequence of WT allele [Arg (R)347; C1039]:

GTCAGTCAAATGTGAGAAACTCATATGTGTTTGGGATCATTTAACCGTTT
AAAAATACAGAAAGATGTCTGTTTGATTGTTTTCCTAGCCAATTGGCTTGC
TGGCTTTCAAATAATATGTATAAATCTGTGTGTTTTCTTCCAGGGTCTTTCT
TCCCTGATTTCAAGCCCTCTGAAACAGTTTTTAAAATAGTATTTTGGCTCG
GATATCTAAACAGCTGCATCAACCCCATCATATACCCATGCTCCAGCCAA
GAGTTCAAAAAGGCCTTTCAGAATGTCTTGAGAATCCAGTGTCTCCGCAG
AAAGCAGTCTTCCAAACATGCCCTGGGCTACACCCTGCACCCGCCCAGCC
AGGCCGTGGAAGGGCAACACAAGGACATGGTGCGCATCCCCGTGGGATC
AAGAGAGACCTTCTACAGGATCTCCAAGACGGATGGCGTTTGTGAATGGA
AATTTTTCTCTTCCATGCCCCGTGGATCTGCCAGGATTACAGTGTCCAAAG
ACCAATCCTCCTGTACCACAGCCCGGGTGAGAAGTAAAAGCTTTTGCAG
GTCTGCTGCTGTGTAGGGCCCTCAACCCCCAGCCTT

### 20. Sequence of mutant (SNP bearing) allele [Cys (C)347, T1039]:

GTCAGTCAAATGTGAGAAACTCATATGTGTTTGGGATCATTTTAACCGTTT
AAAAATACAGAAAGATGTCTGTTTGATTGTTTTCCTAGCCAATTGGCTTGC
TGGCTTTCAAATAATATGTATAAATCTGTGTGTTTTCTTCCAGGGTCTTTCT
TCCCTGATTTCAAGCCCTCTGAAACAGTTTTTAAAATAGTATTTTGGCTCG
GATATCTAAACAGCTGCATCAACCCCCATCATATACCCATGCTCCAGCCAA
GAGTTCAAAAAGGCCTTTCAGAATGTCTTGAGAATCCAGTGTCTCTGCAG
AAAGCAGTCTTCCAAACATGCCCTGGGCTACACCCTGCACCCGCCCAGCC
AGGCCGTGGAAGGGCAACACAAGGACATGGTGCGCATCCCCGTGGGATC
AAGAGAGACCTTCTACAGGATCTCCAAGACGGATGGCGTTTGTGAATGGA
AATTTTTCTCTTCCATGCCCCGTGGATCTGCCAGGATTACAGTGTCCAAAG
ACCAATCCTCCTGTACCACAGCCCGGGTGAGAAGTAAAAGCTTTTTGCAG
GTCTGCTGCTGTGTAGGGCCCTCAACCCCCAGCCTT

### ADRA2B

SNP name and number (in NCBI SNP database):
   ADRA2B 894± AGAGGAGGA (insertion/deletion polymorphism), rs29000568
Gene name and number (in NCBI nucleotide database):
   adrenergic, alpha-2B-, receptor, NM_000682
   Also known as: ADRA2L1; ADRARL1; ADRA2RL1; ALPHA2BAR

### 21. Sequence of WT allele

AGAGGAGGA:TGGGCAGGGTGAGTCCAAGCAGCCCCGACCCGACCATGGT
GGGGCTTTGGCCTCAGCCAAACTGCCAGCCCTGGCCTCTGTGGCTTCTGCC
AGAGAGGTCAACGGACACTCGAAGTCCACTGGGGAGAAGGAGGAGGGGG
AGACCCCTGAAGATACTGGGACCCGGGCCTTGCCACCCAGTTGGGCTGCC
CTTCCCAACTCAGGCCAGGGCCAGAAGGAGGGTGTTTGTGGGGCATCTCC
AGAGGATGAAGCTGAAGAGGAGGAGGAGGAGGAGGAAGAGTGTGAACC
CCAGGCAGTGCCAGTGTCTCCGGCCTCAGCTTGCAGCCCCCCGCTGCAGC
AGCCACAGGGCTCCCGGGTGCTGGCCACCCTACGTGGCCAGGTGCTCCTG
GGCAGGGGCGTGGGTGCTATAGGTGGGCAGTGGTGGCGTCGACGGGCGC
AGCTGACCCGGGAGAAGCGCTTCACCTTCGTGCTGGCTGTGGTCATTGGC
GTTTTTGTGCTCTGCTGGTTCCC

### 22. Sequence of mutant allele (deletion) -

AGAGGAGGATGGGCAGGGTGAGTCCAAGCAGCCCCGACCCGACCATGGT
GGGGCTTTGGCCTCAGCCAAACTGCCAGCCCTGGCCTCTGTGGCTTCTGCC
AGAGAGGTCAACGGACACTCGAAGTCCACTGGGAGAAGGAGGAGGGGG
AGACCCCTGAAGATACTGGGACCCGGGCCTTGCCACCCAGTTGGGCTGCC
CTTCCCAACTCAGGCCAGGGCCAGAAGGAGGGTGTTTGTGGGGCATCTCC
AGAGGATGAAGCTGAXXXXXXXXXGGAGGAGGAGGAAGAGTGTGAACC
CCAGGCAGTGCCAGTGTCTCCGGCCTCAGCTTGCAGCCCCCCGCTGCAGC
AGCCACAGGGCTCCCGGGTGCTGGCCACCCTACGTGGCCAGGTGCTCCTG
GGCAGGGGCGTGGGTGCTATAGGTGGGCAGTGGTGGCGTCGACGGGCGC
AGCTGACCCGGGAGAAGCGCTTCACCTTCGTGCTGGCTGTGGTCATTGGC
GTTTTTGTGCTCTGCTGGTTCCC

### IL10

SNP name and number (in NCBI SNP database):
   IL10 C-592A, rs1800872 (position 377537 on NW_001838536)
Gene name and number (in NCBI nucleotide database):
   IL10, interleukin 10, NM-000572 (mRNA transcript) from *Homo sapiens* chromosome 1
   genomic contig(NW_001838536, nucleotides372078-376969).
   Also known as: CSIF; TGIF; IL-10; IL10A; MGC126450; MGC126451

### 23. Sequence of the WT allele: C-592:

GAGATGGTGTACAGTAGGGTGAGGAAACCAAATTCTCAGTTGGCACTGGT
GTACCCTTGTACAGGTGATGTAACATCTCTGTGCCTCAGTTTGCTCACTAT
AAAATAGAGACGGTAGGGGTCATGGTGAGCACTACCTGACTAGCATATAA

GAAGCTTTCAGCAAGTGCAGACTACTCTTACCCACTTCCCCCAAGCACAG
TTGGGGTGGGGGACAGCTGAAGAGGTGGAAACATGTGCCTGAGAATCCT
AATGAAATCGGGGTAAAGGAGCCTGGAACACATCCTGTGACCCCGCCTGT
CCTGTAGGAAGCCAGTCTCTGGAAAGTAAAATGGAAGGGCTGCTTGGGA
ACTTTGAGGATATTTAGCCCACCCCCTCATTTTTACTTGGGGAAACTAAGG
CCCAGAGACCTAAGGTGACTGCCTAAGTTAGCAAGGAGAAGTCTTGGGTA
TTCATCCCAGGTTGGGGGGACCCAATTATTTCTCAATCCCATTGTATTCTG
GAATGGGCAATTTGTCCACGTCACTGTGACCTAGGAACACGCGAATGAGA
ACCCACAGCTGAGGGCCTCTGCGCACAGAACAGCTGTTCTCCCCAGGAAA

### 24. Sequence of the mutant (SNP bearing) allele: A-592:

GAGATGGTGTACAGTAGGGTGAGGAAACCAAATTCTCAGTTGGCACTGGT
GTACCCTTGTACAGGTGATGTAACATCTCTGTGCCTCAGTTTGCTCACTAT
AAAATAGAGACGGTAGGGGTCATGGTGAGCACTACCTGACTAGCATATAA
GAAGCTTTCAGCAAGTGCAGACTACTCTTACCCACTTCCCCCAAGCACAG
TTGGGGTGGGGGACAGCTGAAGAGGTGGAAACATGTGCCTGAGAATCCT
AATGAAATCGGGGTAAAGGAGCCTGGAACACATCCTGTGACCCCGCCTGT
ACTGTAGGAAGCCAGTCTCTGGAAAGTAAAATGGAAGGGCTGCTTGGGA
ACTTTGAGGATATTTAGCCCACCCCCTCATTTTTACTTGGGGAAACTAAGG
CCCAGAGACCTAAGGTGACTGCCTAAGTTAGCAAGGAGAAGTCTTGGGTA
TTCATCCCAGGTTGGGGGGACCCAATTATTTCTCAATCCCATTGTATTCTG
GAATGGGCAATTTGTCCACGTCACTGTGACCTAGGAACACGCGAATGAGA
ACCCACAGCTGAGGGCCTCTGCGCACAGAACAGCTGTTCTCCCCAGGAAA

### IL10

SNP name and number (in NCBI SNP database):
   IL10 A-1082G, rs 1800896 (position 378027 on NW_001838536)
Gene name and number (in NCBI nucleotide database):
   IL10, interleukin 10, NM_000572 (mRNA transcript) from *Homo sapiens* chromosome 1
   genomic contig (NW_001838536, nucleotides372078-376969).
   Also known as: CSIF; TGIF; IL-10; IL10A; MGC126450; MGC126451

### 25. Sequence of the WT allele: A-1082:

TCAATGCTCCCTGGCAGGCAGGAGGACAGGTGCTATTGCCCTGTTGGGAC
AGATGAAAAACAGACACAGGGAGGATGAGTGATTTGCCCTGACTATAGA
GTGGCAGGGCCAAGGCAGAGCCCAGGCCTCCTGCACCTAGGTCAGTGTTC
CTCCCAGTTACAGTCTAAACTGGAATGGCAGGCAAAGCCCCTGTGGAAGG
GGAAGGTGAAGCTCAAATCAAAGCTCNNCCAGAGACTTTCCAGATATCTG
AAGAAGTCCTGATGTCACTGCCCCGGTCCTTCCCCAGGTAGAGCAACACT
CCTCGCCGCAACCCAACTGGCTCTCCTTACTTTCTACACACACACACACAC

ACACACACACACACACACACACACACACACAAATCCAAGACAACACTACTA
AGGCTTCTTTGGGAAGGGGAAGTAGGGATAGGTAAGAGGAAAGTAAGGG
ACCTCCTATCCAGCCTCCATGGAATCCTGACTTCTTTTCCTTGTTATTTCAA
CTTCTTCCACCCCATCTTTTAAACTTTAGACTCCAGCCACAGAAGCTTACA
ACTAAAAGAAACTCTAAGGCCAATTTAATCCAAGGTTTCATTCTATGTGCT
GGAGATGGTGTACAGTAGGGTGAGGAAACCAAATTCTCAGTTGGCACTGG
TG

### 26. Sequence of the mutant (SNP bearing) allele: G-1082:

TCAATGCTCCCTGGCAGGCAGGAGGACAGGTGCTATTGCCCTGTTGGGAC
AGATGAAAAACAGACACAGGGAGGATGAGTGATTTGCCCTGACTATAGA
GTGGCAGGGCCAAGGCAGAGCCCAGGCCTCCTGCACCTAGGTCAGTGTTC
CTCCCAGTTACAGTCTAAACTGGAATGGCAGGCAAAGCCCCTGTGGAAGG
GGAAGGTGAAGCTCAAATCAAAGCTCNNCCAGAGACTTTCCAGATATCTG
AAGAAGTCCTGATGTCACTGCCCCGGTCCTTCCCCAGGTAGAGCAACACT
CCTCGCCGCAACCCAACTGGCTCTCCTTACTTTCTACACACACACACACAC
ACACACACACACACACACACACACACACACAAATCCAAGACAACACTACTA
AGGCTTCTTTGGGAGGGGGAAGTAGGGATAGGTAAGAGGAAAGTAAGGG
ACCTCCTATCCAGCCTCCATGGAATCCTGACTTCTTTTCCTTGTTATTTCAA
CTTCTTCCACCCCATCTTTTAAACTTTAGACTCCAGCCACAGAAGCTTACA
ACTAAAAGAAACTCTAAGGCCAATTTAATCCAAGGTTTCATTCTATGTGCT
GGAGATGGTGTACAGTAGGGTGAGGAAACCAAATTCTCAGTTGGCACTGG
TG

### IL10

SNP name and number (in ncbi database):
   IL10 T-819C (position 377764 on NW_001838536), no rs number in ncbi SNP database.
Gene name and number (in ncbi database):
   IL10, interleukin 10, NM_000572 (mRNA transcript) from Homo sapiens chromosome 1
   Genomic contig (NW_001838536, nucleotides 372078-376969).
   Also known as: CSIF, TGIF, IL-10, IL10A, MGC126450, MGC126451

### 27. Sequence of the WT T allele:

TATGTGCTGGAGATGGTGTACAGTAGGGTGAGGAAACCAAATTCTCAGT
TAGCACTGGTGTACCCTTGTACAGGTGATGTAACATCTCTGTGCCTCAGT
TTGCTCACTATAAAATAGAGACGGTAGGGGTCATGGTGAGCACTACCTG
AC

### 28. Sequence of the mutant C allele:

TATGTGCTGGAGATGGTGTACAGTAGGGTGAGGAAACCAAATTCTCAGT
TAGCACTGGTGTACCCTTGTACAGGTGATGCAACATCTCTGTGCCTCAGT
TTGCTCACTATAAAATAGAGACGGTAGGGGTCATGGTGAGCACTACCTG
AC

### IL1RN

SNP name and number (in ncbi database):
   IL1RN 86-bp tandem repeat [short tandem repeat (STR); microsatellite) polymorphism, (position 329879 on NW_001838841), rs2234663.
Gene name and number (in ncbi database):
   interleukin 1 receptor antagonist, NM_173842, NM_173841, NM_000577 and NM_173843 (mRNA transcript variants 1-4) from Homo sapiens chromosome 2 genomic contig (NW_001838841)
   Also known as: IRAP; IL1F3; IL1RA; IL-1ra3; ICIL-1RA; MGC10430

### 29. Instead of the normal WT sequence, for reasons given below, a protein sequence is provided instead:

1 maladlyeeg gggggegedn adsketicrp sgrksskmqa friwdvnqkt fylrnnqlva
61 gylqgpnvnl eekidvvpie phalflgihg gkmclscvks gdetrlqlea vnitdlsenr
121 kqdkrfafir sdsgpttsfe saacpgwflc tameadqpvs ltnmpdegvm vtkfyfqede

No WT nucleotide sequence is provided because the below sequence which is bold and underlined font is repeated 2/3/4/5/6 times although of course the present invention is not limited to such a number of repeats.

Intron 2 short tandem repeat (an 86-bp tandem repeat (highlighted), occurs 2/3/4/5/6 times:

### 30.

ACTCCTATTGACCTGGAGCACAGGT[(ATCCTGGGGAAAGTGAGG
GAAATATGGACATCACATGGAACAACATCCAGGAGACTCAGGC
CTCTAGGAGTAACTGGGTAGTGTGC)X2/3/4/5/6]TTGGTTTAATCT
TCTATTTACCTGC

### IL6

SNP name and number (in NCBI SNP database):
   IL6 G-174C, rs56588968 (position 16492950 on NW_001839003)
Gene name and number (in NCBI nucleotide database):
   interleukin 6 (interferon, beta 2), NM_000600 (mRNA transcript) from *Homo sapiens* chromosome 7 genomic contig (NW_001839003).
   Also known as: HGF; HSF; BSF2; IL-6; IFNB2

### 31. Sequence of the WT allele (reversed): G (C-reverse)-174:

GGAAAATCCCACATTTGATAAATCTTTGTTGGAGGGTGAGGGTGGGGCCA
GAGCGGGTGGGGCTGATTGGAAACCTTATTAAGATTGTGCAATGTGACGT
CCTTTAGCATCGCAAGACACAACTAGGGGGAAAAGTGCAGCTTAGGTCGT
CATTGAGGCTAGCGCTAAGAAGCAGAACCACTCTTCCTTTACTTTCTTTTT
TTCTTTTATTAGTGACTCAGCACTTTGGCATGTCTT

### 32. Sequence of the mutant (SNP bearing) allele: C (G-reverse)-174:

GGAAAATCCCACATTTGATAAATCTTTGTTGGAGGGTGAGGGTGGGGCCA
GAGCGGGTGGGGCTGATTGGAAACCTTATTAAGATTGTGCAATGTGACGT
CCTTTAGCATGGCAAGACACAACTAGGGGGAAAAGTGCAGCTTAGGTCGT
CATTGAGGCTAGCGCTAAGAAGCAGAACCACTCTTCCTTTACTTTCTTTTT
TTCTTTTATTAGTGACTCAGCACTTTGGCATGTCTT

### TNF

SNP name and number (in NCBI SNP database):
   TNF G-318A, rs361525 (nucleotide number 104675 on NT_113894)
Gene name and number (in NCBI nucleotide database):
   tumor necrosis factor (TNF superfamily, member 2), NM_000594 (mRNA transcript) from
   *Homo sapiens* chromosome 6 genomic contig (NT_113894, nucleotides 104924-107688).
   Also known as: DIF; TNFA; TNFSF2; TNF-alpha

### 33. Sequence of the WT allele: G-318

CAGTGGGGTCTGTGAATTCCCGGGGGTGATTTCACTCCCCGGGGCTGTCCC
AGGCTTGTCCCTGCTACCCCCACCCAGCCTTTCCTGAGGCCTCAAGCCTGC
CACCAAGCCCCCAGCTCCTTCTCCCCGCAGGGACCCAAACACAGGCCTCA
GGACTCAACACAGCTTTTCCCTCCAACCCCGTTTTCTCTCCCTCAAGGACT
CAGCTTTCTGAAGCCCCTCCCAGTTCTAGTTCTATCTTTTTCCTGCATCCTG
TCTGGAAGTTAGAAGGAAACAGACCACAGACCTGGTCCCCAAAAGAAAT
GGAGGCAATAGGTTTTGAGGGGCATGGGGACGGGGTTCAGCCTCCAGGGT
CCTACACACAAATCAGTCAGTGGCCCAGAAGACCCCCCTCGGAATCGGAG
CAGGGAGGATGGGGAGTGTGAGGGGTATCCTTGATGCTTGTGTGTCCCCA
ACTTTCCAAATCCCCGCCCCCGCGATGGAGAAGAAACCGAGACAGAAGGT
GCAGGGCCCACTACCGCTTCCTCCAGATGAGCTCATGGGTTTCTCCACCAA
GGAAGTTTTCCGCTGGTTGAATGATTCTTTCCCCGCCCTCCTCTCGCCCCA
GGGACATATAAAGGCAGTTGTTGGCACACCCAGCCAGCAGACGCTCCCTC
AGCAAGGACAGCAGAGGACCAGCTAAGAGGGAGAGAAGCAACTACAGA

CCCCCCCTGAAAACAACCCTCAGACGCCACATCCCCTGACAAGCTGCCAG
GCAGGTTCTCTTCCTCTCACATACTGACCCACGGCTCCACCCTCTCT

### 34. Sequence of the mutant (SNP bearing) allele: A-318:

CAGTGGGGTCTGTGAATTCCCGGGGGTGATTTCACTCCCCGGGGCTGTCCC
AGGCTTGTCCCTGCTACCCCCACCCAGCCTTTCCTGAGGCCTCAAGCCTGC
CACCAAGCCCCCAGCTCCTTCTCCCCGCAGGGACCCAAACACAGGCCTCA
GGACTCAACACAGCTTTTCCCTCCAACCCCGTTTTCTCTCCCTCAAGGACT
CAGCTTTCTGAAGCCCCTCCCAGTTCTAGTTCTATCTTTTTCCTGCATCCTG
TCTGGAAGTTAGAAGGAAACAGACCACAGACCTGGTCCCCAAAAGAAAT
GGAGGCAATAGGTTTTGAGGGGCATGGGGACGGGGTTCAGCCTCCAGGGT
CCTACACACAAATCAGTCAGTGGCCCAGAAGACCCCCCTCGGAATCAGAG
CAGGGAGGATGGGGAGTGTGAGGGGTATCCTTGATGCTTGTGTGTCCCCA
ACTTTCCAAATCCCCGCCCCCGCGATGGAGAAGAAACCGAGACAGAAGGT
GCAGGGCCCACTACCGCTTCCTCCAGATGAGCTCATGGGTTTCTCCACCAA
GGAAGTTTTCCGCTGGTTGAATGATTCTTTCCCCGCCCTCCTCTCGCCCCA
GGGACATATAAAGGCAGTTGTTGGCACACCCAGCCAGCAGACGCTCCCTC
AGCAAGGACAGCAGAGGACCAGCTAAGAGGGAGAGAAGCAACTACAGA
CCCCCCCTGAAAACAACCCTCAGACGCCACATCCCCTGACAAGCTGCCAG
GCAGGTTCTCTTCCTCTCACATACTGACCCACGGCTCCACCCTCTCT

### IL1B

SNP name and number (in NCBI SNP database):
   IL1B Phe (F)105 Phe (F), [C315T according to mRNA transcript(NM_000576), nucleotide numbering starts at the start codon, rs1143634
Gene name and number (in NCBI nucleotide database):
   interleukin 1, beta, NM_000576 (mRNA transcript)
   Also known as: IL-1; IL1F2; IL1-BETA

### 35. Sequence of the WT allele: C315

TAGTGGAAACTATTCTTAAAGAAGATCTTGATGGCTACTGACATTTGCAAC
TCCCTCACTCTTTCTCAGGGGCCTTTCACTTACATTGTCACCAGAGGTTCG
TAACCTCCCTGTGGGCTAGTGTTATGACCATCACCATTTACCTAAGTAGC
TCTGTTGCTCGGCCACAGTGAGCAGTAATAGACCTGAAGCTGGAACCCAT
GTCTAATAGTGTCAGGTCCAGTGTTCTTAGCCACCCCACTCCCAGCTTCAT
CCCTACTGGTGTTGTCATCAGACTTTGACCGTATATGCTCAGGTGTCCTCC
AAGAAATCAAATTTTGCCGCCTCGCCTCACGAGGCCTGCCCTTCTGATTTT
ATACCTAAACAACATGTGCTCCACATTTCAGAACCTATCTTCTTCGACACA
TGGGATAACGAGGCTTATGTGCACGATGCACCTGTACGATCACTGAACTG
CACGCTCCGGGACTCACAGCAAAAAAGCTTGGTGATGTCTGGTCCATATG
AACTGAAAGCTCTCCACCTCCAGGGACAGGATATGGAGCAACAAGGTAA
ATGGAAACATCCTGGTTTCCCTGCCTGGCCTCCTGGCAGCTTGCTAATTCT

CCATGTTTAAACAAAGTAGAAAGTTAATTTAAGGCAAATGATCAACACA
AGTGAAAAAAAATATTAAAAAGGAATATACAAACTTTGGTCCTAGAAATG
GCACATTTGATTGCACTGGCCAGTGCATTTGTTAACAGGAGTGTGACCCTG
AGA

### 36. Sequence of the mutant allele:T315:

TAGTGGAAACTATTCTTAAAGAAGATCTTGATGGCTACTGACATTT
GCAACTCCCTCACTCTTTCTCAGGGGCCTTTCACTTACATTGTCACC
AGAGGTTCGTAACCTCCCTGTGGGCTAGTGTTATGACCATCACCAT
TTTACCTAAGTAGCTCTGTTGCTCGGCCACAGTGAGCAGTAATAGA
CCTGAAGCTGGAACCCATGTCTAATAGTGTCAGGTCCAGTGTTCTT
AGCCACCCCACTCCCAGCTTCATCCCTACTGGTGTTGTCATCAGACT
TTGACCGTATATGCTCAGGTGTCCTCCAAGAAATCAAATTTTGCCG
CCTCGCCTCACGAGGCCTGCCCTTCTGATTTATACCTAAACAACAT
GTGCTCCACATTTCAGAACCTATCTTCTTTGACACATGGGATAACG
AGGCTTATGTGCACGATGCACCTGTACGATCACTGAACTGCACGCT
CCGGGACTCACAGCAAAAAAGCTTGGTGATGTCTGGTCCATATGAA
CTGAAAGCTCTCCACCTCCAGGGACAGGATATGGAGCAACAAGGT
AAATGGAAACATCCTGGTTTCCCTGCCTGGCCTCCTGGCAGCTTGC
TAATTCTCCATGTTTTAAACAAAGTAGAAAGTTAATTTAAGGCAAA
TGATCAACACAAGTGAAAAAAAATATTAAAAAGGAATATACAAAC
TTTGGTCCTAGAAATGGCACATTTGATTGCACTGGCCAGTGCATTT
GTTAACAGGAGTGTGACCCTGAGA

### CRP

SNP name and number (in NCBI SNP database):
   CRP Leu (L)184 Leu (L), [G552C according to mRNA transcript (NM_000567), nucleotide numbering starts at the start codon, position 1726559 on NW_001838531], rs 1800947
Gene name and number (in NCBI nucleotide database):
   C-reactive protein, pentraxin-related, NM_000567 (mRNA transcript) from *Homo sapiens*
   chromosome 1 genomic contig (NW_001838531).
   Also known as: PTX1; MGC88244; MGC149895 PTX1; MGC88244; MGC149895

### 37. Sequence of the WT allele: G552:

TTGGTCTAAGGATATAGGATACAGTTTTACAGTGGGTGGGTCTGAAATATT
ATTCGAGGTTCCTGAAGTCACAGTAGCTCCAGTACACATTTGTACAAGCT
GGGAGTCCGCCTCAGGGATCGTGGAGTTCTGGGTAGATGGGAAGCCCAGG
GTGAGGAAGAGTCTGAAGAAGGGATACACTGTGGGGGCAGAAGCAAGCA
TCATCTTGGGGCAGGAGCAGGATTCCTTCGGTGGGAACTTTGAAGGAAGC
CAGTCCCTGGTGGGAGACATTGGAAATGTGAACATGTGGGACTTTGTGCT

GTCACCAGATGAGATTAACACCATCTATCTTGGCGGGCCCTTCAGTCCTA
ATGTCCTGAACTGGCGGGCACTGAAGTATGAAGTGCAAGGCGAAGTGTTC
ACCAAACCCCAGCTGTGGCCCTGAGGCCCAGCTGTGGGTCCTGAAGGTAC
CTCCCGGTTTTTACACCGCATGGGCCCCACGTCTCTGTCTCTGGTACCTC
CCGCTTTTTACACTGCATGGTTCCCACGTCTCTGTCTCTGGGCCTTTGTTC
CCCTATATGCATTGCAGGCCTGCTCCACCCTCCTCAGCGCCTGAGAAT

### 38. Sequence of the mutant (SNP bearing) allele: C552:

TTGGTCTAAGGATATAGGATACAGTTTTACAGTGGGTGGGTCTGAAATATT
ATTCGAGGTTCCTGAAGTCACAGTAGCTCCAGTACACATTTGTACAAGCT
GGGAGTCCGCCTCAGGGATCGTGGAGTTCTGGGTAGATGGGAAGCCCAGG
GTGAGGAAGAGTCTGAAGAAGGGATACACTGTGGGGGCAGAAGCAAGCA
TCATCTTGGGGCAGGAGCAGGATTCCTTCGGTGGGAACTTTGAAGGAAGC
CAGTCCCTGGTGGGAGACATTGGAAATGTGAACATGTGGGACTTTGTGCT
CTCACCAGATGAGATTAACACCATCTATCTTGGCGGGCCCTTCAGTCCTAA
TGTCCTGAACTGGCGGGCACTGAAGTATGAAGTGCAAGGCGAAGTGTTCA
CCAAACCCCAGCTGTGGCCCTGAGGCCCAGCTGTGGGTCCTGAAGGTACC
TCCCGGTTTTTTACACCGCATGGGCCCCACGTCTCTGTCTCTGGTACCTCC
CGCTTTTTTACACTGCATGGTTCCCACGTCTCTGTCTCTGGGCCTTTGTTCC
CCTATATGCATTGCAGGCCTGCTCCACCCTCCTCAGCGCCTGAGAAT

### NPR1

SNP name and number (in NCBI SNP database):
   NPR1 -67±GCTGAGCC (insertion/deletion polymorphism), [-67 nubering is according to the start codon (-1 is the first nucleotide upstream, -67 is nucleotide no. 356 according to mRNA transcript NM_000906), no rs number in NCBI SNP database.
Gene name and number (in NCBI nucleotide database):
   natriuretic peptide receptor A/guanylate cyclase A (atrionatriuretic peptide receptor A),
   NM_000906) (mRNA transcript) from *Homo sapiens* chromosome 1 genomic contig (NW_001838529).
   Also known as: ANPa; NPRA; ANPRA; GUC2A; GUCY2A

### 39. Sequence of WT allele + GCTGAGCC:

CTCACGCACGCTACAAACACACACTCCTCTTTCCTCCCTCGCGCGCCCTCTCTCATC
CTTCTTCACGAAGCGCTCACTCGCACCCTTTCTCTCTCTCTCTCTCTCTCTAACA
CGCACGCACACTCCCAGTTGTTCACACTCGGGTCCTCTCCAGCCCGACGTTCTCCTG
GCACCCACCTGCTCCGCGGCGCCCTGCGCGCCCCCCTCGGTCGCGCCCCTTGCGCTC
TCGGCCCAGACCGTCGCAGCTACAGGGGGCCTCGAGCCCCGGGGTGAGCGTCCCCGT

CCCGCTCCTGCTCCTTCCCATAGGACGCGCCTGATGCCTGGGACCGGCCGCTGAGC
CCAAGGGGACCGAGGAGGCCATGGTAGGAGCGCTCGCCTGCTGCGGTGCCCGCTGAG
GCCATGCCGGGGCCCCGGCGCCCCGCTGGCTCCCGCCTGCGCCTGCTCCTGCTCCTG
CTGCTGCCGCCGCTGCTGCTGCTGCTCCGGGGCAGCCACGCGGGCAACCTGACGGTA
GCCGTGGTACTGCCGCTGGCCAATACCTCGTACCCCTGGTCGTGGGC

### 40. Sequence of the mutant (polymorphism bearing) allele -GCTGAGCC:

CTCACGCACGCTACAAACACACACTCCTCTTTCCTCCCTCGCGCGCCCTCTCTCATC
CTTCTTCACGAAGCGCTCACTCGCACCCTTTCTCTCTCTCTCTCTCTCTCTAACA
CGCACGCACACTCCCAGTTGTTCACACTCGGGTCCTCTCCAGCCCGACGTTCTCCTG
GCACCCACCTGCTCCGCGGCGCCCTGCGCGCCCCCCTCGGTCGCGCCCCTTGCGCTC
TCGGCCCAGACCGTCGCAGCTACAGGGGGCCTCGAGCCCCGGGGTGAGCGTCCCCGT
CCCGCTCCTGCTCCTTCCCATAGGGACGCGCCTGATGCCTGGGACCGGCCXXXXXXX
XCAAGGGGACCGAGGAGGCCATGGTAGGAGCGCTCGCCTGCTGCGGTGCCCGCTGAG
GCCATGCCGGGGCCCCGGCGCCCCGCTGGCTCCCGCCTGCGCCTGCTCCTGCTCCTG
CTGCTGCCGCCGCTGCTGCTGCTGCTCCGGGGCAGCCACGCGGGCAACCTGACGGTA
GCCGTGGTACTGCCGCTGGCCAATACCTCGTACCCCTGGTCGTGGGC

### NPR3

SNP name and number (in NCBI SNP database):
   NPR3 C-251A, rs9716700 (position 11318530 on NW_001838929)
Gene name and number (in NCBI nucleotide database):
   natriuretic peptide receptor C/guanylate cyclase C (atrionatriuretic peptide receptor C), NM-000908 (mRNA transcript) from *Homo sapiens* chromosome 5
   genomic contig (NW_001838529).
   Also known as: CSIF NPRC; ANPRC; GUCY2B

### 41. Sequence of the WT allele C-251:

AATCAATGAGATCAAATGCGAGGAGATGCACCGTCAATTACAAACACTTGGACAAG
TCTAACTTTTTTTCTTCTACAAAAACGCTTTCAAAAGCAACCTTAGCAACGCCCA
AATAAGAAGCCACCTCTAAGCAAAATAGTATATGTATAAACGGAGGGCGAATATATA
CAAGTATATATATATGTATATTACAGACGCACAGGTTTACACCCGGTGAACTTTTTC
TTTTTCTTTTTCTTTTTTTTTTAAGAAAAACTATGACATTGCAGAGAAGGACGCTTC
CTCTCTATCTTTTGGCGCATTAGTGAAGGGGGTATTCTATTTTGTTAAAGCGCCCAA
GGGGGCGCAGGGACCTTGGAGAGAAGAGTGGGAGGAAAGAGGAAGGTGGGTGGGG
GGCAGAGGGCGAGTCGGCGGCGGCGAGGG

### 42. Sequence of the mutant allele A-251:

AATCAATGAGATCAAATGCGAGGAGATGCACCGTCAATTACAAACACTTGGACAAG
TCT

AACTTTTTTTTCTTCTACAAAAACGCTTTCAAAAGCAACCTTAGCAACGCCCAAAT
AAG
AAGCCACCTCTAAGCAAAATAGTATATGTATAAACGGAGGCGAATATATACAAGTA
TAT
ATATATGTATATTACAGACGAACAGGTTTACACCCGGTGAACTTTTTCTTTTTCTTT
TTCTTTTTTTTTAAGAAAAACTATGACATTGCAGAGAAGGACGCTTCCTCTCTATC
TTTTGGCGCATTAGTGAAGGGGGTATTCTATTTTGTTAAAGCGCCCAAGGGGGCGCA
GGGACCTTGGAGAGAAGAGTGGGGAGGAA
AGAGGAAGGGTGGGTGGGGGGCAGAGGGCGAGTCGGCGGCGGCGAGGG

### NOS3

SNP name and number (in NCBI SNP database):
   NOS3 Glu (E)298Asp (D), [G894T (according to mRNA transcript NM_000603, nucleotide numbering starts at the start codon, position on NW 001839088 is 1803394 ],rs57135373
Gene name and number (in NCBI nucleotide database):
   nitric oxide synthase 3 (endothelial cell), NM_000603 (mRNA transcript) from *homo sapiens* chromosome 7 genomic contig (NW 001839088)
   Also known as: eNOS; ECNOS; NOS III

### 43. Sequence of WT allele [Glu (E)298;

### G894]:

GTGGTCACGGAGACCCAGCCAATGAGGGACCCTGGAGATGAAGGCAGGAGACAGTGG
ATGGAGGGGTCCCTGAGGAGGGCATGAGGCTCAGCCCAGAACCCCTCTGGCCCAC
TCCCCACAGCTCTGCATTCAGCACGGCTGGACCCCAGGAAACGGTCGCTTCGACGTG
CTGCCCCTGCTGCTGCAGGCCCCAGATGAGCCCCCAGAACTCTTCCTTCTGCCCCCC
GAGCTGGTCCTTGAGGTGCCCCTGGAGCACCCCACGTGAGCACCAAAGGGATTGACT
GGGTGGGATGGAGGGGGCCATCCCTGAGCCTCTCAAGAAGGCCTGCAAGGGGGTGC
TGATCCCACACCCCAACACCCCCAGGCTGGAGTGGTTTGCAGCCCTGGCCTGCGCT
GG

### 44. Sequence of mutant allele [Asp (D)298;

### T894]:

GTGGTCACGGAGACCCAGCCAATGAGGGACCCTGGAGATGAAGGCAGGAGACAGTGG
ATGGAGGGGTCCCTGAGGAGGGCATGAGGCTCAGCCCCAGAACCCCCTCTGGCCCAC
TCCCCACAGCTCTGCATTCAGCACGGCTGGACCCCAGGAAACGGTCGCTTCGACGTG
CTGCCCCTGCTGCTGCAGGCCCCAGATGATCCCCCAGAACTCTTCCTTCTGCCCCCC

GAGCTGGTCCTTGAGGTGCCCCTGGAGCACCCCACGTGAGCACCAAAGGATTGACT
GGGTGGGATGGAGGGGGCCATCCCTGAGCCTCTCAAGAAGGCCTGCAAGGGGGTGC
TGATCCCACACCCCAACACCCCCAGGCTGGAGTGGTTTGCAGCCCTGGGCCTGCGCT
GG

### SERPINE1

SNP name and number (in NCBI SNP database):
   PAI1 -A/G (nucleotide deleted between nucleotides 1343159:1343160 from NW_001839067), rs1799889.
Gene name and number (in NCBI nucleotide database):
   plasminogen activator inhibitor-1, plasminogen activator inhibitor, type I, serine (or cysteine) proteinase inhibitor, clade E (nexin, plasminogen activator inhibitor type 1), member 1, NM_000602 (mRNA transcript) fro *Homo sapiens* chromosome 7 genomic contig (NW_001839067).
   Also known as: PAI; PAI1; PAI-1; PLANH1

### 45. Sequence of the WT allele +G:

CCCTAAAAGC ACACCCTGCA AACCTGCCAT GAATTGACAC TCTGTTTCTA
TCCCTTTTCC
CCTTGTGTCT GTGTCTGGAG GAAGAGGATA AAGGACAAGC TGCCCCAAGT
CCTAGCGGGC
AGCTCGAGGA AGTGAAACTT ACACGTTGGT CTCCTGTTTC CTTACCAAGC
TTTTACCATG
GTAACCCCTG GTCCCGTTCA GCCACCACCA CCCCACCCAG CACACCTCCA
ACCTCAGCCA
GACAAGGTTG TTGACACAAG AGAGCCCTCA GGGGCACAGA GAGAGTCTGG
ACACGTGGGG
G
AGTCAGCCGT GTATCATCGG AGGCGGCCGG GCACATGGCA GGGATGAGGG
AAAGACCAAG
AGTCCTCTGT TGGGCCCAAG TCCTAGACAG ACAAAACCTA GACAATCACG
TGGCTGGCTG
CATGCCCTGT GGCTGTTGGG CTGGGCCCAG GAGGAGGGAG GGGCGCTCTT
TCCTGGAGGT
GGTCCAGAGC ACCGGGTGGA CAGCCCTGGG GGAAAACTTC CACGTTTTGA
TGGAGGTTAT
CTTTGATAAC TCCACAGTGA CCTGGTTCGC CAAAGGAAAA GCAGGCAACG
TGAGCTGTTT

### 46. Sequence of the mutant allele -G:

CCCTAAAAGC ACACCCTGCA AACCTGCCAT GAATTGACAC TCTGTTTCTA
TCCCTTTTCC
CCTTGTGTCT GTGTCTGGAG GAAGAGGATA AAGGACAAGC TGCCCCAAGT
CCTAGCGGGC

AGCTCGAGGA AGTGAAACTT ACACGTTGGT CTCCTGTTTC CTTACCAAGC
TTTTACCATG
GTAACCCCTG GTCCCGTTCA GCCACCACCA CCCCACCCAG CACACCTCCA
ACCTCAGCCA
GACAAGGTTG TTGACACAAG AGAGCCCTCA GGGGCACAGA GAGAGTCTGG
ACACGTGGGG
X
AGTCAGCCGT GTATCATCGG AGGCGGCCGG GCACATGGCA GGGATGAGGG
AAAGACCAAG
AGTCCTCTGT TGGGCCCAAG TCCTAGACAG ACAAAACCTA GACAATCACG
TGGCTGGCTG
CATGCCCTGT GGCTGTTGGG CTGGGCCCAG GAGGAGGGAG GGCGCTCTT
TCCTGGAGGT
GGTCCAGAGC ACCGGGTGGA CAGCCCTGGG GGAAAACTTC CACGTTTTGA
TGGAGGTTAT
CTTTGATAAC TCCACAGTGA CCTGGTTCGC CAAAGGAAAA GCAGGCAACG
TGAGCTGTTT

### PLA2G7

SNP name and number (in NCBI SNP database):
   PLA2G7 G824T (824 is nucleotide number according to mRNA transcript NM_005084, nucleotide numbering starts at the start codon, 25966 is the nucleotide number in NW_923073), no rs number in NCBI SNP database.
Gene name and number (in NCBI nucleotide database):
   phospholipase A2, group VII (platelet-activating factor acetylhydrolase, plasma), NM_000504 (mRNA transcript) from *homo sapiens* chromosome 6 genomic contig (NW_923073).
   Also known as: PAFAH; LDL-PLA2

### 47. Sequence of the WT allele G824:

TATCCCTCAAGCAGCCACTCTCTTCTGTATCCTTGCCTTTGTACATGTTGTCCCCTT
GGCCTGACACACCCTTCCCCTTGCCTAACTCCTACCTAATTTCAAGACTCCAGTTGA
GCATCACCTCCTCTAAGAAGCTTTCTTGGACCCCAATACCCACTTCTGGACTGGGCT
CGCTGTCTGTCATGTGTGCTCCTTTGTACCACTGTACTGTATTGCATCATGCCTCTG
TATAACTTTCTTCCCTGATGGACTGCAAACTCACTGAAATGAGACTGCAGTACCTGG
CACAGAGTAGGTACTCAATAAATACTCATGGAATGAACAAACAAATAAACATGGGGT
GAGGAGAGGCAGAAGTCAGAACTGATGTTGAAGTTTCCAGTGTGGGTGACTACAAAG
AACATTAAGTTTACTTTCAAACCTTTACATATGTTATATATATGTGTAAATGTGTTT
TATATGTGTATATAGATGTATATGTGTGTATGGTATGTATAAATGTATGTGTGTATA
TGTATATTCTATTTTATAAGAAATCAATGTATTTAACCATCCCCATGAAATGAACAA
TTATATGATTGACAAAATCATTTCTTCTAACACCACGAAATAGCTATAAATTTATAT
CATGCTTTTTCAAATAGGACTCTAATAGCAGTAATTGGACATTCTTTTGGTGGAGCA
ACGGTTATTCAGACTCTTAGTGAAGATCAGAGATTCAGGTAAGAAAATAAGATAGTA
AAGCAAGAGAATAGTAAATTATTGGAAGAAATTATATTGTGAGATATAATTTTTTAT
TCAAATTCTTAGTGAAGAAGGGATCTCTTGGAGTTTATAAGGCTATTCTTTTGCCCC

CATAAAATACTCTATATACATTTTCCTAGGCTAAAACATCTACCTCTCCTGCTATTA
AAATCTCCCCCTACTCCCATAAGTTTTCCCTCATTATTCTTGTTTACCCAAGGGGTT
AACACTTTTCACTGAAAAATTTATCTTTATATAATTTTTTGTGACATAATGATTGTG
ATAATAATA

### 48. Sequence of the mutant allele T824:

TATCCCTCAAGCAGCCACTCTCTTCTGTATCCTTGCCTTTGTACATGTTGTCCCCTT
GGCCTGACACACCCTTCCCCTTGCCTAACTCCTACCTAATTTCAAGACTCCAGTTGA
GCATCACCTCCTCTAAGAAGCTTTCTTGGACCCCAATACCCACTTCTGGACTGGGCT
CGCTGTCTGTCATGTGTGCTCCTTTGTACCACTGTACTGTATTGCATCATGCCTCTG
TATAACTTTCTTCCCTGATGGACTGCAAACTCACTGAAATGAGACTGCAGTACCTGG
CACAGAGTAGGTACTCAATAAATACTCATGGAATGAACAAACAAATAAACATGGGGT
GAGGAGAGGCAGAAGTCAGAACTGATGTTGAAGTTTCCAGTGTGGGTGACTACAAAG
AACATTAAGTTTACTTTCAAACCTTTACATATGTTATATATATGTGTAAATGTGTTT
TATATGTGTATATAGATGTATATGTGTATGGTATGTATAAATGTATGTGTGTATA
TGTATATTCTATTTTATAAGAAATCAATGTATTTAACCATCCCCATGAAATGAACAA
TTATATGATTGACAAAATCATTTCTTCTAACACCACGAAATAGCTATAAATTTATAT
CATGCTTTTTCAAATAGGACTCTAATAGCAGTAATTGGACATTCTTTTGGTGGAGCA
ACGTTTATTCAGACTCTTAGTGAAGATCAGAGATTCAGGTAAGAAAATAAGATAGTA
AAGCAAGAGAATAGTAAATTATTGGAAGAAATTATATTGTGAGATATAATTTTTTAT
TCAAATTCTTAGTGAAGAAGGATCTCTTGGAGTTTATAAGGCTATTCTTTTGCCCC
CATAAAATACTCTATATACATTTTCCTAGGCTAAAACATCTACCTCTCCTGCTATTA
AAATCTCCCCCTACTCCCATAAGTTTTCCCTCATTATTCTTGTTTACCCAAGGGGTT
AACACTTTTCACTGAAAAATTTATCTTTATATAATTTTTTGTGACATAATGATTGTG
ATAATAATA

### FGF2

SNP name and number (in NCBI SNP database):
   FGF2 T-553A (nucleotide number 4320453 in NW_0018389203), rs 308398
Gene name and number (in NCBI nucleotide database):
   fibroblast growth factor 2 (basic), NM_002006 (mRNA transcript) from *homo sapiens* chromosome 4 genomic contig (NW_0018389203).
   Also known as: BFGF; FGFB; HBGF-2

### 49. Sequence of the WT allele T-553:

ATCTCCCACA CACTCAACAT TATGTGTTGC ACACAGTAGG TACTCAATAC
ATGCAAGTTT
TCTGAATAGA TATTTTCCTA GTCATCTGTG GCACCTGCTA TATCCTACTG
AAAATTACCA
AAATGCAATT AACTTCAATT TTACATTTGG GATTTACAGA AAATAACTCT
CTCTCCAAGA

AATGCATAAC AATTTAGCTA GGGCAAATGC CAGGTCCGAG TTAAGACATT
AATGCGCTTC
GATCGCGATA AGGATTTATC CTTATCCCCA TCCTCATCTT TCTGCGTCGT
CTAATTCAAG
T
TAGGTCAGTA AAGGAAACCT TTTCGTTTTA GCAACCCAAT CTGCTCCCCT
TCTCTGGCCT
CTTTCTCTCC TTTTGTTGGT AGACGACTTC AGCCTCTGTC CTTTAATTTT
AAAGTTTATG
CCCCACTTGT ACCCCTCGTC TTTTGGTGAT TTAGAGATTT TCAAAGCCTG
CTCTGACACA
GACTCTTCCT TGGATTGCAA CTTCTCTACT TTGGGGTGGA AACGGCTTCT
CCGTTTTGAA
ACGCTAGCGG GGAAAAAATG GGGGAGAAAG TTGAGTTTAA ACTTTTAAAA
GTTGAGTCAC

### 50. Sequence of the mutant allele A-553:

ATCTCCCACA CACTCAACAT TATGTGTTGC ACACAGTAGG TACTCAATAC
ATGCAAGTTT
TCTGAATAGA TATTTTCCTA GTCATCTGTG GCACCTGCTA TATCCTACTG
AAAATTACCA
AAATGCAATT AACTTCAATT TTACATTTGG GATTTACAGA AAATAACTCT
CTCTCCAAGA
AATGCATAAC AATTTAGCTA GGGCAAATGC CAGGTCCGAG TTAAGACATT
AATGCGCTTC
GATCGCGATA AGGATTTATC CTTATCCCCA TCCTCATCTT TCTGCGTCGT
CTAATTCAAG
A
TAGGTCAGTA AAGGAAACCT TTTCGTTTTA GCAACCCAAT CTGCTCCCCT
TCTCTGGCCT
CTTTCTCTCC TTTTGTTGGT AGACGACTTC AGCCTCTGTC CTTTAATTTT
AAAGTTTATG
CCCCACTTGT ACCCCTCGTC TTTTGGTGAT TTAGAGATTT TCAAAGCCTG
CTCTGACACA
GACTCTTCCT TGGATTGCAA CTTCTCTACT TTGGGGTGGA AACGGCTTCT
CCGTTTTGAA
ACGCTAGCGG GGAAAAAATG GGGGAGAAAG TTGAGTTTAA ACTTTTAAAA
GTTGAGTCAC

### GNB3

SNP name and number (in NCBI SNP database):
   GNB3 Ser (S)275Ser (S), [C825T (according to mRNA transcript NM_002075, nucleotide numbering starts at the start codon, position on NW 001838050 is 1363824], rs5443
Gene name and number (in NCBI nucleotide database):
   guanine nucleotide binding protein (G protein), beta polypeptide 3, NM_002075 (mRNA transcript) from *homo sapiens* chromosome 12 genomic contig (NW 001838050)

### 51. Sequence of the WT allele C825:

TCACTGCAGG CAAGCCTTGG TGCTCTTGCC TGCGACGTGG AAATGATGCC
TGCCTGCAGC
GCTGTATAGT GCAGAGCGGG CGAGGGGCAT AGGGAAGTCA CTGGCACGTG
GTATGTGTTG
GCAGGGCTGC TTCTCACCCC AAACCAAGGG AGGGACAGGC AGGGAGGCTG
AGAGCAGCGG
CTTGCCCTGG AGCTGTCAGG TGGGAGGCAG AGGGCGGGAG AGGCTGTGGG
CTGCCCAGGT
CTGATCCCTG ACCCACTTGC CACCCGTGCC CTCAGTTCTT CCCCAATGGA
GAGGCCATCT
GCACGGGCTC GGATGACGCT TCCTGCCGCT TGTTTGACCT GCGGGCAGAC
CAGGAGCTGA
TCTGCTTCTC CCACGAGAGC ATCATCTGCG GCATCACGTC
C
GTGGCCTTCT CCCTCAGTGG CCGCCTACTA TTCGCTGGCT ACGACGACTT
CAACTGCAAT
GTCTGGGACT CCATGAAGTC TGAGCGTGTG GGTAAGGGCC AGCCCTGGCT
GCTGCTTCCT
CAGCTGGAAG GACCCTCCCC AGCCCTCCCT CCCCATTCTG TACCCCCCAT
CAGCTCCCAT
TTCGGACTCT CTTACTGCTG TCCCTTGTCA CTGGGTGACT CCACCCCTGG
AATCCAGTAC
CCCTTGGTTC CCAACTAGGA CTGTTTTCCC TCAGTGTTGC TCTAAGCAGC
CTCTCTCCAC
TGCCCAATGC CATGACTGCT CCCTGCCCTA GGAGATCTGT GGACCATGAC
TGTCCAGTCA
GTTCTGGGTT CCTGGCATTT CAGGGGCACC CACTGAGAGG

### 52. Sequence of the mutant allele T825:

TCACTGCAGG CAAGCCTTGG TGCTCTTGCC TGCGACGTGG AAATGATGCC
TGCCTGCAGC
GCTGTATAGT GCAGAGCGGG CGAGGGGCAT AGGGAAGTCA CTGGCACGTG
GTATGTGTTG
GCAGGGCTGC TTCTCACCCC AAACCAAGGG AGGGACAGGC AGGGAGGCTG
AGAGCAGCGG
CTTGCCCTGG AGCTGTCAGG TGGGAGGCAG AGGGCGGGAG AGGCTGTGGG
CTGCCCAGGT
CTGATCCCTG ACCCACTTGC CACCCGTGCC CTCAGTTCTT CCCCAATGGA
GAGGCCATCT
GCACGGGCTC GGATGACGCT TCCTGCCGCT TGTTTGACCT GCGGGCAGAC
CAGGAGCTGA
TCTGCTTCTC CCACGAGAGC ATCATCTGCG GCATCACGTC
T
GTGGCCTTCT CCCTCAGTGG CCGCCTACTA TTCGCTGGCT ACGACGACTT
CAACTGCAAT
GTCTGGGACT CCATGAAGTC TGAGCGTGTG GGTAAGGGCC AGCCCTGGCT
GCTGCTTCCT
CAGCTGGAAG GACCCTCCCC AGCCCTCCCT CCCCATTCTG TACCCCCCAT
CAGCTCCCAT
TTCGGACTCT CTTACTGCTG TCCCTTGTCA CTGGGTGACT CCACCCCTGG
AATCCAGTAC

CCCTTGGTTC CCAACTAGGA CTGTTTTCCC TCAGTGTTGC TCTAAGCAGC
CTCTCTCCAC
TGCCCAATGC CATGACTGCT CCCTGCCCTA GGAGATCTGT GGACCATGAC
TGTCCAGTCA
GTTCTGGGTT CCTGGCATTT CAGGGGCACC CACTGAGAGG

### PPARA

SNP name and number (in NCBI SNP database):
   PPARA Leu (L)162Val(V), [C484G (according to mRNA transcript variants:
      NM_005036, NM_001001928, nucleotide numbering starts at the start codon, position on NW_ 001838753 is 233773, or position on NT_011523 is 1884980], rs1800206
Gene name and number (in NCBI nucleotide database):
   peroxisomeproliferator-activated receptor alpha, NM_005036 and NM_001001928 (mRNA transcript variants) from *homo sapiens* chromosome 22 genomic contig (NW 001838753 or NT_011523)
   Also known as: PPAR; NR1C1; hPPAR; MGC2237; MGC2452

### 53. Sequence of the WT allele L162 (C484):

CGCCTCAGCC TCCTAAAGTG CTGGGATTAC AGGCATGATC ACCATGCCTG
GCCTGGAATA
ACTTTTCTCT AAATTTTGTT CATTTAAAAA GAAACAATAA ATGAGCAACA
AAAAAGGTGA
GTAAAGCAAG TGCGCTGGTT TCTCAGTGGC CCAGGTCTTT AAATCCACTG
TGTATTACCC
TCACAGGGCT TCTTTCGGCG AACGATTCGA CTCAAGCTGG TGTATGACAA
GTGCGACCGC
AGCTGCAAGA TCCAGAAAAA GAACAGAAAC AAATGCCAGT ATTGTCGATT
TCACAAGTGC
C
TTTCTGTCGG GATGTCACAC AACGGTAGGT AAGGTGGCCC TGCACATTTT
CCCAGTTCGT
TCCTCAGTTC CCCTTCCTTG CTCCAAGGGA ACAGATCAAG CTATGGATGA
ATGTGCTTCA
ACATTTCACA CCCAAGTCAT TTTGTAATCA GAGTGGCCTA AGAAAATAAA
AGTCGCCCAG
GCGCGGTGGT TCACGCCTGT AATCCCAGCA CTTTGGGAGG CTGAGGTGGG
TGGATCACCT
CAGGTCAGGA GTTTGAGACC AGCCTGGCCA ATATGGTGAA ACCCCGTCTC
TACTAAGAAT

### 54. Sequence of the mutant alleleV162 (G484):

CGCCTCAGCC TCCTAAAGTG CTGGGATTAC AGGCATGATC ACCATGCCTG
GCCTGGAATA
ACTTTTCTCT AAATTTTGTT CATTTAAAAA GAAACAATAA ATGAGCAACA
AAAAAGGTGA
GTAAAGCAAG TGCGCTGGTT TCTCAGTGGC CCAGGTCTTT AAATCCACTG
TGTATTACCC
TCACAGGGCT TCTTTCGGCG AACGATTCGA CTCAAGCTGG TGTATGACAA
GTGCGACCGC
AGCTGCAAGA TCCAGAAAAA GAACAGAAAC AAATGCCAGT ATTGTCGATT
TCACAAGTGC
G
TTTCTGTCGG GATGTCACAC AACGGTAGGT AAGGTGGCCC TGCACATTTT
CCCAGTTCGT
TCCTCAGTTC CCCTTCCTTG CTCCAAGGGA ACAGATCAAG CTATGGATGA
ATGTGCTTCA
ACATTTCACA CCCAAGTCAT TTTGTAATCA GAGTGGCCTA AGAAAATAAA
AGTCGCCCAG
GCGCGGTGGT TCACGCCTGT AATCCCAGCA CTTTGGGAGG CTGAGGTGGG
TGGATCACCT
CAGGTCAGGA GTTTGAGACC AGCCTGGCCA ATATGGTGAA ACCCCGTCTC
TACTAAGAAT

### PPARA

SNP name and number (in NCBI SNP database):
   PPARA T/G(intron 1), [position on NW_ 001838753 is 178627, or position on NT_011523 is 1829973], rs135539
Gene name and number (in NCBI nucleotide database):
   peroxisome proliferator-activated receptor alpha, NM_005036 and NM_001001928 (mRNA transcript variants) from *homo sapiens* chromosome 22 genomic contig (NW 001838753 or NT_011523)
   Also known as: PPAR; NR1C1; hPPAR; MGC2237; MGC2452

### 55. Sequence of the WT allele T:

GTGGCTGCCC TGGAAGGCAC AGACCACTCA TGTCACGTCG TCCTGGGAAA
GGGGCTTCGA
GAAAGGCCTG CATTTGTCTA GGGAATCATC TACCTCTCAT TTCTATCAAA
CCAAATGGAG
TCAAGCTCGC CACAGCCAGG AGCCTGCTCT TCCCGCTTAT GTGACTGTGG
TGAAATCGTG
AGCATGAGGG CTCATTTGCT TTTCAGGCTA GAACTATCAG TGACGGAGCA
AAGGCTGAAG
CCACAACTAA GCAGGCAGTG TATCTTCAAT ATAGGTCATT AGATGTATGA
TTAGAGTTAA
T
AATCACCTAG GATTTAAATT CTGCTAACCT ATGTGGGTCA CAAGGAGTTT
AACTTGAGCG
CTTAATGCCT TTAAGATCAT AATCAGGAGA ACTAAAATGA GCTCTTGAGT
TTCTTGGATA

ATTTGTATTC ACTCTTTCCT CCCCCTGGAC TTGGTTCTTT AGTGAAAGGA
AATTCCGAAG
TTTAAAGACA ACGACATCTG GAGCCGCACA TTCCGTTCAC TGGCAGGTGC
TGCACACACG
CCCCTGCGAC CTCCGTGACC CTCTGTCCCC GCTCTTTCCC CTCTGCAGGC

TGCTCTCAGT

### 56. Sequence of the mutant allele G:

GTGGCTGCCC TGGAAGGCAC AGACCACTCA TGTCACGTCG TCCTGGGAAA
GGGGCTTCGA
GAAAGGCCTG CATTTGTCTA GGGAATCATC TACCTCTCAT TTCTATCAAA
CCAAATGGAG
TCAAGCTCGC CACAGCCAGG AGCCTGCTCT TCCCGCTTAT GTGACTGTGG
TGAAATCGTG
AGCATGAGGG CTCATTTGCT TTTCAGGCTA GAACTATCAG TGACGGAGCA
AAGGCTGAAG
CCACAACTAA GCAGGCAGTG TATCTTCAAT ATAGGTCATT AGATGTATGA
TTAGAGTTAA
G
AATCACCTAG GATTTAAATT CTGCTAACCT ATGTGGGTCA CAAGGAGTTT
AACTTGAGCG
CTTAATGCCT TTAAGATCAT AATCAGGAGA ACTAAAATGA GCTCTTGAGT
TTCTTGGATA
ATTTGTATTC ACTCTTTCCT CCCCCTGGAC TTGGTTCTTT AGTGAAAGGA
AATTCCGAAG
TTTAAAGACA ACGACATCTG GAGCCGCACA TTCCGTTCAC TGGCAGGTGC
TGCACACACG
CCCCTGCGAC CTCCGTGACC CTCTGTCCCC GCTCTTTCCC CTCTGCAGGC

TGCTCTCAGT

### PPARG

SNP name and number (in NCBI SNP database):
   PPARA Pro(P)12Ala(A), [C34G (according to mRNA transcript variant 2:
      NM_015869, nucleotide numbering starts at the start codon, position on NW_ 921654 is 12330520, or position on NT_022517 is 12333125],rs1801282
Gene name and number (in NCBI nucleotide database):
   peroxisome proliferator-activated receptor alpha, NM_005036 and NM_001001928 (mRNA transcript variants) from *homo sapiens* chromosome 3 genomic contig (NW_ 921654 or NT_022517)
   Also known as: NR1C3; PPARG1; PPARG2

### 57. Sequence of the WT allele P12(C34):

TGTACCAAGT CTTGCCAAAG CAGTGAACAT TATGACACAA CTTTTGTCA
CAGCTGGCTC
CTAATAGGAC AGTGCCAGCC AATTCAAGCC CAGTCCTTTC TGTGTTTATT
CCCATCTCTC
CCAAATATTT GGAAACTGAT GTCTTGACTC ATGGGTGTAT TCACAAATTC
TGTTACTTCA
AGTCTTTTTC TTTTAACGGA TTGATCTTTT GCTAGATAGA GACAAAATAT
CAGTGTGAAT
TACAGCAAAC CCCTATTCCA TGCTGTTATG GGTGAAACTC TGGGAGATTC
TCCTATTGAC
C
CAGAAAGCGA TTCCTTCACT GATACACTGT CTGCAAACAT ATCACAAGGT
AAAGTTCCTT
CCAGATACGG CTATTGGGGA CGTGGGGGCA TTTATGTAAG GGTAAAATTG
CTCTTGTAGT
TTGTCTTCCA GGTTGTGTTT GTTTTAATAC TATCATGTGT ACACTCCAGT
ATTTTAATGC
TTAGCTCGTT GCTATCGCGT TCATTTAAAA ACATGTTCAG AACCTTAAAA
AAGGAAACCT
AACCTAATCT ATTTTATCTC TGTGCATGGC TCCCATTTCC TGAATTTTAA
GCATTAAAGG

### 58. Sequence of the mutant allele A12(G34):

TGTACCAAGT CTTGCCAAAG CAGTGAACAT TATGACACAA CTTTTTGTCA
CAGCTGGCTC
CTAATAGGAC AGTGCCAGCC AATTCAAGCC CAGTCCTTTC TGTGTTTATT
CCCATCTCTC
CCAAATATTT GGAAACTGAT GTCTTGACTC ATGGGTGTAT TCACAAATTC
TGTTACTTCA
AGTCTTTTTC TTTTAACGGA TTGATCTTTT GCTAGATAGA GACAAAATAT
CAGTGTGAAT
TACAGCAAAC CCCTATTCCA TGCTGTTATG GGTGAAACTC TGGGAGATTC
TCCTATTGAC
G
CAGAAAGCGA TTCCTTCACT GATACACTGT CTGCAAACAT ATCACAAGGT
AAAGTTCCTT
CCAGATACGG CTATTGGGGA CGTGGGGGCA TTTATGTAAG GGTAAAATTG
CTCTTGTAGT
TTGTCTTCCA GGTTGTGTTT GTTTTAATAC TATCATGTGT ACACTCCAGT
ATTTTAATGC
TTAGCTCGTT GCTATCGCGT TCATTTAAAA ACATGTTCAG AACCTTAAAA
AAGGAAACCT
AACCTAATCT ATTTTATCTC TGTGCATGGC TCCCATTTCC TGAATTTTAA
GCATTAAAGG

### PPARGC1A

SNP name and number (in NCBI SNP database):
   PPARGC1A Gly(G)482Ser(S), [G1444A (according to mRNA transcript:
      NM_013261, nucleotide numbering starts at the start codon, position on NW_ 001838900 is 14439695, or position on NT_006316 is 14491020], rs8192678
Gene name and number (in NCBI nucleotide database):
   peroxisome proliferator-activated receptor gamma, coactivator 1 alpha, (mRNA transcript) from *homo sapiens* chromosome 4 genomic contig (NW_ 001838900 or NT_006316)
   Also known as: LEM6; PGC1; PGC1A; PGC-1v; PPARGC1; PGC-1(alpha)

### 59. Sequence of the WT allele G482 (G1444):

GGTGACCATG ACTATTGCCA GTCAATTAAT TCCAAAACGG AAATACTCAT
TAATATATCA
CAGGAGCTCC AAGACTCTAG ACAACTAGAA AATAAAGATG TCTCCTCTGA
TTGGCAGGGG
CAGATTTGTT CTTCCACAGA TTCAGACCAG TGCTACCTGA GAGAGACTTT
GGAGGCAAGC
AAGCAGGTCT CTCCTTGCAG CACAAGAAAA CAGCTCCAAG ACCAGGAAAT
CCGAGCCGAG
CTGAACAAGC ACTTCGGTCA TCCCAGTCAA GCTGTTTTTG ACGACGAAGC
AGACAAGACC
G
GTGAACTGAG GGACAGTGAT TTCAGTAATG AACAATTCTC CAAACTACCT
ATGTTTATAA
ATTCAGGACT AGCCATGGAT GGCCTGTTTG ATGACAGCGA AGATGAAAGT
GATAAACTGA
GCTACCCTTG GGATGGCACG CAATCCTATT CATTGTTCAA TGTGTCTCCT
TCTTGTTCTT
CTTTTAACTC TCCATGTAGA GATTCTGTGT CACCACCCAA ATCCTTATTT
TCTCAAAGAC
CCCAAAGGAT GCGCTCTCGT TCAAGGTCCT TTTCTCGACA CAGGTCGTGT
TCCCGATCAC

### 60. Sequence of the mutant allele S12 (A 1444):

GGTGACCATG ACTATTGCCA GTCAATTAAT TCCAAAACGG AAATACTCAT
TAATATATCA
CAGGAGCTCC AAGACTCTAG ACAACTAGAA AATAAAGATG TCTCCTCTGA
TTGGCAGGGG
CAGATTTGTT CTTCCACAGA TTCAGACCAG TGCTACCTGA GAGAGACTTT
GGAGGCAAGC
AAGCAGGTCT CTCCTTGCAG CACAAGAAAA CAGCTCCAAG ACCAGGAAAT
CCGAGCCGAG
CTGAACAAGC ACTTCGGTCA TCCCAGTCAA GCTGTTTTTG ACGACGAAGC
AGACAAGACC
A
GTGAACTGAG GGACAGTGAT TTCAGTAATG AACAATTCTC CAAACTACCT
ATGTTTATAA
ATTCAGGACT AGCCATGGAT GGCCTGTTTG ATGACAGCGA AGATGAAAGT
GATAAACTGA
GCTACCCTTG GGATGGCACG CAATCCTATT CATTGTTCAA TGTGTCTCCT
TCTTGTTCTT
CTTTTAACTC TCCATGTAGA GATTCTGTGT CACCACCCAA ATCCTTATTT
TCTCAAAGAC

CCCAAAGGAT GCGCTCTCGT TCAAGGTCCT TTTCTCGACA CAGGTCGTGT
TCCCGATCAC

### PPARGC1A

SNP name and number (in NCBI SNP database):
   PPARGC1A T2842C (according to mRNA transcript: NM_013261, nucleotide numbering starts at the start codon, position on NW_ 001838900 is 14421049, or position on NT_006316 is 14472358], rs6821591
Gene name and number (in NCBI nucleotide database):
   peroxisome proliferator-activated receptor gamma, coactivator 1 alpha, (mRNA transcript) from *homo sapiens* chromosome 4 genomic contig (NW_001838900 or NT_006316)
   Also known as: LEM6; PGC1; PGC1A; PGC-1v; PPARGC1; PGC-1(alpha)

### 61. Sequence of the WT allele T2842:

GAGAGAGAGA GAGACAGGAT ATTAGTTCTA TGGAACCTGT GGTTTCTTCA
GGATTGTCAT
ATAATCATTA CGTTATGAGA GAAAGCTTGC TTCAAGTTGA TTCTGCACTT
TCTTAAAAAA
ACAGAGTACA AAGGCTGATG CCCAGACATC AGCGGCTGTC ATTTTAGGGT
GGTTTGTGGT
TGGTTGGTTG GTTGGTTGGT TGTTAGTTTT CTTTCCTTTT TAATTTATAT
ATATATATAT
ATATATATAT ATTTTTCCTT TTGAATAGAA TACGAACATT TTGAAGTTCT
AGGTTTTAAG
T
GTGTCTTCAT GGAACTGCTG CCATTTGAAA TGGTTTGCCC TTGCGCATTC
TGGTCAGGTG
CCCCCAGTCC TCACATGTAC CCACACATAC TTCCCCTAAA CCAAGCACAC
ACACCACACA
CATACATACA CACACACATA CATGCACACA CGCACACTCC ATCACCAAGA
GACTCCAGGA
AAAGCAAAGC TGACACCCAT GAATAAACAT GTGCTTACTG GATATCATTC
TGTCTCTTGC
CTCTTCAGCA GCTGTGTTCA TGTAAACCAT TGTTGTTATT GTTGTTGTTG
TTCTTGTTGT

### 62. Sequence of the mutant allele C2842:

GAGAGAGAGA GAGACAGGAT ATTAGTTCTA TGGAACCTGT GGTTTCTTCA
GGATTGTCAT
ATAATCATTA CGTTATGAGA GAAAGCTTGC TTCAAGTTGA TTCTGCACTT
TCTTAAAAAA

ACAGAGTACA AAGGCTGATG CCCAGACATC AGCGGCTGTC ATTTTAGGGT
GGTTTGTGGT
TGGTTGGTTG GTTGGTTGGT TGTTAGTTTT CTTTCCTTTT TAATTTATAT
ATATATATAT
ATATATATAT ATTTTTCCTT TTGAATAGAA TACGAACATT TTGAAGTTCT
AGGTTTTAAG
C
GTGTCTTCAT GGAACTGCTG CCATTTGAAA TGGTTTGCCC TTGCGCATTC
TGGTCAGGTG
CCCCCAGTCC TCACATGTAC CCACACATAC TTCCCCTAAA CCAAGCACAC
ACACCACACA
CATACATACA CACACACATA CATGCACACA CGCACACTCC ATCACCAAGA
GACTCCAGGA
AAAGCAAAGC TGACACCCAT GAATAAACAT GTGCTTACTG GATATCATTC
TGTCTCTTGC
CTCTTCAGCA GCTGTGTTCA TGTAAACCAT TGTTGTTATT GTTGTTGTTG
TTCTTGTTGT

### NRF1

SNP name and number (in NCBI SNP database):
   NRF1 A/G (intron) (position on NW_ 001839071 is 2006402, or position on NT_007933 is 54470012], rs6949152
Gene name and number (in NCBI nucleotide database):
   nuclear respiratory factor 1, NM_005011 and NM_1040110 (mRNA transcript variants 1 and 2) from *homo sapiens* chromosome 4 genomic contig (NW_ 001839071 or NT_007933)
   Also known as: ALPHA-PAL

### 63. Sequence of the WT allele A:

CCGGATTGCT GCTTACCAGC ACTGTATGAG TAATTTTGAA TGCAAGTATG
TATTTTCTTC
TTTAAGTATT AATCGGGGCC TAGAAAACCA TGTGTGGCAT TTCAGATAAT
TTAATTCTGG
GAACTGGGTA CACAGGTGTT ACAAGATGAA AGAGCACAAA AGACACTGGG
GTAATCCAGA
TAGGAAGCAC TACCACCCCT AGGTTTGGGG GAACAGAAGA GAAGAACCTG
GAAGCTCAGA
GAGGTCCTCC TGCAGCTGGT AATCTGATCT CCTAGTGGAA TGCCATTCAG
CTGGTACTCA
A
CCACCAGGAG AGAAGAGGCA CAGCTGGAGT TGGGAGGTGC AACACTCCTC
TTGCTGGAGC
AATGCTGATA TTATAACAAT GAAAACAGCA AAGAAATCCC TTCTTCTCTC
CCCTTCCAGT
CTCCCTAGGC AAAACCTAAC AGGATACTGG CAGTAAAGTC TGGAAAACAT
GGTTGGCAGG

CTCAGCCCCA GAGCGAGGAA TAGTGCAACT CTGACAAGAA TAGAAGACAG
GGCTTGGAAG
TGAGACGGAG AGAGAGAGAT AAACAGCAAG CATAACCTTG TAGGAGAGTT
ACAGAAACTG

### 64. Sequence of the mutant allele G:

CCGGATTGCT GCTTACCAGC ACTGTATGAG TAATTTTGAA TGCAAGTATG
TATTTTCTTC
TTTAAGTATT AATCGGGGCC TAGAAAACCA TGTGTGGCAT TTCAGATAAT
TTAATTCTGG
GAACTGGGTA CACAGGTGTT ACAAGATGAA AGAGCACAAA AGACACTGGG
GTAATCCAGA
TAGGAAGCAC TACCACCCCT AGGTTTGGGG GAACAGAAGA GAAGAACCTG
GAAGCTCAGA
GAGGTCCTCC TGCAGCTGGT AATCTGATCT CCTAGTGGAA TGCCATTCAG
CTGGTACTCA
G
CCACCAGGAG AGAAGAGGCA CAGCTGGAGT TGGGAGGTGC AACACTCCTC
TTGCTGGAGC
AATGCTGATA TTATAACAAT GAAAACAGCA AAGAAATCCC TTCTTCTCTC
CCCTTCCAGT
CTCCCTAGGC AAAACCTAAC AGGATACTGG CAGTAAAGTC TGGAAAACAT
GGTTGGCAGG
CTCAGCCCCA GAGCGAGGAA TAGTGCAACT CTGACAAGAA TAGAAGACAG
GGCTTGGAAG
TGAGACGGAG AGAGAGAGAT AAACAGCAAG CATAACCTTG TAGGAGAGTT
ACAGAAACTG

### NRF1

SNP name and number (in NCBI SNP database):
   NRF1 C/T (intron) (position on NW_001839071 is 1913409, or position on NT_007933 is 54563377], rs2402970
Gene name and number (in NCBI nucleotide database):
   nuclear respiratory factor 1, NM_005011 and NM_1040110 (mRNA transcript variants 1 and 2) from *homo sapiens* chromosome 4 genomic contig (NW_ 001839071 or NT_007933)
   Also known as: ALPHA-PAL

### 65. Sequence of the WT allele C:

AGACCGGAGA CAGTGGTGAC ATCAAGACTC ACTTCCTAGT GAGTTTATTC
ATGACTCAGG
CCATGTGCAT ATTTCCACTT GGATTCCTCA CAAGTGCAAG TTTCTCTTCT
AGGCTACAGG
TATCTGCAGC CAGGTGCTGT AATGGATTTA TAGTAGCCCG CCAAAAGATC
AAATATCTGA

AGCCTGACGG GTCACAAAGC TGACAGGAGC CATGACTATT GCATGAATTC
CAGATTTCAC
AGTGAAACAG AGCTGCTGCT GAAAAATGGG AGCACAGAGC TGTTCACTGG
GGCT
C
TTGATTCTAG ACACTCAAAG AGGGGTACAG TTCTCTGCCC CATGTGTGTT
TCTCACCCAA
CCTTTTCATG GTGTTGAGGA GGGGAAGGGA CAGCTCAGTG GTGATTCTTC
ACTTCTCAAA
CGTGTCCTCC TCCTCTGTCT TCATAATGAC CTCCTGAAGT CCAAAGTGCC
TTCCAAAATA
GCTCTTGGAA AGAGGTGGCA GTTTCCTGAC TCCCCAATCT CCCCCATCCT
GGGAGATGTG
TTGATGGCAC TAATTTGCGG GGTAGCAGAG ACTATAGTCC TACTCTCTTT
ACTGGCTGGG
GCATTGGAGG CATGCAGTAA CTGTTGACTA CCACTGCTAC AACTGCTGTT
ATGATTATTA
CCATTATAAT GATTAATCAC TCACACCGAA TGGGAAGGGA AGTAACCTTC
AGAGGTTCAC
TCTTAGTACT TCTGGCACAT ATGCTTAGGG CCAGGGCCAG TGAGTCTTCA
TTCGCAGAGA
GGTG

### 66. Sequence of the mutant allele T:

AGACCGGAGA CAGTGGTGAC ATCAAGACTC ACTTCCTAGT GAGTTTATTC
ATGACTCAGG
CCATGTGCAT ATTTCCACTT GGATTCCTCA CAAGTGCAAG TTTCTCTTCT
AGGCTACAGG
TATCTGCAGC CAGGTGCTGT AATGGATTTA TAGTAGCCCG CCAAAAGATC
AAATATCTGA
AGCCTGACGG GTCACAAAGC TGACAGGAGC CATGACTATT GCATGAATTC
CAGATTTCAC
AGTGAAACAG AGCTGCTGCT GAAAAATGGG AGCACAGAGC TGTTCACTGG
GGCT
T
TTGATTCTAG ACACTCAAAG AGGGGTACAG TTCTCTGCCC CATGTGTGTT
TCTCACCCAA
CCTTTTCATG GTGTTGAGGA GGGGAAGGGA CAGCTCAGTG GTGATTCTTC
ACTTCTCAAA
CGTGTCCTCC TCCTCTGTCT TCATAATGAC CTCCTGAAGT CCAAAGTGCC
TTCCAAAATA
GCTCTTGGAA AGAGGTGGCA GTTTCCTGAC TCCCCAATCT CCCCCATCCT
GGGAGATGTG
TTGATGGCAC TAATTTGCGG GGTAGCAGAG ACTATAGTCC TACTCTCTTT
ACTGGCTGGG
GCATTGGAGG CATGCAGTAA CTGTTGACTA CCACTGCTAC AACTGCTGTT
ATGATTATTA
CCATTATAAT GATTAATCAC TCACACCGAA TGGGAAGGGA AGTAACCTTC
AGAGGTTCAC
TCTTAGTACT TCTGGCACAT ATGCTTAGGG CCAGGGCCAG TGAGTCTTCA
TTCGCAGAGA
GGTG

### GABPB2

SNP name and number (in NCBI SNP database):
   GABPB2 C/T (intron) (position on NW_ 001838218 is 23762751, or position on NT_010194 is 21387881], rs8031031
Gene name and number (in NCBI nucleotide database):
   GA binding protein transcription factor, beta subunit 2, NM_005254, NM_016654, NM_002041 NM_0166545 an NM_181427 (mRNA transcript variants 1-5) from *homo sapiens* chromosome 15 genomic contig (NW_ 001838218 or NT_010194) Also known as: E4TF1; GABPB; BABPB2; E4TF1B; GABPB1; NRF2B1; NRF2B2; E4TF1-47; E4TF1-53

### 67. Sequence of the WT allele C:

TCTTTTTAAA GGGATTTTTT TCAAAGGAAA AATCTTAGGT GCATTTGCCA
AAGTTGAATC
AGGACAAAAC CAAAACAGAG CCCAATTCTC CTGAAATTGG TTCCATGTGG
TCTAGTCATT
AAATTGACAT CCACAATACA CAGAGGGAGT ACTAAAATGT GAGGGAAGGA
AGATTAAGTT
CTATCACATG CATTTAAAGG
C
ACAGAAAATC CCTATAACCC TCTGATTACC ACATCACTTG ATATTCCAAA
GAACTAAGAT
ATTTGGACTA AGGAAATTTA CACATATAAA CAGTTTATTA TGTTATGAAG
AGATTAAGTG
AGATATATGT ATAAAGGTCC TTAGTC

### 68. Sequence of the mutant allele T:

TCTTTTTAAA GGGATTTTTT TCAAAGGAAA AATCTTAGGT GCATTTGCCA
AAGTTGAATC
AGGACAAAAC CAAAACAGAG CCCAATTCTC CTGAAATTGG TTCCATGTGG
TCTAGTCATT
AAATTGACAT CCACAATACA CAGAGGGAGT ACTAAAATGT GAGGGAAGGA
AGATTAAGTT
CTATCACATG CATTTAAAGG
T
ACAGAAAATC CCTATAACCC TCTGATTACC ACATCACTTG ATATTCCAAA
GAACTAAGAT
ATTTGGACTA AGGAAATTTA CACATATAAA CAGTTTATTA TGTTATGAAG
AGATTAAGTG
AGATATATGT ATAAAGGTCC TTAGTC

### GABPB2

SNP name and number (in NCBI SNP database):
   GABPB2 A/G (intron) (position on NW_001838218 is 23749281, or position on NT_010I94 is 21401349), rs7181866
Gene name and number (in NCBI nucleotide database):
   GA binding protein transcription factor, beta subunit 2, NM_005254, NM_016654, NM_002041 NM_0166545 an NM_181427 (mRNA transcript variants 1-5) from *homo sapiens* chromosome 15 genomic contig (NW_001838218 or NT_010194) Also known as: E4TF1; GABPB; BABPB2; E4TF1B; GABPB1; NRF2B1; NRF2B2; E4TF1-47; E4TF1-53

### 69. Sequence of the WT allele A:

TTCTCAATTC TTCCTGTGTT CTAAGTTGAA GAACACTAGG AATCTGCTGG
GATATAAAGG
GCATGTCCTC CAAGGATTTA AGTTTAGTGT CTCCCAGTGT AATTCCTTTG
AAACGAAAAA
TCATCACATG AACTCTGGTA TATTTGGATT TTTTCCTCCA TGCCCATTTT
ACCTAATGGA
GTTTTTTTCC CCTTTATTTT taaaataaat gatattttca aatattatat
ccatatatgt
atctcccata taccccttttt tcagaaaacc actagatgac gtgttccacc
aaaaccaaaa
gtaaactaga aagaataatc aagggcttca ggaaataaag atccaacata
gaataggaga
gagt
A
cccaaaatga tggtgaaggg agaccccaag acaacagctg tgacacagac
ctagaaggca
aacccagatg ggagccagag aacagagttc caggaaggag gtctccaagg
aaaagacagg
ctggtaagtc tcctaagaag agaatgaatg tcagtggaga gtttgggatg
cgttaatgac
ggatacaaga aaactaagaa aagaaaaaac tagactattt ttcagaaggg
agaaaaaaac
tataccataa aggtatacat gtctcttgta cagtttttcc tttacacaca
cacacacaga

### 70. Sequence of the mutant allele G:

TTCTCAATTC TTCCTGTGTT CTAAGTTGAA GAACACTAGG AATCTGCTGG
GATATAAAGG
GCATGTCCTC CAAGGATTTA AGTTTAGTGT CTCCCAGTGT AATTCCTTTG
AAACGAAAAA
TCATCACATG AACTCTGGTA TATTTGGATT TTTTCCTCCA TGCCCATTTT
ACCTAATGGA
GTTTTTTTCC CCTTTATTTT taaaataaat gatattttca aatattatat
ccatatatgt
atctcccata taccccttttt tcagaaaacc actagatgac gtgttccacc
aaaaccaaaa
gtaaactaga aagaataatc aagggcttca ggaaataaag atccaacata
gaataggaga

gagt
G
cccaaaatga tggtgaaggg agaccccaag acaacagctg tgacacagac
ctagaaggca
aacccagatg ggagccagag aacagagttc caggaaggag gtctccaagg
aaaagacagg
ctggtaagtc tcctaagaag agaatgaatg tcagtggaga gtttgggatg
cgttaatgac
ggatacaaga aaactaagaa aagaaaaaac tagactattt ttcagaaggg
agaaaaaaac
tataccataa aggtatacat gtctcttgta cagtttttcc tttacacaca
cacacacaga

## Claims

1. Use of a polymorphism for diagnosis of susceptibility to cardiac disease in a subject, wherein said polymorphism occurs in a renin angiotensin system gene, **characterized in that** said polymorphism occurs in AT1R, further **characterized in that** said cardiac disease comprises heart failure and atrial fibrillation, wherein said atrial fibrillation is a complication of heart failure.

2. The use according to claim 1 wherein the AT1R gene comprises SEQ ID NO: 2.

3. The use of claim 1 or 2, wherein said AT1R polymorphism is an A1166C polymorphism.

## Patentansprüche

1. Verwendung eines Polymorphismus für die Diagnose von Anfälligkeit für Herzerkrankung in einem Individuum, wobei der genannte Polymorphismus in einem Renin-Angiotensin-System-Gen stattfindet, **dadurch gekennzeichnet, dass** der genannte Polymorphismus in AT1R stattfindet, zusätzlich **dadurch gekennzeichnet, dass** die genannte Herzerkrankung Herzinsuffizienz und Vorhofflimmern umfasst, wobei das genannte Vorhofflimmern eine Komplikation der Herzinsuffizienz ist.

2. Verwendung nach Anspruch 1, wobei das AT1R-Gen SEQ ID NO:2 umfasst.

3. Verwendung nach Anspruch 1 oder 2, wobei der genannte AT1R-Polymorphismus ein A1166C-Polymorphismus ist.

## Revendications

1. Utilisation d'un polymorphisme pour le diagnostic de susceptibilité d'une maladie cardiaque chez un sujet, dans laquelle ledit polymorphisme à lieu dans un gène du système rénine-angiotensine, **caractérisé en ce que** ledit polymorphisme à lieu dans AT1R, **caractérisé en outre en ce que** ladite maladie cardiaque comprend l'insuffisance cardiaque et la fibrillation auriculaire, dans laquelle ladite fibrillation auriculaire est une complication de l'insuffisance cardiaque.

2. Utilisation selon la revendication 1 dans laquelle le gène AT1R comprend la SEQ ID NO : 2.

3. Utilisation selon la revendication 1 ou 2, dans laquelle ledit polymorphisme AT1R est un polymorphisme A1166C.
